# EUROPEAN PATENT APPLICATION

(11) **EP 4 722 245 A1**
(43) Date of publication of application: **08.04.2026**
(21) Application number: 23938990.1
(22) Date of filing: 02.06.2023
(51) Int. Cl.: C07K 16/28, C12N 15/13, A61K 39/395, A61P 35/00

(54) **ANTIBODY TARGETING ITGA2, ANTIBODY DRUG CONJUGATE THEREOF, AND USE**

(71) Applicant: Foreseen Technology (Beijing) Co., Ltd., Beijing 100192 (CN); Foreseen Biotechnology (Shanghai) Co., Ltd., Shanghai 201210 (CN)
(72) Inventor: WONG, Catherine C., Beijing 100192 (CN); LI, Wei, Shanghai 201210 (CN); HAN, Pei, Beijing 100192 (CN)
(74) Representative: Mathys & Squire
(86) International application number: PCT/CN2023/098087
(87) International publication number: WO 2024/244006

(57) **Abstract**

The present invention provides a series of antibodies or antigen-binding fragments thereof with high specific binding ability to tumor cells expressing the ITGA2 protein. The antibody or the antigen-binding fragment thereof according to the present invention may achieve excellent killing effect on tumor cells after being conjugated with a drug to form an antibody-drug conjugate.

## Description

### Technical Field

The invention relates to the bio-pharmaceutical field. Specifically, the invention relates to an antibody targeting ITGA2 or an antigen-binding fragment thereof, an antibody-drug conjugate comprising the antibody, and use of the antibody and/or the antibody-drug conjugate in treating related diseases.

### Background Art

Malignant tumors (cancers) have become the "number one killer" threatening the lives and health of people around the world. There are more than 14 million cancer cases per year in the world, among which more than 3 million new cancer patients occur in China alone each year.

The root cause for high mortality of cancer is the spread, metastasis of cancer cells and the relapse and drug resistance of most patients after treatment. Among the present clinically available treatments, surgery, radiotherapy, and chemotherapy have little effect on metastasis of cancer cells, relapse, and drug resistance, or have only short-term effect, which makes it unable to change the long-term survival of the patients. At present, excision is effective for about 10-20% of early patients, but it is almost ineffective for patients who have undergone spread and metastasis. As radiotherapy can only treat local lesions, it is often used as adjunctive therapy before and after surgery and radical treatment of a few types of cancers. Though chemotherapy can be used for patients who have cancer spread and metastasis, but due to its strong toxic and side effects and its tendency to induce short-term or long-term drug resistance, it can only have a significant short-term therapeutic effect on about 20-30% of patients. Even with a comprehensive treatment measure integrating the surgery, radiotherapy and chemotherapy, its five-year survival long term therapeutic effect has been kept from 20% to 30% for many years, where about 70% to 80% patients have died within 5 years after treatment due to metastasis, relapse, and drug resistance. Even in early cancer patients who do not have metastasis at the time of the visit, some also have died due to metastasis and relapse after treatment.

At present, it is effective to treat tumor by identifying a tumor-associated antigen, preparing a drug in the form of an antibody-drug conjugate, and then administering the drug to the tumor cells. This drug form has the both the advantage of monoclonal antibody targeting tumor cells and the advantage of micromolecule cytotoxins that inflict strong killing on tumors, thereby not only significantly reducing the toxic and side effects of micromolecule cytotoxins, but also improving the efficacy of the drug, which results in significant treatment advantages.

### Summary of the Invention

The inventors found that ITGA2 is a tumor-associated antigen that is specifically overexpressed by tumor cells, and it is up-regulated in most solid tumors, and is overexpressed especially in cancers such as colorectal cancer, bile duct cancer, pancreatic cancer, and skin cancer. However, the existing antibodies that can target ITGA2 alone do not have a good inhibition effect on the growth of tumor cells. Therefore, the inventors developed a series of antibodies with high specific binding ability to tumor cells expressing the ITGA2 protein, and the antibodies target ITGA2-positive tumor cells in the form of antibody-drug conjugates to achieve excellent killing effect on cells.

In one aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 42, (b) LCDR2 as set forth in SEQ ID NO: 43, and (c) LCDR3 as set forth in SEQ ID NO: 44, and a heavy chain variable region that comprises (d) HCDR1 as set forth in SEQ ID NO: 46, (e) HCDR2 as set forth in SEQ ID NO: 47, and (f) HCDR3 as set forth in SEQ ID NO: 48;
2) a light chain variable region that comprises (a) LCDR1 as set forth in SEQ ID NO: 50, (b) LCDR2 as set forth in SEQ ID NO: 51, and (c) LCDR3 as set forth in SEQ ID NO: 52, and a heavy chain variable region that comprises (d) HCDR1 as set forth in SEQ ID NO: 54, (e) HCDR2 as set forth in SEQ ID NO: 55, and (f) HCDR3 as set forth in SEQ ID NO:56;
3) a light chain variable region that comprises (a) LCDR1 as set forth in SEQ ID NO: 58, (b) LCDR2 as set forth in SEQ ID NO: 59, and (c) LCDR3 as set forth in SEQ ID NO: 60, and a heavy chain variable region that comprises (d) HCDR1 as set forth in SEQ ID NO: 62, (e) HCDR2 as set forth in SEQ ID NO: 63, and (f) HCDR3 as set forth in SEQ ID NO: 54; and
4) a light chain variable region that comprises (a) LCDR1 as set forth in SEQ ID NO: 66, (b) LCDR2 as set forth in SEQ ID NO: 67, and (c) LCDR3 as set forth in SEQ ID NO: 68, and a heavy chain variable region that comprises (d) HCDR1 as set forth in SEQ ID NO: 70, (e) HCDR2 as set forth in SEQ ID NO: 71, and (f) HCDR3 as set forth in SEQ ID NO: 72.

In one aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 74, (b) LCDR2 as set forth in SEQ ID NO: 75, and (c) LCDR3 as set forth in SEQ ID NO: 76, and a heavy chain variable region that comprises (d) HCDR1 as set forth in SEQ ID NO: 78, (e) HCDR2 as set forth in SEQ ID NO: 79, and (f) HCDR3 as set forth in SEQ ID NO: 80;
2) a light chain variable region that comprises (a) LCDR1 as set forth in SEQ ID NO: 82, (b) LCDR2 as set forth in SEQ ID NO: 83, and (c) LCDR3 as set forth in SEQ ID NO: 84, and a heavy chain variable region that comprises (d) HCDR1 as set forth in SEQ ID NO: 86, (e) HCDR2 as set forth in SEQ ID NO: 87, and (f) HCDR3 as set forth in SEQ ID NO:88;
3) a light chain variable region that comprises (a) LCDR1 as set forth in SEQ ID NO: 90, (b) LCDR2 as set forth in SEQ ID NO: 91, and (c) LCDR3 as set forth in SEQ ID NO: 92, and a heavy chain variable region that comprises (d) HCDR1 as set forth in SEQ ID NO: 94, (e) HCDR2 as set forth in SEQ ID NO: 95, and (f) HCDR3 as set forth in SEQ ID NO: 96;
4) a light chain variable region that comprises (a) LCDR1 as set forth in SEQ ID NO: 98, (b) LCDR2 as set forth in SEQ ID NO: 99, and (c) LCDR3 as set forth in SEQ ID NO: 100, and a heavy chain variable region that comprises (d) HCDR1 as set forth in SEQ ID NO: 102, (e) HCDR2 as set forth in SEQ ID NO: 103, and (f) HCDR3 as set forth in SEQ ID NO: 104;
5) a light chain variable region that comprises (a) LCDR1 as set forth in SEQ ID NO: 106, (b) LCDR2 as set forth in SEQ ID NO: 107, and (c) LCDR3 as set forth in SEQ ID NO: 108, and a heavy chain variable region that comprises (d) HCDR1 as set forth in SEQ ID NO: 110, (e) HCDR2 as set forth in SEQ ID NO: 111, and (f) HCDR3 as set forth in SEQ ID NO: 112;
6) a light chain variable region that comprises (a) LCDR1 as set forth in SEQ ID NO: 114, (b) LCDR2 as set forth in SEQ ID NO: 115, and (c) LCDR3 as set forth in SEQ ID NO: 116, and a heavy chain variable region that comprises (d) HCDR1 as set forth in SEQ ID NO: 118, (e) HCDR2 as set forth in SEQ ID NO: 119, and (f) HCDR3 as set forth in SEQ ID NO: 120; and
7) a light chain variable region that comprises (a) LCDR1 as set forth in SEQ ID NO: 122, (b) LCDR2 as set forth in SEQ ID NO: 123, and (c) LCDR3 as set forth in SEQ ID NO: 124, and a heavy chain variable region that comprises (d) HCDR1 as set forth in SEQ ID NO: 126, (e) HCDR2 as set forth in SEQ ID NO: 127, and (f) HCDR3 as set forth in SEQ ID NO: 128.

In one aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 130, (b) LCDR2 as set forth in SEQ ID NO: 131, and (c) LCDR3 as set forth in SEQ ID NO: 132, and a heavy chain variable region that comprises (d) HCDR1 as set forth in SEQ ID NO: 134, (e) HCDR2 as set forth in SEQ ID NO: 135, and (f) HCDR3 as set forth in SEQ ID NO: 136;
2) a light chain variable region that comprises (a) LCDR1 as set forth in SEQ ID NO: 138, (b) LCDR2 as set forth in SEQ ID NO: 139, and (c) LCDR3 as set forth in SEQ ID NO: 140, and a heavy chain variable region that comprises (d) HCDR1 as set forth in SEQ ID NO: 142, (e) HCDR2 as set forth in SEQ ID NO: 143, and (f) HCDR3 as set forth in SEQ ID NO:144;
3) a light chain variable region that comprises (a) LCDR1 as set forth in SEQ ID NO: 146, (b) LCDR2 as set forth in SEQ ID NO: 147, and (c) LCDR3 as set forth in SEQ ID NO: 148, and a heavy chain variable region that comprises (d) HCDR1 as set forth in SEQ ID NO: 150, (e) HCDR2 as set forth in SEQ ID NO: 151, and (f) HCDR3 as set forth in SEQ ID NO: 152; and
4) a light chain variable region that comprises (a) LCDR1 as set forth in SEQ ID NO: 154, (b) LCDR2 as set forth in SEQ ID NO: 155, and (c) LCDR3 as set forth in SEQ ID NO: 156, and a heavy chain variable region that comprises (d) HCDR1 as set forth in SEQ ID NO: 158, (e) HCDR2 as set forth in SEQ ID NO: 159, and (f) HCDR3 as set forth in SEQ ID NO: 160.

In one aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 162, (b) LCDR2 as set forth in SEQ ID NO: 163, and (c) LCDR3 as set forth in SEQ ID NO: 164, and a heavy chain variable region that comprises (d) HCDR1 as set forth in SEQ ID NO: 166, (e) HCDR2 as set forth in SEQ ID NO: 167, and (f) HCDR3 as set forth in SEQ ID NO: 168;
2) a light chain variable region that comprises (a) LCDR1 as set forth in SEQ ID NO: 170, (b) LCDR2 as set forth in SEQ ID NO: 171, and (c) LCDR3 as set forth in SEQ ID NO: 172, and a heavy chain variable region that comprises (d) HCDR1 as set forth in SEQ ID NO: 174, (e) HCDR2 as set forth in SEQ ID NO: 175, and (f) HCDR3 as set forth in SEQ ID NO:176;
3) a light chain variable region that comprises (a) LCDR1 as set forth in SEQ ID NO: 178, (b) LCDR2 as set forth in SEQ ID NO: 179, and (c) LCDR3 as set forth in SEQ ID NO: 180, and a heavy chain variable region that comprises (d) HCDR1 as set forth in SEQ ID NO: 182, (e) HCDR2 as set forth in SEQ ID NO: 183, and (f) HCDR3 as set forth in SEQ ID NO: 184;
4) a light chain variable region that comprises (a) LCDR1 as set forth in SEQ ID NO: 186, (b) LCDR2 as set forth in SEQ ID NO: 187, and (c) LCDR3 as set forth in SEQ ID NO: 188, and a heavy chain variable region that comprises (d) HCDR1 as set forth in SEQ ID NO: 190, (e) HCDR2 as set forth in SEQ ID NO: 191, and (f) HCDR3 as set forth in SEQ ID NO: 192; and
5) a light chain variable region that comprises (a) LCDR1 as set forth in SEQ ID NO: 194, (b) LCDR2 as set forth in SEQ ID NO: 195, and (c) LCDR3 as set forth in SEQ ID NO: 196, and a heavy chain variable region that comprises (d) HCDR1 as set forth in SEQ ID NO: 198, (e) HCDR2 as set forth in SEQ ID NO: 199, and (f) HCDR3 as set forth in SEQ ID NO: 200.

In yet another aspect, the present invention provides an isolated nucleic acid molecule encoding a monoclonal antibody or an antigen-binding fragment thereof according to the present invention.

In yet another aspect, the present invention provides an expression vector comprising the nucleic acid molecule according to the present invention.

In yet another aspect, the present invention provides a host cell comprising the nucleic acid molecule according to the present invention or the expression vector according to the invention.

In yet another aspect, the present invention provides a hybridoma cell expressing a monoclonal antibody or an antigen-binding fragment thereof according to the present invention.

In yet another aspect, the present invention provides use of the monoclonal antibody or the antigen-binding fragment thereof according to the present invention or the antibody-drug conjugate according to the present invention in preparing a pharmaceutical composition; optionally, the pharmaceutical composition is used to treat a malignant tumor, or prevent and/or treat metastasis or relapse of a malignant tumor in a patient.

In yet another aspect, the present invention provides a method for treating a malignant tumor, preventing and/or treating metastasis or relapse of a malignant tumor in a patient, wherein the method comprises administering to the patient an effective amount of the monoclonal antibody or the antigen-binding fragment thereof according to the present invention, the antibody-drug conjugate according to the present invention, or the pharmaceutical composition according to the present invention.

In yet another aspect, the present invention provides use of the monoclonal antibody or the antigen-binding fragment thereof according to the present invention or the antibody-drug conjugate according to the present invention in preparing a diagnostic/prognostic composition; optionally, the diagnostic/prognostic composition is used to detect presence of a malignant tumor in a patient; optionally, the diagnostic/prognostic composition is used to prognosticate relapse or progression of a malignant tumor in a patient.

In yet another aspect, the invention provides a method for prognosticating relapse or progression of a malignant tumor in a patient, wherein the method comprises:
(a) isolating a biological sample comprising tumor cells from the patient;
(b) contacting the biological sample comprising the tumor cells with the monoclonal antibody or the antigen-binding fragment thereof according to the present invention; and
(c) identifying the presence of the tumor cells that bind to the monoclonal antibody or the antigen-binding fragment thereof,
thereby prognosticating the relapse or progression of the malignant tumor in the patient.

In yet another aspect, the present invention provides a method for detecting/diagnosing presence of a malignant tumor in a patient, comprising:
a) contacting a biological sample obtained from the patient with the monoclonal antibody or the antigen-binding fragment thereof according to the invention or the antibody-drug conjugate according to the present invention;
b) detecting binding of the monoclonal antibody or the antigen-binding fragment thereof to a target antigen in the biological sample, wherein the detected binding represents the presence of the malignant tumor in the patient.

In yet another aspect, the invention provides a method for detecting the presence of tumor cells in a biological sample, comprising:
a) contacting the biological sample with the monoclonal antibody or the antigen-binding fragment thereof according to the invention or the antibody-drug conjugate according to the present invention;
b) detecting binding of the monoclonal antibody or the antigen-binding fragment thereof or the antibody-drug conjugate to a target antigen in the biological sample,
wherein the detected binding represents presence of the tumor cells in the biological sample.

In yet another aspect, the present invention provides a method for isolating tumor cells, wherein the method comprises:
(a) providing a cell population suspected of comprising the tumor cells;
(b) identifying a subpopulation of the cells that bind to the monoclonal antibody or the antigen-binding fragment thereof or the antibody-drug conjugate according to the present invention; and
(c) isolating the subpopulation.

In yet another aspect, the present invention provides a method for producing a monoclonal antibody or an antigen-binding fragment thereof targeting human tumor cells that express ITGA2, wherein the method comprises:
(i) culturing the host cell of the present invention in a condition suitable for expressing the nucleic acid molecule or the expression vector, and
(ii) isolating and purifying the antibody or the antigen-binding fragment thereof expressed by the nucleic acid molecule or the expression vector.

The antibody according to the present invention has an excellent binding ability to the ITGA2 protein, and is capable of binding to tumor cells that express ITGA2, especially colorectal cancer cells, bile duct cancer cells, pancreatic cancer cells and skin cancer cells. The antibody-drug conjugate according to the present invention has an excellent killing effect on tumor cells that express ITGA2, especially colorectal cancer cells, bile duct cancer cells, pancreatic cancer cells and skin cancer cells.

### Sequence Information:

In the following tables, the first left column lists the sequence numbering (SEQ ID NO:) of the present invention.

**Table 1: Nucleic acid coding sequences SEQ ID NO:1-40 of the light and heavy chains of the antibody according to the present invention (the underlined sequences refer to the coding sequences of the constant regions)**

| **No.** | **Description** | **Nucleic Acid Sequence** |
|---|---|---|
| 1 | Chimeric mouse antibody FS002-A103 light chain coding sequence | GACATCGTGATGACCCAGTCTCCAAAGTCCATGTCCATGAGCGTG GGCGAGAGAGTGACCCTGTCTTGCAAGGCCTCTGAGAACGTGGG CACCTACGTGTCCTGGTATCAGCAGAAGCCTGAGCAGTCCCCTAA GCTGCTGATCTACGGCGCCTCCACCAGAGAATCTGGCGTGCCCGA TAGATTCACCGGCTCTGGCTCTGGCACCGACTTTACCCTGACCAT CAGCTCTGTGCAGGCCGAGGATCTGGCCGTGTACTACTGCCAGAA CGACTACAGCTACCCCTTCACCTTTGGCTCCGGCACCAAGCTGGA AATCAAGAGGACCGTGGCTGCCCCCAGCGTGTTCATCTTCCCTCC TAGCGACGAGCAGCTGAAGAGCGGCACCGCTAGCGTGGTGTGTC TGCTGAATAACTTCTATCCCAGGGAGGCCAAGGTGCAGTGGAAG GTGGATAACGCCCTGCAGAGCGGCAACTCCCAGGAGTCCGTGAC CGAGCAGGACTCCAAGGACAGCACCTACTCCCTGAGCTCCACCCT GACCCTGTCCAAGGCTGATTATGAGAAGCACAAGGTGTATGCTTG CGAGGTGACACACCAGGGCCTGTCCAGCCCTGTGACCAAGAGCTT CAACCGGGGCGAGTGC |
| 2 | Chimeric mouse antibody FS002-A103 heavy chain coding sequence | CAGGTTCAGTTGCAGCAGTCTGGCGCCGAACTCGTTAGACCTGGC GCTTCTGTGACCCTGTCCTGCAAGGCTTCTGGCTACACCTTCACCG ACTACGAGATGCACTGGGTCAAGCAGACCCCTGTGCATGGCCTGG AATGGATCGGCGTGATCGATCCTGAGACAGGCGGCACCACCTAC AACCAGAAGTTCAACGACAAGGCTACCCTGACCGCCGACAAGTC CTCTTCCACCGCCTACATGGACCTGCGGTCCCTGACCTCTGAGGA CTCTGCCGTGTACTACTGCTCCAGATCCGGCTACTACGTGTCCTCC TACGAGTCCTACGCTATGGACTATTGGGGCCAGGGCACCTCCGTG ACAGTCTCTTCTGCTTCCACCAAGGGCCCCTCCGTGTTCCCCCTGG CTCCCTCTTCCAAGAGCACCAGCGGCGGCACCGCTGCTCTGGGAT GTCTGGTGAAGGACTACTTCCCTGAGCCTGTGACCGTGTCCTGGA ATTCCGGCGCCCTGACCTCCGGCGTGCACACATTCCCTGCTGTGC TGCAGTCCTCCGGCCTGTATAGCCTGTCCTCCGTGGTGACAGTGC CTAGCTCCAGCCTGGGCACCCAGACCTATATCTGCAACGTGAACC ACAAGCCTAGCAATACCAAGGTGGACAAGAAGGTGGAGCCTAAG AGCTGCGACAAGACCCACACCTGTCCTCCATGTCCTGCTCCAGAA CTGCTCGGCGGACCTTCCGTGTTCCTGTTTCCTCCAAAGCCTAAGG ACACCCTGATGATCAGCAGAACCCCTGAAGTGACCTGCGTGGTGG TGGATGTGTCCCACGAGGATCCCGAAGTGAAGTTCAATTGGTACG TGGACGGCGTGGAAGTGCACAACGCCAAGACCAAGCCTAGAGAG GAACAGTACAACAGCACCTACAGAGTGGTGTCCGTGCTGACCGT GCTGCACCAGGATTGGCTGAACGGCAAAGAGTACAAGTGCAAGG TGTCCAACAAGGCCCTGCCTGCTCCTATCGAGAAAACCATCAGCA AGGCCAAGGGCCAGCCTAGGGAACCCCAGGTTTACACACTGCCT CCAAGCAGGGACGAGCTGACCAAGAATCAGGTGTCCCTGACCTG |
| | | CCTGGTCAAGGGCTTCTACCCTTCCGATATCGCCGTGGAATGGGA GAGCAATGGCCAGCCTGAGAACAACTACAAGACAACCCCTCCTG TGCTGGACAGCGACGGCTCATTCTTCCTGTACAGCAAGCTGACAG TGGACAAGAGCAGATGGCAGCAGGGCAACGTGTTCAGCTGCAGC GTGATGCACGAGGCCCTGCACAACCACTACACCCAGAAGTCCCTG AGCCTGTCTCCTGGCAAA |
| 3 | Humanized antibody FS002-A103-H4 L2 light chain coding sequence | GAGATCGTGATGACCCAGTCTCCTAAGACCATGTCCATGTCTCCC GGCGAGAGAGTGACCCTGTCTTGCAAGGCCTCTGAGAACGTGGG CACCTACGTGTCCTGGTATCAGCAGAAGCCTGGACAGGCCCCTCG GCTGTTGATCTACGGCGCTTCCACAAGAGAATCCGGCGTGCCCGA TAGATTCTCCGGCTCTGGCTCTGGCACCGACTTTACCCTGACAATC TCCAGCCTGCAGCCTGAGGACTTCGCCGTGTACTACTGCCAGAAC GACTACAGCTACCCCTTCACCTTTGGCGGAGGCACCAAGCTGGAA ATCAAGAGGACCGTGGCTGCCCCCAGCGTGTTCATCTTCCCTCCT AGCGACGAGCAGCTGAAGAGCGGCACCGCTAGCGTGGTGTGTCT GCTGAATAACTTCTATCCCAGGGAGGCCAAGGTGCAGTGGAAGG TGGATAACGCCCTGCAGAGCGGCAACTCCCAGGAGTCCGTGACC GAGCAGGACTCCAAGGACAGCACCTACTCCCTGAGCTCCACCCTG ACCCTGTCCAAGGCTGATTATGAGAAGCACAAGGTGTATGCTTGC GAGGTGACACACCAGGGCCTGTCCAGCCCTGTGACCAAGAGCTTC AACCGGGGCGAGTGC |
| 4 | Humanized antibody FS002-A103-H4 L2 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATGCACTGGGTCCGACAGGCTCCAGGACAAGGCTT GGAATGGATGGGCGTGATCGACCCTGAGACAGGCGGCACCACCT ACAACCAGAAATTCCAGGGCAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 5 | Humanized antibody FS002-A103-H4 L3 light chain coding sequence | GAGATCGTGATGACCCAGTCTCCTCCTACCATGTCCATGTCTCCC GGCGAGAGAGTGACCCTGTCTTGCAAGGCCTCTGAGAACGTGGG CACCTACGTGTCCTGGTATCAGCAGAAGCCTGGACAGGCCCCTCG GCTGTTGATCTACGGCGCTTCCACAAGAGAATCCGGCGTGCCAGC CAGATTCTCCGGCTCTGGATCTGGCACCGACTTTACCCTGACAAT CTCCAGCCTGCAGCCTGAGGACTTCGCCGTGTACTACTGCCAGAA CGACTACAGCTACCCCTTCACCTTTGGCGGAGGCACCAAGCTGGA AATCAAGAGGACCGTGGCTGCCCCCAGCGTGTTCATCTTCCCTCC TAGCGACGAGCAGCTGAAGAGCGGCACCGCTAGCGTGGTGTGTC TGCTGAATAACTTCTATCCCAGGGAGGCCAAGGTGCAGTGGAAG GTGGATAACGCCCTGCAGAGCGGCAACTCCCAGGAGTCCGTGAC CGAGCAGGACTCCAAGGACAGCACCTACTCCCTGAGCTCCACCCT GACCCTGTCCAAGGCTGATTATGAGAAGCACAAGGTGTATGCTTG CGAGGTGACACACCAGGGCCTGTCCAGCCCTGTGACCAAGAGCTT CAACCGGGGCGAGTGC |
| 6 | Humanized antibody FS002-A103-H4 L3 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC |
| | | GACTACGAGATGCACTGGGTCCGACAGGCTCCAGGACAAGGCTT GGAATGGATGGGCGTGATCGACCCTGAGACAGGCGGCACCACCT ACAACCAGAAATTCCAGGGCAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 7 | Humanized antibody FS002-A103-H4 L4 light chain coding sequence | GAGATCGTGATGACCCAGTCTCCTCCTACACTGTCTCTGAGCCCT GGCGAGAGAGTGACCCTGTCTTGTAGAGCCTCTGAGAACGTGGG CACCTACGTGTCCTGGTATCAGCAGAAGCCTGGACAGGCCCCTCG GCTGTTGATCTACGGCGCTTCTACCAGAGAGAGCGGCATCCCTGC CAGATTCTCCGGCTCTGGCTCTGGCACAGACTTTACCCTGACAAT CTCCAGCCTGCAGCCTGAGGACTTCGCCGTGTACTACTGCCAGAA CGACTACAGCTACCCCTTCACCTTTGGCGGAGGCACCAAGCTGGA AATCAAGAGGACCGTGGCTGCCCCCAGCGTGTTCATCTTCCCTCC TAGCGACGAGCAGCTGAAGAGCGGCACCGCTAGCGTGGTGTGTC TGCTGAATAACTTCTATCCCAGGGAGGCCAAGGTGCAGTGGAAG GTGGATAACGCCCTGCAGAGCGGCAACTCCCAGGAGTCCGTGAC CGAGCAGGACTCCAAGGACAGCACCTACTCCCTGAGCTCCACCCT GACCCTGTCCAAGGCTGATTATGAGAAGCACAAGGTGTATGCTTG CGAGGTGACACACCAGGGCCTGTCCAGCCCTGTGACCAAGAGCTT CAACCGGGGCGAGTGC |
| 8 | Humanized antibody FS002-A103-H4 L4 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATGCACTGGGTCCGACAGGCTCCAGGACAAGGCTT GGAATGGATGGGCGTGATCGACCCTGAGACAGGCGGCACCACCT ACAACCAGAAATTCCAGGGCAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA |
| | | CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 9 | Chimeric mouse antibody FS002-A153 light chain coding sequence | GACATCGTGATGACCCAGTCTCCATCCAGCCTGACCGTGACCGCT GGCGAGAAAGTGACCATGTCCTGCAAGTCCTCTCAGTCCCTGCTG AACTCCGGCAACCAGAAGAACTACCTGACCTGGTATCAGCAGAA GCCCGGCCAGCCTCCTAAGCTGTTGATCTACGGCGCCTCCACCAG AGAATCTGGCGTGCCCGATAGATTCACCGGCTCTGGCTCTGGCAC CGACTTTACCCTGACCATCAGCTCTGTGCAGGCCGAGGATCTGGC CGTGTACTACTGCCAGAACGACTACAGCTACCCCTTCACCTTTGG CTCCGGCACCAAGCTGGAAATCAAGAGGACCGTGGCTGCCCCCA GCGTGTTCATCTTCCCTCCTAGCGACGAGCAGCTGAAGAGCGGCA CCGCTAGCGTGGTGTGTCTGCTGAATAACTTCTATCCCAGGGAGG CCAAGGTGCAGTGGAAGGTGGATAACGCCCTGCAGAGCGGCAAC TCCCAGGAGTCCGTGACCGAGCAGGACTCCAAGGACAGCACCTA CTCCCTGAGCTCCACCCTGACCCTGTCCAAGGCTGATTATGAGAA GCACAAGGTGTATGCTTGCGAGGTGACACACCAGGGCCTGTCCA GCCCTGTGACCAAGAGCTTCAACCGGGGCGAGTGC |
| 10 | Chimeric mouse antibody FS002-A153 heavy chain coding sequence | CAGGTTCAGTTGCAGCAGTCTGGCGCCGAACTCGTTAGACCTGGC GCTTCTGTGACCCTGTCCTGCAAGGCTTCTGGCTACACCTTCACCG ACTACGAGATGCACTGGGTCAAGCAGACCCCTGTGCATGGCCTGG AATGGATCGGCACCATCGATCCTGAGACAGGCGGCACCACCTAC AACCAGAAGTTCAACGACAAGGCTACCCTGACCGCCGACAAGTC CTCTTCCACCGCCTACATGGACCTGCGGTCCCTGACCTCTGAGGA CTCTGCCGTGTACTACTGCTCCAGATCCGGCTACTACGTGTCCTCC TACGAGTCCTACGCTATGGACTATTGGGGCCAGGGCACCTCCGTG ACAGTCTCTTCTGCTTCCACCAAGGGCCCCTCCGTGTTCCCCCTGG CTCCCTCTTCCAAGAGCACCAGCGGCGGCACCGCTGCTCTGGGAT GTCTGGTGAAGGACTACTTCCCTGAGCCTGTGACCGTGTCCTGGA ATTCCGGCGCCCTGACCTCCGGCGTGCACACATTCCCTGCTGTGC TGCAGTCCTCCGGCCTGTATAGCCTGTCCTCCGTGGTGACAGTGC CTAGCTCCAGCCTGGGCACCCAGACCTATATCTGCAACGTGAACC ACAAGCCTAGCAATACCAAGGTGGACAAGAAGGTGGAGCCTAAG AGCTGCGACAAGACCCACACCTGTCCTCCATGTCCTGCTCCAGAA CTGCTCGGCGGACCTTCCGTGTTCCTGTTTCCTCCAAAGCCTAAGG ACACCCTGATGATCAGCAGAACCCCTGAAGTGACCTGCGTGGTGG TGGATGTGTCCCACGAGGATCCCGAAGTGAAGTTCAATTGGTACG TGGACGGCGTGGAAGTGCACAACGCCAAGACCAAGCCTAGAGAG GAACAGTACAACAGCACCTACAGAGTGGTGTCCGTGCTGACCGT GCTGCACCAGGATTGGCTGAACGGCAAAGAGTACAAGTGCAAGG TGTCCAACAAGGCCCTGCCTGCTCCTATCGAGAAAACCATCAGCA AGGCCAAGGGCCAGCCTAGGGAACCCCAGGTTTACACACTGCCT CCAAGCAGGGACGAGCTGACCAAGAATCAGGTGTCCCTGACCTG CCTGGTCAAGGGCTTCTACCCTTCCGATATCGCCGTGGAATGGGA GAGCAATGGCCAGCCTGAGAACAACTACAAGACAACCCCTCCTG TGCTGGACAGCGACGGCTCATTCTTCCTGTACAGCAAGCTGACAG TGGACAAGAGCAGATGGCAGCAGGGCAACGTGTTCAGCTGCAGC GTGATGCACGAGGCCCTGCACAACCACTACACCCAGAAGTCCCTG AGCCTGTCTCCTGGCAAA |
| 11 | Humanized antibody FS002-A153-H3 L3 light chain coding sequence | GACATCGTGATGACCCAGTCTCCAGACAGCCTGGCTGTGTCTCTG GGCGAGAGAGCCACCATCAACTGCAAGTCCTCTCAGTCCCTGCTG AACTCCGGCAACCAGAAGAACTACCTGACCTGGTATCAGCAGAA GCCCGGCCAGCCTCCTAAGCTGTTGATCTACGGCGCCTCCACCAG AGAATCTGGCGTGCCCGATAGATTCTCCGGCTCTGGCTCTGGCAC CGACTTTACCCTGACAATCAGCTCCCTGCAGGCCGAGGATGTGGC CGTGTACTACTGCCAGAACGACTACAGCTACCCCTTCACCTTTGG CGGAGGCACCAAGCTGGAAATCAAGAGGACCGTGGCTGCCCCCA GCGTGTTCATCTTCCCTCCTAGCGACGAGCAGCTGAAGAGCGGCA CCGCTAGCGTGGTGTGTCTGCTGAATAACTTCTATCCCAGGGAGG CCAAGGTGCAGTGGAAGGTGGATAACGCCCTGCAGAGCGGCAAC TCCCAGGAGTCCGTGACCGAGCAGGACTCCAAGGACAGCACCTA CTCCCTGAGCTCCACCCTGACCCTGTCCAAGGCTGATTATGAGAA GCACAAGGTGTATGCTTGCGAGGTGACACACCAGGGCCTGTCCA GCCCTGTGACCAAGAGCTTCAACCGGGGCGAGTGC |
| 12 | Humanized antibody FS002-A153-H3 L3 heavy chain coding sequence | CAGGTTCAGCTGGTTCAGTCTGGCGCCGAAGTGAAGAAACCTGGC GCCTCCGTGAAGCTGTCCTGCAAGGCTTCTGGCTACACCTTCACC |
| | | GACTACGAGATGCACTGGGTCCGACAGGCTCCAGGACAAGGCCT GGAATGGATCGGCACCATCGATCCTGAGACAGGCGGCACCACCT ACAACCAGAAATTCCAGGGCAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 13 | Humanized antibody FS002-A153-H4 L2 light chain coding sequence | GACATCGTGATGACCCAGTCTCCAGACAGCCTGGCTGTGTCTCTG GGCGAGCGCGTGACCATGAACTGCAAGTCCTCTCAGTCCCTGCTG AACTCCGGCAACCAGAAGAACTACCTGACCTGGTATCAGCAGAA GCCCGGCCAGCCTCCTAAGCTGTTGATCTACGGCGCCTCCACCAG AGAATCTGGCGTGCCCGATAGATTCTCCGGCTCTGGCTCTGGCAC CGACTTTACCCTGACAATCAGCTCCCTGCAGGCCGAGGATGTGGC CGTGTACTACTGCCAGAACGACTACAGCTACCCCTTCACCTTTGG CGGAGGCACCAAGCTGGAAATCAAGAGGACCGTGGCTGCCCCCA GCGTGTTCATCTTCCCTCCTAGCGACGAGCAGCTGAAGAGCGGCA CCGCTAGCGTGGTGTGTCTGCTGAATAACTTCTATCCCAGGGAGG CCAAGGTGCAGTGGAAGGTGGATAACGCCCTGCAGAGCGGCAAC TCCCAGGAGTCCGTGACCGAGCAGGACTCCAAGGACAGCACCTA CTCCCTGAGCTCCACCCTGACCCTGTCCAAGGCTGATTATGAGAA GCACAAGGTGTATGCTTGCGAGGTGACACACCAGGGCCTGTCCA GCCCTGTGACCAAGAGCTTCAACCGGGGCGAGTGC |
| 14 | Humanized antibody FS002-A153-H4 L2 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATGCACTGGGTCCGACAGGCTCCAGGACAAGGCTT GGAGTGGATGGGCACCATCGATCCTGAGACAGGCGGCACCACCT ACGCTCAGAAATTCCAGGGCAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA |
| | | CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 15 | Humanized antibody FS002-A153-H5 L1 light chain coding sequence | GACATCGTGATGACCCAGTCTCCAGACAGCCTGGCTGTGTCTCTG GGCGAGCGAGTGACCATGTCCTGCAAGTCCTCTCAGTCCCTGCTG AACTCCGGCAACCAGAAGAACTACCTGACCTGGTATCAGCAGAA GCCCGGCCAGCCTCCTAAGCTGTTGATCTACGGCGCCTCCACCAG AGAATCTGGCGTGCCCGATAGATTCACCGGCTCTGGCTCTGGCAC CGACTTTACCCTGACAATCAGCTCCCTGCAGGCCGAGGATGTGGC CGTGTACTACTGCCAGAACGACTACAGCTACCCCTTCACCTTTGG CGGAGGCACCAAGCTGGAAATCAAGAGGACCGTGGCTGCCCCCA GCGTGTTCATCTTCCCTCCTAGCGACGAGCAGCTGAAGAGCGGCA CCGCTAGCGTGGTGTGTCTGCTGAATAACTTCTATCCCAGGGAGG CCAAGGTGCAGTGGAAGGTGGATAACGCCCTGCAGAGCGGCAAC TCCCAGGAGTCCGTGACCGAGCAGGACTCCAAGGACAGCACCTA CTCCCTGAGCTCCACCCTGACCCTGTCCAAGGCTGATTATGAGAA GCACAAGGTGTATGCTTGCGAGGTGACACACCAGGGCCTGTCCA GCCCTGTGACCAAGAGCTTCAACCGGGGCGAGTGC |
| 16 | Humanized antibody FS002-A153-H5 L1 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATGCACTGGGTCCGACAGGCTCCAGGACAAGGCTT GGAGTGGATGGGCACCATCGATCCTGAGACAGGCGGCACCACCT ACGCTCAGAAATTCCAGGGCAGAGTGACCATGACCGCCGACACC TCTATCTCCACCGCCTACATGGAACTGTCCCGGCTGAGATCTGAC GACACCGCCGTGTACTACTGTGCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 17 | Humanized antibody FS002-A153-H5 L2 light chain coding sequence | GACATCGTGATGACCCAGTCTCCAGACAGCCTGGCTGTGTCTCTG GGCGAGCGCGTGACCATGAACTGCAAGTCCTCTCAGTCCCTGCTG AACTCCGGCAACCAGAAGAACTACCTGACCTGGTATCAGCAGAA GCCCGGCCAGCCTCCTAAGCTGTTGATCTACGGCGCCTCCACCAG AGAATCTGGCGTGCCCGATAGATTCTCCGGCTCTGGCTCTGGCAC CGACTTTACCCTGACAATCAGCTCCCTGCAGGCCGAGGATGTGGC CGTGTACTACTGCCAGAACGACTACAGCTACCCCTTCACCTTTGG CGGAGGCACCAAGCTGGAAATCAAGAGGACCGTGGCTGCCCCCA GCGTGTTCATCTTCCCTCCTAGCGACGAGCAGCTGAAGAGCGGCA CCGCTAGCGTGGTGTGTCTGCTGAATAACTTCTATCCCAGGGAGG CCAAGGTGCAGTGGAAGGTGGATAACGCCCTGCAGAGCGGCAAC TCCCAGGAGTCCGTGACCGAGCAGGACTCCAAGGACAGCACCTA CTCCCTGAGCTCCACCCTGACCCTGTCCAAGGCTGATTATGAGAA GCACAAGGTGTATGCTTGCGAGGTGACACACCAGGGCCTGTCCA GCCCTGTGACCAAGAGCTTCAACCGGGGCGAGTGC |
| 18 | Humanized antibody FS002-A153-H5 L2 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC |
| | | GACTACGAGATGCACTGGGTCCGACAGGCTCCAGGACAAGGCTT GGAGTGGATGGGCACCATCGATCCTGAGACAGGCGGCACCACCT ACGCTCAGAAATTCCAGGGCAGAGTGACCATGACCGCCGACACC TCTATCTCCACCGCCTACATGGAACTGTCCCGGCTGAGATCTGAC GACACCGCCGTGTACTACTGTGCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 19 | Humanized antibody FS002-A153-H5 L4 light chain coding sequence | GACATCGTGATGACCCAGTCTCCACCTACACTGTCCGTGTCTCCA GGCGAGCGCGTGACCATGTCTTGCAAGTCCTCTCAGTCCCTGCTG AACTCCGGCAACCAGAAGAACTACCTGACCTGGTATCAGCAGAA GCCCGGCCAGGCTCCTAGACTGTTGATCTACGGCGCCTCCACCAG AGAATCTGGCGTGCCCGATAGATTCTCCGGCTCTGGCTCTGGCAC CGACTTTACCCTGACAATCTCCAGCCTGCAGCCTGAGGACTTCGC CGTGTACTACTGCCAGAACGACTACAGCTACCCCTTCACCTTTGG CGGAGGCACCAAGCTGGAAATCAAGAGGACCGTGGCTGCCCCCA GCGTGTTCATCTTCCCTCCTAGCGACGAGCAGCTGAAGAGCGGCA CCGCTAGCGTGGTGTGTCTGCTGAATAACTTCTATCCCAGGGAGG CCAAGGTGCAGTGGAAGGTGGATAACGCCCTGCAGAGCGGCAAC TCCCAGGAGTCCGTGACCGAGCAGGACTCCAAGGACAGCACCTA CTCCCTGAGCTCCACCCTGACCCTGTCCAAGGCTGATTATGAGAA GCACAAGGTGTATGCTTGCGAGGTGACACACCAGGGCCTGTCCA GCCCTGTGACCAAGAGCTTCAACCGGGGCGAGTGC |
| 20 | Humanized antibody FS002-A153-H5 L4 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATGCACTGGGTCCGACAGGCTCCAGGACAAGGCTT GGAGTGGATGGGCACCATCGATCCTGAGACAGGCGGCACCACCT ACGCTCAGAAATTCCAGGGCAGAGTGACCATGACCGCCGACACC TCTATCTCCACCGCCTACATGGAACTGTCCCGGCTGAGATCTGAC GACACCGCCGTGTACTACTGTGCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA |
| | | CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 21 | Humanized antibody FS002-A153-H5 L5 light chain coding sequence | GAGATCGTGATGACCCAGTCTCCTCCTACACTGTCTCTGAGCCCT GGCGAGCGCGTGACCATGTCCTGTAGATCCTCTCAGTCCCTGCTG AACTCCGGCAACCAGAAGAACTACCTGACCTGGTATCAGCAGAA GCCCGGCCAGGCTCCTAGACTGTTGATCTACGGCGCCTCCACCAG AGAATCTGGCGTGCCAGCTAGATTCTCCGGCTCTGGCTCTGGCAC CGACTTTACCCTGACAATCTCCAGCCTGCAGCCTGAGGACTTCGC CGTGTACTACTGCCAGAACGACTACAGCTACCCCTTCACCTTTGG CGGAGGCACCAAGCTGGAAATCAAGAGGACCGTGGCTGCCCCCA GCGTGTTCATCTTCCCTCCTAGCGACGAGCAGCTGAAGAGCGGCA CCGCTAGCGTGGTGTGTCTGCTGAATAACTTCTATCCCAGGGAGG CCAAGGTGCAGTGGAAGGTGGATAACGCCCTGCAGAGCGGCAAC TCCCAGGAGTCCGTGACCGAGCAGGACTCCAAGGACAGCACCTA CTCCCTGAGCTCCACCCTGACCCTGTCCAAGGCTGATTATGAGAA GCACAAGGTGTATGCTTGCGAGGTGACACACCAGGGCCTGTCCA GCCCTGTGACCAAGAGCTTCAACCGGGGCGAGTGC |
| 22 | Humanized antibody FS002-A153-H5 L5 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATGCACTGGGTCCGACAGGCTCCAGGACAAGGCTT GGAGTGGATGGGCACCATCGATCCTGAGACAGGCGGCACCACCT ACGCTCAGAAATTCCAGGGCAGAGTGACCATGACCGCCGACACC TCTATCTCCACCGCCTACATGGAACTGTCCCGGCTGAGATCTGAC GACACCGCCGTGTACTACTGTGCCAGATCCGGCTACTACGTGTCC TCCTACGAGTCCTACGCTATGGACTATTGGGGCCAGGGCACCACC GTGACAGTCTCTTCTGCTTCCACCAAGGGCCCCTCCGTGTTCCCCC TGGCTCCCTCTTCCAAGAGCACCAGCGGCGGCACCGCTGCTCTGG GATGTCTGGTGAAGGACTACTTCCCTGAGCCTGTGACCGTGTCCT GGAATTCCGGCGCCCTGACCTCCGGCGTGCACACATTCCCTGCTG TGCTGCAGTCCTCCGGCCTGTATAGCCTGTCCTCCGTGGTGACAG TGCCTAGCTCCAGCCTGGGCACCCAGACCTATATCTGCAACGTGA ACCACAAGCCTAGCAATACCAAGGTGGACAAGAAGGTGGAGCCT AAGAGCTGCGACAAGACCCACACCTGTCCTCCATGTCCTGCTCCA GAACTGCTCGGCGGACCTTCCGTGTTCCTGTTTCCTCCAAAGCCTA AGGACACCCTGATGATCAGCAGAACCCCTGAAGTGACCTGCGTG GTGGTGGATGTGTCCCACGAGGATCCCGAAGTGAAGTTCAATTGG TACGTGGACGGCGTGGAAGTGCACAACGCCAAGACCAAGCCTAG AGAGGAACAGTACAACAGCACCTACAGAGTGGTGTCCGTGCTGA CCGTGCTGCACCAGGATTGGCTGAACGGCAAAGAGTACAAGTGC AAGGTGTCCAACAAGGCCCTGCCTGCTCCTATCGAGAAAACCATC AGCAAGGCCAAGGGCCAGCCTAGGGAACCCCAGGTTTACACACT GCCTCCAAGCAGGGACGAGCTGACCAAGAATCAGGTGTCCCTGA CCTGCCTGGTCAAGGGCTTCTACCCTTCCGATATCGCCGTGGAAT GGGAGAGCAATGGCCAGCCTGAGAACAACTACAAGACAACCCCT CCTGTGCTGGACAGCGACGGCTCATTCTTCCTGTACAGCAAGCTG ACAGTGGACAAGAGCAGATGGCAGCAGGGCAACGTGTTCAGCTG CAGCGTGATGCACGAGGCCCTGCACAACCACTACACCCAGAAGT CCCTGAGCCTGTCTCCTGGCAAA |
| 23 | Chimeric mouse antibody FS002-A208 light chain coding sequence | GACATCGTGATGACCCAGAGCCAGAAATTCATGTCCACCTCCGTG GGCGACAGAGTGTCCGTGACATGCAAGGCCTCTCAGAACGTGGG CACCAACGTGGCCTGGTATCAGCAGAAGCCTGGCCAGTCTCCTAA GACTCTGATCTACTCCGCCTCCTACCGGTTCTCTGGCGTGCCCGAT AGATTCACCGGCTCTGGATCTGGCACCGACTTCACCCTGACCATC AACAACGTGCAGTCCGAGGACCTGGCCGACTACTTCTGCCAGCAG TACAACATCTACCCTCTGTCCTTCGGCGCTGGCACCAAGCTGGAA CTGAAAAGGACCGTGGCTGCCCCCAGCGTGTTCATCTTCCCTCCT AGCGACGAGCAGCTGAAGAGCGGCACCGCTAGCGTGGTGTGTCT GCTGAATAACTTCTATCCCAGGGAGGCCAAGGTGCAGTGGAAGG TGGATAACGCCCTGCAGAGCGGCAACTCCCAGGAGTCCGTGACC GAGCAGGACTCCAAGGACAGCACCTACTCCCTGAGCTCCACCCTG ACCCTGTCCAAGGCTGATTATGAGAAGCACAAGGTGTATGCTTGC GAGGTGACACACCAGGGCCTGTCCAGCCCTGTGACCAAGAGCTTC AACCGGGGCGAGTGC |
| 24 | Chimeric mouse antibody FS002-A208 heavy chain coding sequence | CAGGTTCAGTTGCAGCAGTCTGGCGCCGAACTCGTTAGACCTGGC GCTTCTGTGACCCTGTCCTGCAAGGCTTCTGGCTACACCTTCACCG |
| | | ACTACGAGATCCACTGGGTCAAGCAGACCCCTGTGCATGGCCTGG AATGGATCGGCGCCATCGATCCTGAAACCGGCGGCACCAACTAC AACCAGAAGTTCAACGACAAGGCTACCCTGACCGCCGACAAGTC CTCTTCCACCGCCTACATGGACCTGCGGTCCCTGACCTCTGAGGA CTCTGCCGTGTACTACTGCTCCAGATCCGGCTACTACGTGTCCAA CTACGAGTCCTACGCTATGGACTACTGGGGCCAGGGCACCTCTGT GACAGTCTCTTCTGCTTCCACCAAGGGCCCCTCCGTGTTCCCCCTG GCTCCCTCTTCCAAGAGCACCAGCGGCGGCACCGCTGCTCTGGGA TGTCTGGTGAAGGACTACTTCCCTGAGCCTGTGACCGTGTCCTGG AATTCCGGCGCCCTGACCTCCGGCGTGCACACATTCCCTGCTGTG CTGCAGTCCTCCGGCCTGTATAGCCTGTCCTCCGTGGTGACAGTG CCTAGCTCCAGCCTGGGCACCCAGACCTATATCTGCAACGTGAAC CACAAGCCTAGCAATACCAAGGTGGACAAGAAGGTGGAGCCTAA GAGCTGCGACAAGACCCACACCTGTCCTCCATGTCCTGCTCCAGA ACTGCTCGGCGGACCTTCCGTGTTCCTGTTTCCTCCAAAGCCTAAG GACACCCTGATGATCAGCAGAACCCCTGAAGTGACCTGCGTGGTG GTGGATGTGTCCCACGAGGATCCCGAAGTGAAGTTCAATTGGTAC GTGGACGGCGTGGAAGTGCACAACGCCAAGACCAAGCCTAGAGA GGAACAGTACAACAGCACCTACAGAGTGGTGTCCGTGCTGACCG TGCTGCACCAGGATTGGCTGAACGGCAAAGAGTACAAGTGCAAG GTGTCCAACAAGGCCCTGCCTGCTCCTATCGAGAAAACCATCAGC AAGGCCAAGGGCCAGCCTAGGGAACCCCAGGTTTACACACTGCC TCCAAGCAGGGACGAGCTGACCAAGAATCAGGTGTCCCTGACCT GCCTGGTCAAGGGCTTCTACCCTTCCGATATCGCCGTGGAATGGG AGAGCAATGGCCAGCCTGAGAACAACTACAAGACAACCCCTCCT GTGCTGGACAGCGACGGCTCATTCTTCCTGTACAGCAAGCTGACA GTGGACAAGAGCAGATGGCAGCAGGGCAACGTGTTCAGCTGCAG CGTGATGCACGAGGCCCTGCACAACCACTACACCCAGAAGTCCCT GAGCCTGTCTCCTGGCAAA |
| 25 | Humanized antibody FS002-A208-H3 L4 light chain coding sequence | GACATCCAGATGACCCAGTCTCCATCCTCTCTGTCCGCCTCTGTGG GCGACAGAGTGACCATCACCTGTCGGGCCTCTCAGAACGTGGGC ACCAATGTGGCTTGGTATCAGCAGAAGCCCGAGAAGGCCCCTAA GAGCCTGATCTACTCCGCCTCCTACCGGTTCTCTGGCGTGCCCTCT AGATTCTCCGGCTCTGGCTCTGGCACCGACTTTACCCTGACAATCT CCAGCCTGCAGCCTGAGGACTTCGCCACCTACTACTGCCAGCAGT ACAACATCTACCCTCTGTCCTTCGGCGGAGGCACCAAGCTGGAAC TGAAAAGGACCGTGGCTGCCCCCAGCGTGTTCATCTTCCCTCCTA GCGACGAGCAGCTGAAGAGCGGCACCGCTAGCGTGGTGTGTCTG CTGAATAACTTCTATCCCAGGGAGGCCAAGGTGCAGTGGAAGGT GGATAACGCCCTGCAGAGCGGCAACTCCCAGGAGTCCGTGACCG AGCAGGACTCCAAGGACAGCACCTACTCCCTGAGCTCCACCCTGA CCCTGTCCAAGGCTGATTATGAGAAGCACAAGGTGTATGCTTGCG AGGTGACACACCAGGGCCTGTCCAGCCCTGTGACCAAGAGCTTCA ACCGGGGCGAGTGC |
| 26 | Humanized antibody FS002-A208-H3 L4 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATCCACTGGGTCCGACAGGCTCCAGGACAAGGCCT GGAATGGATCGGCGCCATCGATCCTGAAACCGGCGGCACCAACT ACAACCAGAAATTCAACGACAGAGTGACCCTGACCGCCGACAAG TCCATCTCCACCGCCTACATGGAACTGTCCCGGCTGAGATCTGAC GACACCGCCGTGTACTACTGCTCCAGATCCGGCTACTACGTGTCC AACTACGAGTCCTACGCTATGGACTACTGGGGCCAGGGCACCAC AGTGACAGTCTCTTCTGCTTCCACCAAGGGCCCCTCCGTGTTCCCC CTGGCTCCCTCTTCCAAGAGCACCAGCGGCGGCACCGCTGCTCTG GGATGTCTGGTGAAGGACTACTTCCCTGAGCCTGTGACCGTGTCC TGGAATTCCGGCGCCCTGACCTCCGGCGTGCACACATTCCCTGCT GTGCTGCAGTCCTCCGGCCTGTATAGCCTGTCCTCCGTGGTGACA GTGCCTAGCTCCAGCCTGGGCACCCAGACCTATATCTGCAACGTG AACCACAAGCCTAGCAATACCAAGGTGGACAAGAAGGTGGAGCC TAAGAGCTGCGACAAGACCCACACCTGTCCTCCATGTCCTGCTCC AGAACTGCTCGGCGGACCTTCCGTGTTCCTGTTTCCTCCAAAGCCT AAGGACACCCTGATGATCAGCAGAACCCCTGAAGTGACCTGCGT GGTGGTGGATGTGTCCCACGAGGATCCCGAAGTGAAGTTCAATTG GTACGTGGACGGCGTGGAAGTGCACAACGCCAAGACCAAGCCTA GAGAGGAACAGTACAACAGCACCTACAGAGTGGTGTCCGTGCTG ACCGTGCTGCACCAGGATTGGCTGAACGGCAAAGAGTACAAGTG CAAGGTGTCCAACAAGGCCCTGCCTGCTCCTATCGAGAAAACCAT CAGCAAGGCCAAGGGCCAGCCTAGGGAACCCCAGGTTTACACAC TGCCTCCAAGCAGGGACGAGCTGACCAAGAATCAGGTGTCCCTG |
| | | ACCTGCCTGGTCAAGGGCTTCTACCCTTCCGATATCGCCGTGGAA TGGGAGAGCAATGGCCAGCCTGAGAACAACTACAAGACAACCCC TCCTGTGCTGGACAGCGACGGCTCATTCTTCCTGTACAGCAAGCT GACAGTGGACAAGAGCAGATGGCAGCAGGGCAACGTGTTCAGCT GCAGCGTGATGCACGAGGCCCTGCACAACCACTACACCCAGAAG TCCCTGAGCCTGTCTCCTGGCAAA |
| 27 | Humanized antibody FS002-A208-H4 L4 light chain coding sequence | GACATCCAGATGACCCAGTCTCCATCCTCTCTGTCCGCCTCTGTGG GCGACAGAGTGACCATCACCTGTCGGGCCTCTCAGAACGTGGGC ACCAATGTGGCTTGGTATCAGCAGAAGCCCGAGAAGGCCCCTAA GAGCCTGATCTACTCCGCCTCCTACCGGTTCTCTGGCGTGCCCTCT AGATTCTCCGGCTCTGGCTCTGGCACCGACTTTACCCTGACAATCT CCAGCCTGCAGCCTGAGGACTTCGCCACCTACTACTGCCAGCAGT ACAACATCTACCCTCTGTCCTTCGGCGGAGGCACCAAGCTGGAAC TGAAAAGGACCGTGGCTGCCCCCAGCGTGTTCATCTTCCCTCCTA GCGACGAGCAGCTGAAGAGCGGCACCGCTAGCGTGGTGTGTCTG CTGAATAACTTCTATCCCAGGGAGGCCAAGGTGCAGTGGAAGGT GGATAACGCCCTGCAGAGCGGCAACTCCCAGGAGTCCGTGACCG AGCAGGACTCCAAGGACAGCACCTACTCCCTGAGCTCCACCCTGA CCCTGTCCAAGGCTGATTATGAGAAGCACAAGGTGTATGCTTGCG AGGTGACACACCAGGGCCTGTCCAGCCCTGTGACCAAGAGCTTCA ACCGGGGCGAGTGC |
| 28 | Humanized antibody FS002-A208-H4 L4 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATCCACTGGGTCCGACAGGCTCCAGGACAAGGCCT GGAATGGATCGGCGCCATCGATCCTGAAACCGGCGGCACCAACT ACAACCAGAAATTCCAGGGCAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC AACTACGAGTCCTACGCTATGGACTACTGGGGCCAGGGCACCAC AGTGACAGTCTCTTCTGCTTCCACCAAGGGCCCCTCCGTGTTCCCC CTGGCTCCCTCTTCCAAGAGCACCAGCGGCGGCACCGCTGCTCTG GGATGTCTGGTGAAGGACTACTTCCCTGAGCCTGTGACCGTGTCC TGGAATTCCGGCGCCCTGACCTCCGGCGTGCACACATTCCCTGCT GTGCTGCAGTCCTCCGGCCTGTATAGCCTGTCCTCCGTGGTGACA GTGCCTAGCTCCAGCCTGGGCACCCAGACCTATATCTGCAACGTG AACCACAAGCCTAGCAATACCAAGGTGGACAAGAAGGTGGAGCC TAAGAGCTGCGACAAGACCCACACCTGTCCTCCATGTCCTGCTCC AGAACTGCTCGGCGGACCTTCCGTGTTCCTGTTTCCTCCAAAGCCT AAGGACACCCTGATGATCAGCAGAACCCCTGAAGTGACCTGCGT GGTGGTGGATGTGTCCCACGAGGATCCCGAAGTGAAGTTCAATTG GTACGTGGACGGCGTGGAAGTGCACAACGCCAAGACCAAGCCTA GAGAGGAACAGTACAACAGCACCTACAGAGTGGTGTCCGTGCTG ACCGTGCTGCACCAGGATTGGCTGAACGGCAAAGAGTACAAGTG CAAGGTGTCCAACAAGGCCCTGCCTGCTCCTATCGAGAAAACCAT CAGCAAGGCCAAGGGCCAGCCTAGGGAACCCCAGGTTTACACAC TGCCTCCAAGCAGGGACGAGCTGACCAAGAATCAGGTGTCCCTG ACCTGCCTGGTCAAGGGCTTCTACCCTTCCGATATCGCCGTGGAA TGGGAGAGCAATGGCCAGCCTGAGAACAACTACAAGACAACCCC TCCTGTGCTGGACAGCGACGGCTCATTCTTCCTGTACAGCAAGCT GACAGTGGACAAGAGCAGATGGCAGCAGGGCAACGTGTTCAGCT GCAGCGTGATGCACGAGGCCCTGCACAACCACTACACCCAGAAG TCCCTGAGCCTGTCTCCTGGCAAA |
| 29 | Humanized antibody FS002-A208-H5 L4 light chain coding sequence | GACATCCAGATGACCCAGTCTCCATCCTCTCTGTCCGCCTCTGTGG GCGACAGAGTGACCATCACCTGTCGGGCCTCTCAGAACGTGGGC ACCAATGTGGCTTGGTATCAGCAGAAGCCCGAGAAGGCCCCTAA GAGCCTGATCTACTCCGCCTCCTACCGGTTCTCTGGCGTGCCCTCT AGATTCTCCGGCTCTGGCTCTGGCACCGACTTTACCCTGACAATCT CCAGCCTGCAGCCTGAGGACTTCGCCACCTACTACTGCCAGCAGT ACAACATCTACCCTCTGTCCTTCGGCGGAGGCACCAAGCTGGAAC TGAAAAGGACCGTGGCTGCCCCCAGCGTGTTCATCTTCCCTCCTA GCGACGAGCAGCTGAAGAGCGGCACCGCTAGCGTGGTGTGTCTG CTGAATAACTTCTATCCCAGGGAGGCCAAGGTGCAGTGGAAGGT GGATAACGCCCTGCAGAGCGGCAACTCCCAGGAGTCCGTGACCG AGCAGGACTCCAAGGACAGCACCTACTCCCTGAGCTCCACCCTGA CCCTGTCCAAGGCTGATTATGAGAAGCACAAGGTGTATGCTTGCG AGGTGACACACCAGGGCCTGTCCAGCCCTGTGACCAAGAGCTTCA ACCGGGGCGAGTGC |
| 30 | Humanized antibody FS002-A208-H5 L4 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC |
| | | GACTACGAGATCCACTGGGTCCGACAGGCTCCAGGACAAGGCTT GGAATGGATGGGCGCCATCGATCCTGAGACAGGCGGCACCAATT ACGCCCAGAAATTCCAGGGCAGAGTGACCATGACCGCCGACACC TCTATCTCCACCGCCTACATGGAACTGTCCCGGCTGAGATCTGAC GACACCGCCGTGTACTACTGCTCCAGATCCGGCTACTACGTGTCC AACTACGAGTCCTACGCTATGGACTACTGGGGCCAGGGCACCAC AGTGACAGTCTCTTCTGCTTCCACCAAGGGCCCCTCCGTGTTCCCC CTGGCTCCCTCTTCCAAGAGCACCAGCGGCGGCACCGCTGCTCTG GGATGTCTGGTGAAGGACTACTTCCCTGAGCCTGTGACCGTGTCC TGGAATTCCGGCGCCCTGACCTCCGGCGTGCACACATTCCCTGCT GTGCTGCAGTCCTCCGGCCTGTATAGCCTGTCCTCCGTGGTGACA GTGCCTAGCTCCAGCCTGGGCACCCAGACCTATATCTGCAACGTG AACCACAAGCCTAGCAATACCAAGGTGGACAAGAAGGTGGAGCC TAAGAGCTGCGACAAGACCCACACCTGTCCTCCATGTCCTGCTCC AGAACTGCTCGGCGGACCTTCCGTGTTCCTGTTTCCTCCAAAGCCT AAGGACACCCTGATGATCAGCAGAACCCCTGAAGTGACCTGCGT GGTGGTGGATGTGTCCCACGAGGATCCCGAAGTGAAGTTCAATTG GTACGTGGACGGCGTGGAAGTGCACAACGCCAAGACCAAGCCTA GAGAGGAACAGTACAACAGCACCTACAGAGTGGTGTCCGTGCTG ACCGTGCTGCACCAGGATTGGCTGAACGGCAAAGAGTACAAGTG CAAGGTGTCCAACAAGGCCCTGCCTGCTCCTATCGAGAAAACCAT CAGCAAGGCCAAGGGCCAGCCTAGGGAACCCCAGGTTTACACAC TGCCTCCAAGCAGGGACGAGCTGACCAAGAATCAGGTGTCCCTG ACCTGCCTGGTCAAGGGCTTCTACCCTTCCGATATCGCCGTGGAA TGGGAGAGCAATGGCCAGCCTGAGAACAACTACAAGACAACCCC TCCTGTGCTGGACAGCGACGGCTCATTCTTCCTGTACAGCAAGCT GACAGTGGACAAGAGCAGATGGCAGCAGGGCAACGTGTTCAGCT GCAGCGTGATGCACGAGGCCCTGCACAACCACTACACCCAGAAG TCCCTGAGCCTGTCTCCTGGCAAA |
| 31 | Chimeric mouse antibody FS002-A226 light chain coding sequence | GACATCAAGATCACCCAGTCTCCATCCTCTATGTACGCCAGCCTG GGCGAGAGAGTGACCATTACCTGCAAGGCCAGCCAGGACATCAA GTCCTACCTGTCCTGGTATCAGCAGAAGCCCGGCAGCTCTCCCAG ACTGCTGATCTACGACACCTCCAACCTGGCCTCTGGCGTGCCAGT CAGATTTTCCGGCAGAGGCTCTGGCACCTCCTACAGCCTGACCAT CTCCAGAATGGAAGCCGAGGATGCCGCCACCTACTACTGTCAGCA GTGGTCTAGCTACCCCTTCACCTTTGGCTCCGGCACCAAGCTGGA AATCAAGAGGACCGTGGCTGCCCCCAGCGTGTTCATCTTCCCTCC TAGCGACGAGCAGCTGAAGAGCGGCACCGCTAGCGTGGTGTGTC TGCTGAATAACTTCTATCCCAGGGAGGCCAAGGTGCAGTGGAAG GTGGATAACGCCCTGCAGAGCGGCAACTCCCAGGAGTCCGTGAC CGAGCAGGACTCCAAGGACAGCACCTACTCCCTGAGCTCCACCCT GACCCTGTCCAAGGCTGATTATGAGAAGCACAAGGTGTATGCTTG CGAGGTGACACACCAGGGCCTGTCCAGCCCTGTGACCAAGAGCTT CAACCGGGGCGAGTGC |
| 32 | Chimeric mouse antibody FS002-A226 heavy chain coding sequence | CAGTTGCAGTTGCAGCAGTCTGGCGCCGAACTCGTTAGACCTGGC GCTTCTGTGACCCTGTCCTGCAAGGCTTCTGGCTACACCTTCACCG ACTACGAGATCCACTGGGTCAAGCAGACCCCTGTGCATGGCCTGG AATGGATCGGCGCCATCGATCCTGAAACCGGCGGCACCAACTAC AACCAGAAGTTCAACGACAAGGCTACCCTGACCGCCGACAAGTC CTCTTCCACCGCCTACATGGACCTGCGGTCCCTGACCTCTGAGGA CTCTGCCGTGTACTACTGCTCCAGATCCGGCTACTACGTGTCCAA CTACGAGTCCTACGCTATGGACTACTGGGGCCAGGGCACCTCTGT GACAGTCTCTTCTGCTTCCACCAAGGGCCCCTCCGTGTTCCCCCTG GCTCCCTCTTCCAAGAGCACCAGCGGCGGCACCGCTGCTCTGGGA TGTCTGGTGAAGGACTACTTCCCTGAGCCTGTGACCGTGTCCTGG AATTCCGGCGCCCTGACCTCCGGCGTGCACACATTCCCTGCTGTG CTGCAGTCCTCCGGCCTGTATAGCCTGTCCTCCGTGGTGACAGTG CCTAGCTCCAGCCTGGGCACCCAGACCTATATCTGCAACGTGAAC CACAAGCCTAGCAATACCAAGGTGGACAAGAAGGTGGAGCCTAA GAGCTGCGACAAGACCCACACCTGTCCTCCATGTCCTGCTCCAGA ACTGCTCGGCGGACCTTCCGTGTTCCTGTTTCCTCCAAAGCCTAAG GACACCCTGATGATCAGCAGAACCCCTGAAGTGACCTGCGTGGTG GTGGATGTGTCCCACGAGGATCCCGAAGTGAAGTTCAATTGGTAC GTGGACGGCGTGGAAGTGCACAACGCCAAGACCAAGCCTAGAGA GGAACAGTACAACAGCACCTACAGAGTGGTGTCCGTGCTGACCG TGCTGCACCAGGATTGGCTGAACGGCAAAGAGTACAAGTGCAAG GTGTCCAACAAGGCCCTGCCTGCTCCTATCGAGAAAACCATCAGC AAGGCCAAGGGCCAGCCTAGGGAACCCCAGGTTTACACACTGCC TCCAAGCAGGGACGAGCTGACCAAGAATCAGGTGTCCCTGACCT |
| | | GCCTGGTCAAGGGCTTCTACCCTTCCGATATCGCCGTGGAATGGG AGAGCAATGGCCAGCCTGAGAACAACTACAAGACAACCCCTCCT GTGCTGGACAGCGACGGCTCATTCTTCCTGTACAGCAAGCTGACA GTGGACAAGAGCAGATGGCAGCAGGGCAACGTGTTCAGCTGCAG CGTGATGCACGAGGCCCTGCACAACCACTACACCCAGAAGTCCCT GAGCCTGTCTCCTGGCAAA |
| 33 | Humanized antibody FS002-A226-H3 L4 light chain coding sequence | GACATCCAGCTGACCCAGTCTCCATCCTCTCTGTCTGCCTCTGTGG GCGACAGAGTGACCATCACCTGTAGAGCCAGCCAGGACATCAAG TCCTACCTGTCCTGGTATCAGCAGAAGCCCGGCAAGGCTCCCAAG CTGCTGATCTACGACACCTCCAATCTGGCCTCTGGCGTGCCCTCTA GATTCTCCGGTTCTGGCTCTGGCACCGACTTTACCCTGACAATCTC CAGCCTGCAGCCTGAGGACTTCGCCACCTACTACTGTCAGCAGTG GTCTAGCTACCCCTTCACCTTTGGCGGAGGCACCAAGCTGGAAAT CAAGAGGACCGTGGCTGCCCCCAGCGTGTTCATCTTCCCTCCTAG CGACGAGCAGCTGAAGAGCGGCACCGCTAGCGTGGTGTGTCTGC TGAATAACTTCTATCCCAGGGAGGCCAAGGTGCAGTGGAAGGTG GATAACGCCCTGCAGAGCGGCAACTCCCAGGAGTCCGTGACCGA GCAGGACTCCAAGGACAGCACCTACTCCCTGAGCTCCACCCTGAC CCTGTCCAAGGCTGATTATGAGAAGCACAAGGTGTATGCTTGCGA GGTGACACACCAGGGCCTGTCCAGCCCTGTGACCAAGAGCTTCAA CCGGGGCGAGTGC |
| 34 | Humanized antibody FS002-A226-H3 L4 heavy chain coding sequence | CAGCTGCAGTTGGTTCAGTCTGGCGCCGAAGTGAAGAAACCTGGC GCCTCTGTGAAGGTGTCCTGCAAGGCTTCTGGCTACACCTTTACC GACTACGAGATCCACTGGGTCCGACAGGCTCCAGGACAAGGCCT GGAATGGATCGGCGCCATCGATCCTGAAACCGGCGGCACCAACT ACAACCAGAAATTCAACGACAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC AACTACGAGTCCTACGCTATGGACTACTGGGGCCAGGGCACCAC AGTGACAGTCTCTTCTGCTTCCACCAAGGGCCCCTCCGTGTTCCCC CTGGCTCCCTCTTCCAAGAGCACCAGCGGCGGCACCGCTGCTCTG GGATGTCTGGTGAAGGACTACTTCCCTGAGCCTGTGACCGTGTCC TGGAATTCCGGCGCCCTGACCTCCGGCGTGCACACATTCCCTGCT GTGCTGCAGTCCTCCGGCCTGTATAGCCTGTCCTCCGTGGTGACA GTGCCTAGCTCCAGCCTGGGCACCCAGACCTATATCTGCAACGTG AACCACAAGCCTAGCAATACCAAGGTGGACAAGAAGGTGGAGCC TAAGAGCTGCGACAAGACCCACACCTGTCCTCCATGTCCTGCTCC AGAACTGCTCGGCGGACCTTCCGTGTTCCTGTTTCCTCCAAAGCCT AAGGACACCCTGATGATCAGCAGAACCCCTGAAGTGACCTGCGT GGTGGTGGATGTGTCCCACGAGGATCCCGAAGTGAAGTTCAATTG GTACGTGGACGGCGTGGAAGTGCACAACGCCAAGACCAAGCCTA GAGAGGAACAGTACAACAGCACCTACAGAGTGGTGTCCGTGCTG ACCGTGCTGCACCAGGATTGGCTGAACGGCAAAGAGTACAAGTG CAAGGTGTCCAACAAGGCCCTGCCTGCTCCTATCGAGAAAACCAT CAGCAAGGCCAAGGGCCAGCCTAGGGAACCCCAGGTTTACACAC TGCCTCCAAGCAGGGACGAGCTGACCAAGAATCAGGTGTCCCTG ACCTGCCTGGTCAAGGGCTTCTACCCTTCCGATATCGCCGTGGAA TGGGAGAGCAATGGCCAGCCTGAGAACAACTACAAGACAACCCC TCCTGTGCTGGACAGCGACGGCTCATTCTTCCTGTACAGCAAGCT GACAGTGGACAAGAGCAGATGGCAGCAGGGCAACGTGTTCAGCT GCAGCGTGATGCACGAGGCCCTGCACAACCACTACACCCAGAAG TCCCTGAGCCTGTCTCCTGGCAAA |
| 35 | Humanized antibody FS002-A226-H4 L3 light chain coding sequence | GACATCCAGATCACCCAGTCTCCATCCTCCATGTCCGCCTCTGTG GGCGACAGAGTGACCATCACATGCAAGGCCAGCCAGGACATCAA GTCCTACCTGTCCTGGTATCAGCAGAAGCCCGGCAAGGCTCCCAA GCTGCTGATCTACGACACCTCCAATCTGGCCTCTGGCGTGCCCTCT AGATTCTCCGGTTCTGGCTCTGGCACCGACTATACCCTGACAATC TCCAGCCTGCAGCCTGAGGACTTCGCCACCTACTACTGTCAGCAG TGGTCTAGCTACCCCTTCACCTTTGGCGGAGGCACCAAGCTGGAA ATCAAGAGGACCGTGGCTGCCCCCAGCGTGTTCATCTTCCCTCCT AGCGACGAGCAGCTGAAGAGCGGCACCGCTAGCGTGGTGTGTCT GCTGAATAACTTCTATCCCAGGGAGGCCAAGGTGCAGTGGAAGG TGGATAACGCCCTGCAGAGCGGCAACTCCCAGGAGTCCGTGACC GAGCAGGACTCCAAGGACAGCACCTACTCCCTGAGCTCCACCCTG ACCCTGTCCAAGGCTGATTATGAGAAGCACAAGGTGTATGCTTGC GAGGTGACACACCAGGGCCTGTCCAGCCCTGTGACCAAGAGCTTC AACCGGGGCGAGTGC |
| 36 | Humanized antibody FS002-A226-H4 L3 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC |
| | | GACTACGAGATCCACTGGGTCCGACAGGCTCCAGGACAAGGCCT GGAATGGATCGGCGCCATCGATCCTGAAACCGGCGGCACCAACT ACAACCAGAAATTCCAGGGCAGAGTGACCCTGACCGCCGACACC TCTATCTCCACCGCCTACATGGAACTGTCCCGGCTGAGATCTGAC GACACCGCCGTGTACTACTGCTCCAGATCCGGCTACTACGTGTCC AACTACGAGTCCTACGCTATGGACTACTGGGGCCAGGGCACCAC AGTGACAGTCTCTTCTGCTTCCACCAAGGGCCCCTCCGTGTTCCCC CTGGCTCCCTCTTCCAAGAGCACCAGCGGCGGCACCGCTGCTCTG GGATGTCTGGTGAAGGACTACTTCCCTGAGCCTGTGACCGTGTCC TGGAATTCCGGCGCCCTGACCTCCGGCGTGCACACATTCCCTGCT GTGCTGCAGTCCTCCGGCCTGTATAGCCTGTCCTCCGTGGTGACA GTGCCTAGCTCCAGCCTGGGCACCCAGACCTATATCTGCAACGTG AACCACAAGCCTAGCAATACCAAGGTGGACAAGAAGGTGGAGCC TAAGAGCTGCGACAAGACCCACACCTGTCCTCCATGTCCTGCTCC AGAACTGCTCGGCGGACCTTCCGTGTTCCTGTTTCCTCCAAAGCCT AAGGACACCCTGATGATCAGCAGAACCCCTGAAGTGACCTGCGT GGTGGTGGATGTGTCCCACGAGGATCCCGAAGTGAAGTTCAATTG GTACGTGGACGGCGTGGAAGTGCACAACGCCAAGACCAAGCCTA GAGAGGAACAGTACAACAGCACCTACAGAGTGGTGTCCGTGCTG ACCGTGCTGCACCAGGATTGGCTGAACGGCAAAGAGTACAAGTG CAAGGTGTCCAACAAGGCCCTGCCTGCTCCTATCGAGAAAACCAT CAGCAAGGCCAAGGGCCAGCCTAGGGAACCCCAGGTTTACACAC TGCCTCCAAGCAGGGACGAGCTGACCAAGAATCAGGTGTCCCTG ACCTGCCTGGTCAAGGGCTTCTACCCTTCCGATATCGCCGTGGAA TGGGAGAGCAATGGCCAGCCTGAGAACAACTACAAGACAACCCC TCCTGTGCTGGACAGCGACGGCTCATTCTTCCTGTACAGCAAGCT GACAGTGGACAAGAGCAGATGGCAGCAGGGCAACGTGTTCAGCT GCAGCGTGATGCACGAGGCCCTGCACAACCACTACACCCAGAAG TCCCTGAGCCTGTCTCCTGGCAAA |
| 37 | Humanized antibody FS002-A226-H5 L3 light chain coding sequence | GACATCCAGATCACCCAGTCTCCATCCTCCATGTCCGCCTCTGTG GGCGACAGAGTGACCATCACATGCAAGGCCAGCCAGGACATCAA GTCCTACCTGTCCTGGTATCAGCAGAAGCCCGGCAAGGCTCCCAA GCTGCTGATCTACGACACCTCCAATCTGGCCTCTGGCGTGCCCTCT AGATTCTCCGGTTCTGGCTCTGGCACCGACTATACCCTGACAATC TCCAGCCTGCAGCCTGAGGACTTCGCCACCTACTACTGTCAGCAG TGGTCTAGCTACCCCTTCACCTTTGGCGGAGGCACCAAGCTGGAA ATCAAGAGGACCGTGGCTGCCCCCAGCGTGTTCATCTTCCCTCCT AGCGACGAGCAGCTGAAGAGCGGCACCGCTAGCGTGGTGTGTCT GCTGAATAACTTCTATCCCAGGGAGGCCAAGGTGCAGTGGAAGG TGGATAACGCCCTGCAGAGCGGCAACTCCCAGGAGTCCGTGACC GAGCAGGACTCCAAGGACAGCACCTACTCCCTGAGCTCCACCCTG ACCCTGTCCAAGGCTGATTATGAGAAGCACAAGGTGTATGCTTGC GAGGTGACACACCAGGGCCTGTCCAGCCCTGTGACCAAGAGCTTC AACCGGGGCGAGTGC |
| 38 | Humanized antibody FS002-A226-H5 L3 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATCCACTGGGTCCGACAGGCTCCAGGACAAGGCTT GGAATGGATGGGCGCCATCGATCCTGAGACAGGCGGCACCAATT ACGCCCAGAAATTCCAGGGCAGAGTGACCATGACCGCCGACACC TCTATCTCCACCGCCTACATGGAACTGTCCCGGCTGAGATCTGAC GACACCGCCGTGTACTACTGCTCCAGATCCGGCTACTACGTGTCC AACTACGAGTCCTACGCTATGGACTACTGGGGCCAGGGCACCAC AGTGACAGTCTCTTCTGCTTCCACCAAGGGCCCCTCCGTGTTCCCC CTGGCTCCCTCTTCCAAGAGCACCAGCGGCGGCACCGCTGCTCTG GGATGTCTGGTGAAGGACTACTTCCCTGAGCCTGTGACCGTGTCC TGGAATTCCGGCGCCCTGACCTCCGGCGTGCACACATTCCCTGCT GTGCTGCAGTCCTCCGGCCTGTATAGCCTGTCCTCCGTGGTGACA GTGCCTAGCTCCAGCCTGGGCACCCAGACCTATATCTGCAACGTG AACCACAAGCCTAGCAATACCAAGGTGGACAAGAAGGTGGAGCC TAAGAGCTGCGACAAGACCCACACCTGTCCTCCATGTCCTGCTCC AGAACTGCTCGGCGGACCTTCCGTGTTCCTGTTTCCTCCAAAGCCT AAGGACACCCTGATGATCAGCAGAACCCCTGAAGTGACCTGCGT GGTGGTGGATGTGTCCCACGAGGATCCCGAAGTGAAGTTCAATTG GTACGTGGACGGCGTGGAAGTGCACAACGCCAAGACCAAGCCTA GAGAGGAACAGTACAACAGCACCTACAGAGTGGTGTCCGTGCTG ACCGTGCTGCACCAGGATTGGCTGAACGGCAAAGAGTACAAGTG CAAGGTGTCCAACAAGGCCCTGCCTGCTCCTATCGAGAAAACCAT CAGCAAGGCCAAGGGCCAGCCTAGGGAACCCCAGGTTTACACAC TGCCTCCAAGCAGGGACGAGCTGACCAAGAATCAGGTGTCCCTG |
| | | ACCTGCCTGGTCAAGGGCTTCTACCCTTCCGATATCGCCGTGGAA TGGGAGAGCAATGGCCAGCCTGAGAACAACTACAAGACAACCCC TCCTGTGCTGGACAGCGACGGCTCATTCTTCCTGTACAGCAAGCT GACAGTGGACAAGAGCAGATGGCAGCAGGGCAACGTGTTCAGCT GCAGCGTGATGCACGAGGCCCTGCACAACCACTACACCCAGAAG TCCCTGAGCCTGTCTCCTGGCAAA |
| 39 | Humanized antibody FS002-A226-H5 L4 light chain coding sequence | GACATCCAGCTGACCCAGTCTCCATCCTCTCTGTCTGCCTCTGTGG GCGACAGAGTGACCATCACCTGTAGAGCCAGCCAGGACATCAAG TCCTACCTGTCCTGGTATCAGCAGAAGCCCGGCAAGGCTCCCAAG CTGCTGATCTACGACACCTCCAATCTGGCCTCTGGCGTGCCCTCTA GATTCTCCGGTTCTGGCTCTGGCACCGACTTTACCCTGACAATCTC CAGCCTGCAGCCTGAGGACTTCGCCACCTACTACTGTCAGCAGTG GTCTAGCTACCCCTTCACCTTTGGCGGAGGCACCAAGCTGGAAAT CAAGAGGACCGTGGCTGCCCCCAGCGTGTTCATCTTCCCTCCTAG CGACGAGCAGCTGAAGAGCGGCACCGCTAGCGTGGTGTGTCTGC TGAATAACTTCTATCCCAGGGAGGCCAAGGTGCAGTGGAAGGTG GATAACGCCCTGCAGAGCGGCAACTCCCAGGAGTCCGTGACCGA GCAGGACTCCAAGGACAGCACCTACTCCCTGAGCTCCACCCTGAC CCTGTCCAAGGCTGATTATGAGAAGCACAAGGTGTATGCTTGCGA GGTGACACACCAGGGCCTGTCCAGCCCTGTGACCAAGAGCTTCAA CCGGGGCGAGTGC |
| 40 | Humanized antibody FS002-A226-H5 L4 heavy chain coding sequence | CAGGTTCAGCTGGTTCAGTCTGGCGCCGAAGTGAAGAAACCTGGC GCCTCTGTGAAGGTGTCCTGCAAGGCTTCTGGCTACACCTTTACC GACTACGAGATCCACTGGGTCCGACAGGCTCCAGGACAAGGCTT GGAATGGATGGGCGCCATCGATCCTGAGACAGGCGGCACCAATT ACGCCCAGAAATTCCAGGGCAGAGTGACCATGACCGCCGACACC TCTATCTCCACCGCCTACATGGAACTGTCCCGGCTGAGATCTGAC GACACCGCCGTGTACTACTGCTCCAGATCCGGCTACTACGTGTCC AACTACGAGTCCTACGCTATGGACTACTGGGGCCAGGGCACCAC AGTGACAGTCTCTTCTGCTTCCACCAAGGGCCCCTCCGTGTTCCCC CTGGCTCCCTCTTCCAAGAGCACCAGCGGCGGCACCGCTGCTCTG GGATGTCTGGTGAAGGACTACTTCCCTGAGCCTGTGACCGTGTCC TGGAATTCCGGCGCCCTGACCTCCGGCGTGCACACATTCCCTGCT GTGCTGCAGTCCTCCGGCCTGTATAGCCTGTCCTCCGTGGTGACA GTGCCTAGCTCCAGCCTGGGCACCCAGACCTATATCTGCAACGTG AACCACAAGCCTAGCAATACCAAGGTGGACAAGAAGGTGGAGCC TAAGAGCTGCGACAAGACCCACACCTGTCCTCCATGTCCTGCTCC AGAACTGCTCGGCGGACCTTCCGTGTTCCTGTTTCCTCCAAAGCCT AAGGACACCCTGATGATCAGCAGAACCCCTGAAGTGACCTGCGT GGTGGTGGATGTGTCCCACGAGGATCCCGAAGTGAAGTTCAATTG GTACGTGGACGGCGTGGAAGTGCACAACGCCAAGACCAAGCCTA GAGAGGAACAGTACAACAGCACCTACAGAGTGGTGTCCGTGCTG ACCGTGCTGCACCAGGATTGGCTGAACGGCAAAGAGTACAAGTG CAAGGTGTCCAACAAGGCCCTGCCTGCTCCTATCGAGAAAACCAT CAGCAAGGCCAAGGGCCAGCCTAGGGAACCCCAGGTTTACACAC TGCCTCCAAGCAGGGACGAGCTGACCAAGAATCAGGTGTCCCTG ACCTGCCTGGTCAAGGGCTTCTACCCTTCCGATATCGCCGTGGAA TGGGAGAGCAATGGCCAGCCTGAGAACAACTACAAGACAACCCC TCCTGTGCTGGACAGCGACGGCTCATTCTTCCTGTACAGCAAGCT GACAGTGGACAAGAGCAGATGGCAGCAGGGCAACGTGTTCAGCT GCAGCGTGATGCACGAGGCCCTGCACAACCACTACACCCAGAAG TCCCTGAGCCTGTCTCCTGGCAAA |

**Table 2: Amino acid sequences (SEQ ID NO: 41-200) of the variable regions and CDRs of the light and heavy chains of the antibody according to the present invention**

| No. | Description | Amino Acid Sequence |
|---|---|---|
| 41 | Chimeric mouse antibody FS002-A103 light chain variable region | DIVMTQSPKSMSMSVGERVTLSCKASENVGTYVSWYQQK PEQSPKLLIYGASTRESGVPDRFTGSGSGTDFTLTISSVQAE DLAVYYCONDYSYPFTFGSGTKLEIK |
| 42 | Chimeric mouse antibody FS002-A103 VL CDR1 | ***KASENVGTYVS*** |
| 43 | Chimeric mouse antibody FS002-A103 VL CDR2 | ***GASTRES*** |
| 44 | Chimeric mouse antibody FS002-A103 VL CDR3 | ***ONDYSYPFT*** |
| 45 | Chimeric mouse antibody FS002-A103 heavy chain | QVQLQQSGAELVRPGASVTLSCKASGYTFTDYEMHWVK QTPVHGLEWIGVIDPETGGTTYNQKFNDKATLTADKSSST |
| | variable region | AYMDLRSLTSEDSAVYYCSRSGYYVSSYESYAMDYWGQG TSVTVSS |
| 46 | Chimeric mouse antibody FS002-A103 VH CDR1 | ***GYTFTDYEMH*** |
| 47 | Chimeric mouse antibody FS002-A103 VH CDR2 | ***VIDPETGGTT*** |
| 48 | Chimeric mouse antibody FS002-A103 VH CDR3 | ***SGYYVSSYESYAMDY*** |
| 49 | Humanized antibody FS002-A103-H4L2 light chain variable region | EIVMTQSPKTMSMSPGERVTLSCKASENVGTYVSWYQQK PGQAPRLLIYGASTRESGVPDRFSGSGSGTDFTLTISSLQPE DFAVYYCONDYSYPFTFGGGTKLEIK |
| 50 | Humanized antibody FS002-A103-H4L2 VL CDR1 | ***KASENVGTYVS*** |
| 51 | Humanized antibody FS002-A103-H4L2 VL CDR2 | ***GASTRES*** |
| 52 | Humanized antibody FS002-A103-H4L2 VL CDR3 | ***ONDYSYPFT*** |
| 53 | Humanized antibody FS002-A103-H4L2 heavy chain variable region | QVQLVQSGAEVKKPGASVKVSCKASGYTFTDYEMHWVR QAPGQGLEWMGVIDPETGGTTYNQKFQGRVTLTADTSIS TAYMELSRLRSDDTAVYYCSRSGYYVSSYESYAMDYWGQ GTTVTVSS |
| 54 | Humanized antibody FS002-A103-H4L2 VH CDR1 | ***GYTFTDYEMH*** |
| 55 | Humanized antibody FS002-A103-H4L2 VH CDR2 | ***VIDPETGGTT*** |
| 56 | Humanized antibody FS002-A103-H4L2 VH CDR3 | ***SGYYVSSYESYAMDY*** |
| 57 | Humanized antibody FS002-A103-H4L3 light chain variable region | EIVMTQSPPTMSMSPGERVTLSCKASENVGTYVSWYQQKP GQAPRLLIYGASTRESGVPARFSGSGSGTDFTLTISSLQPED FAVYYCQNDYSYPFTFGGGTKLEIK |
| 58 | Humanized antibody FS002-A103-H4L3 VL CDR1 | ***KASENVGTYVS*** |
| 59 | Humanized antibody FS002-A103-H4L3 VL CDR2 | ***GASTRES*** |
| 60 | Humanized antibody FS002-A103-H4L3 VL CDR3 | ***ONDYSYPFT*** |
| 61 | Humanized antibody FS002-A103-H4L3 heavy chain variable region | QVQLVQSGAEVKKPGASVKVSCKASGYTFTDYEMHWVR QAPGQGLEWMGVIDPETGGTTYNQKFQGRVTLTADTSIS TAYMELSRLRSDDTAVYYCSRSGYYVSSYESYAMDYWGQ GTTVTVSS |
| 62 | Humanized antibody FS002-A103-H4L3 VH CDR1 | ***GYTFTDYEMH*** |
| 63 | Humanized antibody FS002-A103-H4L3 VH CDR2 | ***VIDPETGGTT*** |
| 64 | Humanized antibody FS002-A103-H4L3 VH CDR3 | ***SGYYVSSYESYAMDY*** |
| 65 | Humanized antibody FS002-A103-H4L4 light chain variable region | EIVMTQSPPTLSLSPGERVTLSCRASENVGTYVSWYQQKP GQAPRLLIYGASTRESGIPARFSGSGSGTDFTLTISSLQPED FAVYYCQNDYSYPFTFGGGTKLEIK |
| 66 | Humanized antibody FS002-A103-H4L4 VL CDR1 | ***RASENVGTYVS*** |
| 67 | Humanized antibody FS002-A103-H4L4 VL CDR2 | ***GASTRES*** |
| 68 | Humanized antibody FS002-A103-H4L4 VL CDR3 | ***ONDYSYPFT*** |
| 69 | Humanized antibody FS002-A103-H4L4 heavy chain variable region | QVQLVQSGAEVKKPGASVKVSCKASGYTFTDYEMHWVR QAPGQGLEWMGVIDPETGGTTYNQKFQGRVTLTADTSIS TAYMELSRLRSDDTAVYYCSRSGYYVSSYESYAMDYWGQ GTTVTVSS |
| 70 | Humanized antibody FS002-A103-H4L4 VH CDR1 | ***GYTFTDYEMH*** |
| 71 | Humanized antibody FS002-A103-H4L4 VH CDR2 | ***VIDPETGGTT*** |
| 72 | Humanized antibody FS002-A103-H4L4 VH CDR3 | ***SGYYVSSYESYAMDY*** |
| 73 | Chimeric mouse antibody FS002-A153 light chain variable region | DIVMTQSPSSLTVTAGEKVTMSCKSSQSLLNSGNQKNYLT WYQQKPGQPPKLLIYGASTRESGVPDRFTGSGSGTDFTLTI SSVQAEDLAVYYCQNDYSYPFTFGSGTKLEIK |
| 74 | Chimeric mouse antibody FS002-A153 VL CDR1 | ***KSSOSLLNSGNOKNYLT*** |
| 75 | Chimeric mouse antibody FS002-A153 VL CDR2 | ***GASTRES*** |
| 76 | Chimeric mouse antibody FS002-A153 VL CDR3 | ***ONDYSYPFT*** |
| 77 | Chimeric mouse antibody FS002-A153 heavy chain variable region | QVQLQQSGAELVRPGASVTLSCKASGYTFTDYEMHWVK QTPVHGLEWIGTIDPETGGTTYNQKFNDKATLTADKSSST AYMDLRSLTSEDSAVYYCSRSGYYVSSYESYAMDYWGQG TSVTVSS |
| 78 | Chimeric mouse antibody FS002-A153 VH CDR1 | ***GYTFTDYEMH*** |
| 79 | Chimeric mouse antibody FS002-A153 VH CDR2 | ***TIDPETGGTT*** |
| 80 | Chimeric mouse antibody FS002-A153 VH CDR3 | ***SGYYVSSYESYAMDY*** |
| 81 | Humanized antibody FS002-A153-H3L3 light chain variable region | DIVMTQSPDSLAVSLGERATINCKSSQSLLNSGNQKNYLT WYQQKPGQPPKLLIYGASTRESGVPDRFSGSGSGTDFTLTI SSLQAEDVAVYYCONDYSYPFTFGGGTKLEIK |
| 82 | Humanized antibody FS002-A153-H3L3 VL CDR1 | ***KSSOSLLNSGNOKNYLT*** |
| 83 | Humanized antibody FS002-A153-H3L3 VL CDR2 | ***GASTRES*** |
| 84 | Humanized antibody FS002-A153-H3L3 VL CDR3 | ***ONDYSYPFT*** |
| 85 | Humanized antibody FS002-A153-H3L3 heavy chain variable region | QVQLVQSGAEVKKPGASVKLSCKASGYTFTDYEMHWVR QAPGQGLEWIGTIDPETGGTTYNQKFQGRVTLTADTSIST AYMELSRLRSDDTAVYYCSRSGYYVSSYESYAMDYWGQG TTVTVSS |
| 86 | Humanized antibody FS002-A153-H3L3 VH CDR1 | ***GYTFTDYEMH*** |
| 87 | Humanized antibody FS002-A153-H3L3 VH CDR2 | ***TIDPETGGTT*** |
| 88 | Humanized antibody FS002-A153-H3L3 VH CDR3 | ***SGYYVSSYESYAMDY*** |
| 89 | Humanized antibody FS002-A153-H4L2 light chain variable region | DIVMTQSPDSLAVSLGERVTMNCKSSQSLLNSGNQKNYLT WYQQKPGQPPKLLIYGASTRESGVPDRFSGSGSGTDFTLTI SSLQAEDVAVYYCQNDYSYPFTFGGGTKLEIK |
| 90 | Humanized antibody FS002-A153-H4L2 VL CDR1 | ***KSSOSLLNSGNOKNYLT*** |
| 91 | Humanized antibody FS002-A153-H4L2 VL CDR2 | ***GASTRES*** |
| 92 | Humanized antibody FS002-A153-H4L2 VL CDR3 | ***ONDYSYPFT*** |
| 93 | Humanized antibody FS002-A153-H4L2 heavy chain variable region | QVQLVQSGAEVKKPGASVKVSCKASGYTFTDYEMHWVR QAPGQGLEWMGTIDPETGGTTYAQKFQGRVTLTADTSIS TAYMELSRLRSDDTAVYYCSRSGYYVSSYESYAMDYWGQ GTTVTVSS |
| 94 | Humanized antibody FS002-A153-H4L2 VH CDR1 | ***GYTFTDYEMH*** |
| 95 | Humanized antibody FS002-A153-H4L2 VH CDR2 | ***TIDPETGGTT*** |
| 96 | Humanized antibody FS002-A153-H4L2 VH CDR3 | ***SGYYVSSYESYAMDY*** |
| 97 | Humanized antibody FS002-A153-H5L1 light chain variable region | DIVMTQSPDSLAVSLGERVTMSCKSSQSLLNSGNQKNYLT WYQQKPGQPPKLLIYGASTRESGVPDRFTGSGSGTDFTLTI SSLQAEDVAVYYCONDYSYPFTFGGGTKLEIK |
| 98 | Humanized antibody FS002-A153-H5L1 VL CDR1 | ***KSSOSLLNSGNOKNYLT*** |
| 99 | Humanized antibody FS002-A153-H5L1 VL CDR2 | ***GASTRES*** |
| 100 | Humanized antibody FS002-A153-H5L1 VL CDR3 | ***ONDYSYPFT*** |
| 101 | Humanized antibody FS002-A153-H5L1 heavy chain variable region | QVQLVQSGAEVKKPGASVKVSCKASGYTFTDYEMHWVR QAPGQGLEWMGTIDPETGGTTYAQKFQGRVTMTADTSIS TAYMELSRLRSDDTAVYYCARSGYYVSSYESYAMDYWGQ GTTVTVSS |
| 102 | Humanized antibody FS002-A153-H5L1 VH CDR1 | ***GYTFTDYEMH*** |
| 103 | Humanized antibody FS002-A153-H5L1 VH CDR2 | ***TIDPETGGTT*** |
| 104 | Humanized antibody FS002-A153-H5L1 VH CDR3 | ***SGYYVSSYESYAMD*** |
| 105 | Humanized antibody FS002-A153-H5L2 light chain variable region | DIVMTQSPDSLAVSLGERVTMNCKSSQSLLNSGNQKNYLT WYQQKPGQPPKLLIYGASTRESGVPDRFSGSGSGTDFTLTI SSLQAEDVAVYYCQNDYSYPFTFGGGTKLEIK |
| 106 | Humanized antibody FS002-A153-H5L2 VL CDR1 | ***KSSOSLLNSGNOKNYLT*** |
| 107 | Humanized antibody FS002-A153-H5L2 VL CDR2 | ***GASTRES*** |
| 108 | Humanized antibody FS002-A153-H5L2 VL CDR3 | ***ONDYSYPFT*** |
| 109 | Humanized antibody FS002-A153-H5L2 heavy chain variable region | QVQLVQSGAEVKKPGASVKVSCKASGYTFTDYEMHWVR QAPGQGLEWMGTIDPETGGTTYAQKFQGRVTMTADTSIS TAYMELSRLRSDDTAVYYCARSGYYVSSYESYAMDYWGQ GTTVTVSS |
| 110 | Humanized antibody FS002-A153-H5L2 VH CDR1 | ***GYTFTDYEMH*** |
| 111 | Humanized antibody FS002-A153-H5L2 VH CDR2 | ***TIDPETGGTT*** |
| 112 | Humanized antibody FS002-A153-H5L2 VH CDR3 | ***SGYYVSSYESYAMD*** |
| 113 | Humanized antibody FS002-A153-H5L4 light chain variable region | DIVMTQSPPTLSVSPGERVTMSCKSSQSLLNSGNQKNYLT WYQQKPGQAPRLLIYGASTRESGVPDRFSGSGSGTDFTLTI SSLQPEDFAVYYCQNDYSYPFTFGGGTKLEIK |
| 114 | Humanized antibody FS002-A153-H5L4 VL CDR1 | ***KSSOSLLNSGNOKNYLT*** |
| 115 | Humanized antibody FS002-A153-H5L4 VL CDR2 | ***GASTRES*** |
| 116 | Humanized antibody FS002-A153-H5L4 VL CDR3 | ***ONDYSYPFT*** |
| 117 | Humanized antibody FS002-A153-H5L4 heavy chain variable region | QVQLVQSGAEVKKPGASVKVSCKASGYTFTDYEMHWVR QAPGQGLEWMGTIDPETGGTTYAQKFQGRVTMTADTSIS TAYMELSRLRSDDTAVYYCARSGYYVSSYESYAMDYWGQ GTTVTVSS |
| 118 | Humanized antibody FS002-A153-H5L4 VH CDR1 | ***GYTFTDYEMH*** |
| 119 | Humanized antibody FS002-A153-H5L4 VH CDR2 | ***TIDPETGGTT*** |
| 120 | Humanized antibody FS002-A153-H5L4 VH CDR3 | ***SGYYVSSYESYAMD*** |
| 121 | Humanized antibody FS002-A153-H5L5 light chain variable region | EIVMTQSPPTLSLSPGERVTMSCRSSQSLLNSGNQKNYLT WYQQKPGQAPRLLIYGASTRESGVPARFSGSGSGTDFTLTI SSLQPEDFAVYYCQNDYSYPFTFGGGTKLEIK |
| 122 | Humanized antibody FS002-A153-H5L5 VL CDR1 | ***RSSOSLLNSGNOKNYLT*** |
| 123 | Humanized antibody FS002-A153-H5L5 VL CDR2 | ***GASTRES*** |
| 124 | Humanized antibody FS002-A153-H5L5 VL CDR3 | ***ONDYSYPFT*** |
| 125 | Humanized antibody FS002-A153-H5L5 heavy chain variable region | QVQLVQSGAEVKKPGASVKVSCKASGYTFTDYEMHWVR QAPGQGLEWMGTIDPETGGTTYAQKFQGRVTMTADTSIS TAYMELSRLRSDDTAVYYCARSGYYVSSYESYAMDYWGQ GTTVTVSS |
| 126 | Humanized antibody FS002-A153-H5L5 VH CDR1 | ***GYTFTDYEMH*** |
| 127 | Humanized antibody FS002-A153-H5L5 VH CDR2 | ***TIDPETGGTT*** |
| 128 | Humanized antibody FS002-A153-H5L5 VH CDR3 | ***SGYYVSSYESYAMD*** |
| 129 | Chimeric mouse antibody FS002-A208 light chain variable region | DIVMTQSQKFMSTSVGDRVSVTCKASQNVGTNVAWYQQ KPGQSPKTLIYSASYRFSGVPDRFTGSGSGTDFTLTINNVQ SEDLADYFCQQYNIYPLSFGAGTKLELK |
| 130 | Chimeric mouse antibody FS002-A208 VL CDR1 | ***KASONVGTNVA*** |
| 131 | Chimeric mouse antibody FS002-A208 VL CDR2 | ***SASYRFS*** |
| 132 | Chimeric mouse antibody FS002-A208 VL CDR3 | ***OOYNIYPLS*** |
| 133 | Chimeric mouse antibody FS002-A208 heavy chain variable region | QVQLQQSGAELVRPGASVTLSCKASGYTFTDYEIHWVKQ TPVHGLEWIGAIDPETGGTNYNQKFNDKATLTADKSSSTA YMDLRSLTSEDSAVYYCSRSGYYVSNYESYAMDYWGQGT SVTVSS |
| 134 | Chimeric mouse antibody FS002-A208 VH CDR1 | ***GYTFTDYEIH*** |
| 135 | Chimeric mouse antibody FS002-A208 VH CDR2 | ***AIDPETGGTN*** |
| 136 | Chimeric mouse antibody FS002-A208 VH CDR3 | ***SGYYVSNYESYAMDY*** |
| 137 | Humanized antibody FS002-A208-H3L4 light chain variable region | DIQMTQSPSSLSASVGDRVTITCRASQNVGTNVAWYQQKP EKAPKSLIYSASYRFSGVPSRFSGSGSGTDFTLTISSLQPED FATYYCQQYNIYPLSFGGGTKLELK |
| 138 | Humanized antibody FS002-A208-H3L4 VL CDR1 | ***RASONVGTNVA*** |
| 139 | Humanized antibody FS002-A208-H3L4 VL CDR2 | ***SASYRFS*** |
| 140 | Humanized antibody FS002-A208-H3L4 VL CDR3 | ***OOYNIYPLS*** |
| 141 | Humanized antibody FS002-A208-H3L4 heavy chain variable region | QVQLVQSGAEVKKPGASVKVSCKASGYTFTDYEIHWVR QAPGQGLEWIGAIDPETGGTNYNQKFNDRVTLTADKSIST AYMELSRLRSDDTAVYYCSRSGYYVSNYESYAMDYWGQG TTVTVSS |
| 142 | Humanized antibody FS002-A208-H3L4 VH CDR1 | ***GYTFTDYEIH*** |
| 143 | Humanized antibody FS002-A208-H3L4 VH CDR2 | ***AIDPETGGTN*** |
| 144 | Humanized antibody FS002-A208-H3L4 VH CDR3 | ***SGYYVSNYESYAMDY*** |
| 145 | Humanized antibody FS002-A208-H4L4 light chain variable region | DIQMTQSPSSLSASVGDRVTITCRASQNVGTNVAWYQQKP EKAPKSLIYSASYRFSGVPSRFSGSGSGTDFTLTISSLQPED FATYYCQQYNIYPLSFGGGTKLELK |
| 146 | Humanized antibody FS002-A208-H4L4 VL CDR1 | ***RASONVGTNVA*** |
| 147 | Humanized antibody FS002-A208-H4L4 VL CDR2 | ***SASYRFS*** |
| 148 | Humanized antibody FS002-A208-H4L4 VL CDR3 | ***OOYNIYPLS*** |
| 149 | Humanized antibody FS002-A208-H4L4 heavy chain variable region | QVQLVQSGAEVKKPGASVKVSCKASGYTFTDYEIHWVR QAPGQGLEWIGAIDPETGGTNYNQKFQGRVTLTADTSIST AYMELSRLRSDDTAVYYCSRSGYYVSNYESYAMDYWGQG TTVTVSS |
| 150 | Humanized antibody FS002-A208-H4L4 VH CDR1 | ***GYTFTDYEIH*** |
| 151 | Humanized antibody FS002-A208-H4L4 VH CDR2 | ***AIDPETGGTN*** |
| 152 | Humanized antibody FS002-A208-H4L4 VH CDR3 | ***SGYYVSNYESYAMDY*** |
| 153 | Humanized antibody FS002-A208-H5L4 light chain variable region | DIQMTQSPSSLSASVGDRVTITCRASQNVGTNVAWYQQKP EKAPKSLIYSASYRFSGVPSRFSGSGSGTDFTLTISSLQPED FATYYCQQYNIYPLSFGGGTKLELK |
| 154 | Humanized antibody FS002-A208-H5L4 VL CDR1 | ***RASONVGTNVA*** |
| 155 | Humanized antibody FS002-A208-H5L4 VL CDR2 | ***SASYRFS*** |
| 156 | Humanized antibody FS002-A208-H5L4 VL | ***OOYNIYPLS*** |
| | CDR3 | |
| 157 | Humanized antibody FS002-A208-H5L4 heavy chain variable region | QVQLVQSGAEVKKPGASVKVSCKASGYTFTDYEIHWVR QAPGQGLEWMGAIDPETGGTNYAQKFQGRVTMTADTSIS TAYMELSRLRSDDTAVYYCSRSGYYVSNYESYAMDYWGQ GTTVTVSS |
| 158 | Humanized antibody FS002-A208-H5L4 VH CDR1 | ***GYTFTDYEIH*** |
| 159 | Humanized antibody FS002-A208-H5L4 VH CDR2 | ***AIDPETGGTN*** |
| 160 | Humanized antibody FS002-A208-H5L4 VH CDR3 | ***SGYYVSNYESYAMDY*** |
| 161 | Chimeric mouse antibody FS002-A226 light chain variable region | DIKITQSPSSMYASLGERVTITCKASQDIKSYLSWYQQKPG SSPRLLIYDTSNLASGVPVRFSGRGSGTSYSLTISRMEAED AATYYCQQWSSYPFTFGSGTKLEIK |
| 162 | Chimeric mouse antibody FS002-A226 VL CDR1 | ***KASQDIKSYLS*** |
| 163 | Chimeric mouse antibody FS002-A226 VL CDR2 | ***DTSNLAS*** |
| 164 | Chimeric mouse antibody FS002-A226 VL CDR3 | ***OOWSSYPFT*** |
| 165 | Chimeric mouse antibody FS002-A226 heavy chain variable region | QLQLQQSGAELVRPGASVTLSCKASGYTFTDYEIHWVKQ TPVHGLEWIGAIDPETGGTNYNQKFNDKATLTADKSSSTA YMDLRSLTSEDSAVYYCSRSGYYVSNYESYAMDYWGQGT SVTVSS |
| 166 | Chimeric mouse antibody FS002-A226 VH CDR1 | ***GYTFTDYEIH*** |
| 167 | Chimeric mouse antibody FS002-A226 VH CDR2 | ***AIDPETGGTN*** |
| 168 | Chimeric mouse antibody FS002-A226 VH CDR3 | ***SGYYVSNYESYAMDY*** |
| 169 | Humanized antibody FS002-A226-H3L4 light chain variable region | DIQLTQSPSSLSASVGDRVTITCRASQDIKSYLSWYQQKPG KAPKLLIYDTSNLASGVPSRFSGSGSGTDFTLTISSLQPEDF ATYYCOOWSSYPFTFGGGTKLEIK |
| 170 | Humanized antibody FS002-A226-H3L4 VL CDR1 | ***RASODIKSYLS*** |
| 171 | Humanized antibody FS002-A226-H3L4 VL CDR2 | ***DTSNLAS*** |
| 172 | Humanized antibody FS002-A226-H3L4 VL CDR3 | ***OOWSSYPFT*** |
| 173 | Humanized antibody FS002-A226-H3L4 heavy chain variable region | QLQLVQSGAEVKKPGASVKVSCKASGYTFTDYEIHWVRQ APGQGLEWIGAIDPETGGTNYNQKFNDRVTLTADTSISTA YMELSRLRSDDTAVYYCSRSGYYVSNYESYAMDYWGQGT TVTVSS |
| 174 | Humanized antibody FS002-A226-H3L4 VH CDR1 | ***GYTFTDYEIH*** |
| 175 | Humanized antibody FS002-A226-H3L4 VH CDR2 | ***AIDPETGGTN*** |
| 176 | Humanized antibody FS002-A226-H3L4 VH CDR3 | ***SGYYVSNYESYAMDY*** |
| 177 | Humanized antibody FS002-A226-H4L3 light chain variable region | DIQITQSPSSMSASVGDRVTITCKASQDIKSYLSWYQQKPG KAPKLLIYDTSNLASGVPSRFSGSGSGTDYTLTISSLQPEDF ATYYCQQWSSYPFTFGGGTKLEIK |
| 178 | Humanized antibody FS002-A226-H4L3 VL CDR1 | ***KASQDIKSYLS*** |
| 179 | Humanized antibody FS002-A226-H4L3 VL CDR2 | ***DTSNLAS*** |
| 180 | Humanized antibody FS002-A226-H4L3 VL CDR3 | ***OOWSSYPFT*** |
| 181 | Humanized antibody FS002-A226-H4L3 heavy chain variable region | QVQLVQSGAEVKKPGASVKVSCKASGYTFTDYEIHWVR QAPGQGLEWIGAIDPETGGTNYNQKFQGRVTLTADTSIST AYMELSRLRSDDTAVYYCSRSGYYVSNYESYAMDYWGQG TTVTVSS |
| 182 | Humanized antibody FS002-A226-H4L3 VH CDR1 | ***GYTFTDYEIH*** |
| 183 | Humanized antibody FS002-A226-H4L3 VH CDR2 | ***AIDPETGGTN*** |
| 184 | Humanized antibody FS002-A226-H4L3 VH CDR3 | ***SGYYVSNYESYAMDY*** |
| 185 | Humanized antibody FS002-A226-H5L3 light chain variable region | DIQITQSPSSMSASVGDRVTITCKASQDIKSYLSWYQQKPG KAPKLLIYDTSNLASGVPSRFSGSGSGTDYTLTISSLQPEDF ATYYCOOWSSYPFTFGGGTKLEIK |
| 186 | Humanized antibody FS002-A226-H5L3 VL CDR1 | ***KASQDIKSYLS*** |
| 187 | Humanized antibody FS002-A226-H5L3 VL CDR2 | ***DTSNLAS*** |
| 188 | Humanized antibody FS002-A226-H5L3 VL CDR3 | ***OOWSSYPFT*** |
| 189 | Humanized antibody FS002-A226-H5L3 heavy chain variable region | QVQLVQSGAEVKKPGASVKVSCKASGYTFTDYEIHWVR QAPGQGLEWMGAIDPETGGTNYAQKFQGRVTMTADTSIS TAYMELSRLRSDDTAVYYCSRSGYYVSNYESYAMDYWGQ GTTVTVSS |
| 190 | Humanized antibody FS002-A226-H5L3 VH CDR1 | ***GYTFTDYEIH*** |
| 191 | Humanized antibody FS002-A226-H5L3 VH CDR2 | ***AIDPETGGTN*** |
| 192 | Humanized antibody FS002-A226-H5L3 VH CDR3 | ***SGYYVSNYESYAMDY*** |
| 193 | Humanized antibody FS002-A226-H5L4 light chain variable region | DIQLTQSPSSLSASVGDRVTITCRASQDIKSYLSWYQQKPG KAPKLLIYDTSNLASGVPSRFSGSGSGTDFTLTISSLQPEDF ATYYCQQWSSYPFTFGGGTKLEIK |
| 194 | Humanized antibody FS002-A226-H5L4 VL CDR1 | ***RASODIKSYLS*** |
| 195 | Humanized antibody FS002-A226-H5L4 VL CDR2 | ***DTSNLAS*** |
| 196 | Humanized antibody FS002-A226-H5L4 VL CDR3 | ***OOWSSYPFT*** |
| 197 | Humanized antibody FS002-A226-H5L4 heavy chain variable region | QVQLVQSGAEVKKPGASVKVSCKASGYTFTDYEIHWVR QAPGQGLEWMGAIDPETGGTNYAQKFQGRVTMTADTSIS TAYMELSRLRSDDTAVYYCSRSGYYVSNYESYAMDYWGQ GTTVTVSS |
| 198 | Humanized antibody FS002-A226-H5L4 VH CDR1 | ***GYTFTDYEIH*** |
| 199 | Humanized antibody FS002-A226-H5L4 VH CDR2 | ***AIDPETGGTN*** |
| 200 | Humanized antibody FS002-A226-H5L4 VH CDR3 | ***SGYYVSNYESYAMDY*** |

| | | |
|---|---|---|
| Note: The bold and underlined parts of amino acid sequences in the table are the corresponding CDR sequences. | | |

**Table 3: Amino acid sequences (SEQ ID NO: 201-202) of the light chain constant region and the heavy chain constant region of the antibody according to the present invention**

| No. | Description | Amino Acid Sequence |
|---|---|---|
| 201 | Kappa type light chain constant region | |
| 202 | IgG1 type heavy chain constant region | |

**Table 4: Amino acid sequences (SEQ ID NO: 203-242) of the light and heavy chains of the antibody according to the present invention**

| No. | Description | Amino Acid Sequence |
|---|---|---|
| 203 | Chimeric mouse antibody FS002-A103 light chain | DIVMTQSPKSMSMSVGERVTLSCKASENVGTYVSWYQQKPEQSPKLLIY GASTRESGVPDRFTGSGSGTDFTLTISSVQAEDLAVYYCQNDYSYPFTFG SGTKLEIKRTVAAPSVFIFPPSDEQLKSGTASVVCLLNNFYPREAKVQWK VDNALQSGNSQESVTEQDSKDSTYSLSSTLTLSKADYEKHKVYACEVTH QGLSSPVTKSFNRGEC |
| 204 | Chimeric mouse antibody FS002-A103 heavy chain | QVQLQQSGAELVRPGASVTLSCKASGYTFTDYEMHWVKQTPVHGLEWI GVIDPETGGTTYNQKFNDKATLTADKSSSTAYMDLRSLTSEDSAVYYCS RSGYYVSSYESYAMDYWGQGTSVTVSSASTKGPSVFPLAPSSKSTSGGTA ALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGK |
| 205 | Humanized antibody FS002-A103-H4L 2 light chain | EIVMTQSPKTMSMSPGERVTLSCKASENVGTYVSWYQQKPGQAPRLLIY GASTRESGVPDRFSGSGSGTDFTLTISSLQPEDFAVYYCQNDYSYPFTFGG GTKLEIKRTVAAPSVFIFPPSDEQLKSGTASVVCLLNNFYPREAKVQWKV DNALQSGNSQESVTEQDSKDSTYSLSSTLTLSKADYEKHKVYACEVTHQ GLSSPVTKSFNRGEC |
| 206 | Humanized antibody FS002-A103-H4L 2 heavy chain | QVQLVQSGAEVKKPGASVKVSCKASGYTFTDYEMHWVRQAPGQGLEW MGVIDPETGGTTYNQKFQGRVTLTADTSISTAYMELSRLRSDDTAVYYC SRSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 207 | Humanized antibody FS002-A103-H4L 3 light chain | EIVMTQSPPTMSMSPGERVTLSCKASENVGTYVSWYQQKPGQAPRLLIYG ASTRESGVPARFSGSGSGTDFTLTISSLQPEDFAVYYCQNDYSYPFTFGGG TKLEIKRTVAAPSVFIFPPSDEQLKSGTASVVCLLNNFYPREAKVQWKVD NALQSGNSQESVTEQDSKDSTYSLSSTLTLSKADYEKHKVYACEVTHQG LSSPVTKSFNRGEC |
| 208 | Humanized antibody FS002-A103-H4L 3 heavy chain | QVQLVQSGAEVKKPGASVKVSCKASGYTFTDYEMHWVRQAPGQGLEW MGVIDPETGGTTYNQKFQGRVTLTADTSISTAYMELSRLRSDDTAVYYC SRSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA |
| | | KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 209 | Humanized antibody FS002-A103-H4L 4 light chain | EIVMTQSPPTLSLSPGERVTLSCRASENVGTYVSWYQQKPGQAPRLLIYG ASTRESGIPARFSGSGSGTDFTLTISSLQPEDFAVYYCQNDYSYPFTFGGG TKLEIKRTVAAPSVFIFPPSDEQLKSGTASVVCLLNNFYPREAKVQWKVD NALQSGNSQESVTEQDSKDSTYSLSSTLTLSKADYEKHKVYACEVTHQG LSSPVTKSFNRGEC |
| 210 | Humanized antibody FS002-A103-H4L 4 heavy chain | QVQLVQSGAEVKKPGASVKVSCKASGYTFTDYEMHWVRQAPGQGLEW MGVIDPETGGTTYNQKFQGRVTLTADTSISTAYMELSRLRSDDTAVYYC SRSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 211 | Chimeric mouse antibody FS002-A153 light chain | DIVMTQSPSSLTVTAGEKVTMSCKSSQSLLNSGNQKNYLTWYQQKPGQP PKLLIYGASTRESGVPDRFTGSGSGTDFTLTISSVQAEDLAVYYCQNDYSY PFTFGSGTKLEIKRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC |
| 212 | Chimeric mouse antibody FS002-A153 heavy chain | QVQLQQSGAELVRPGASVTLSCKASGYTFTDYEMHWVKQTPVHGLEWI GTIDPETGGTTYNQKFNDKATLTADKSSSTAYMDLRSLTSEDSAVYYCS RSGYYVSSYESYAMDYWGQGTSVTVSSASTKGPSVFPLAPSSKSTSGGTA ALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGK |
| 213 | Humanized antibody FS002-A153-H3L 3 light chain | DIVMTQSPDSLAVSLGERATINCKSSQSLLNSGNQKNYLTWYQQKPGQPP KLLIYGASTRESGVPDRFSGSGSGTDFTLTISSLQAEDVAVYYCQNDYSYP FTFGGGTKLEIKRTVAAPSVFIFPPSDEQLKSGTASVVCLLNNFYPREAKV QWKVDNALQSGNSQESVTEQDSKDSTYSLSSTLTLSKADYEKHKVYACE VTHQGLSSPVTKSFNRGEC |
| 214 | Humanized antibody FS002-A153-H3L 3 heavy chain | QVQLVQSGAEVKKPGASVKLSCKASGYTFTDYEMHWVRQAPGQGLEWI GTIDPETGGTTYNQKFQGRVTLTADTSISTAYMELSRLRSDDTAVYYCSR SGYYVSSYESYAMDYWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYT QKSLSLSPGK |
| 215 | Humanized antibody FS002-A153-H4L 2 light chain | DIVMTQSPDSLAVSLGERVTMNCKSSQSLLNSGNQKNYLTWYQQKPGQP PKLLIYGASTRESGVPDRFSGSGSGTDFTLTISSLQAEDVAVYYCQNDYSY PFTFGGGTKLEIKRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC |
| 216 | Humanized antibody FS002-A153-H4L 2 heavy chain | QVQLVQSGAEVKKPGASVKVSCKASGYTFTDYEMHWVRQAPGQGLEW MGTIDPETGGTTYAQKFQGRVTLTADTSISTAYMELSRLRSDDTAVYYC SRSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 217 | Humanized antibody FS002-A153-H5L 1 light chain | DIVMTQSPDSLAVSLGERVTMSCKSSQSLLNSGNQKNYLTWYQQKPGQP PKLLIYGASTRESGVPDRFTGSGSGTDFTLTISSLQAEDVAVYYCQNDYSY PFTFGGGTKLEIKRTVAAPSVFIFPPSDEQLKSGTASVVCLLNNFYPREAK VQWKVDNALQSGNSQESVTEQDSKDSTYSLSSTLTLSKADYEKHKVYA |
| | | CEVTHQGLSSPVTKSFNRGEC |
| 218 | Humanized antibody FS002-A153-H5L 1 heavy chain | QVQLVQSGAEVKKPGASVKVSCKASGYTFTDYEMHWVRQAPGQGLEW MGTIDPETGGTTYAQKFQGRVTMTADTSISTAYMELSRLRSDDTAVYYC ARSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 219 | Humanized antibody FS002-A153-H5L 2 light chain | DIVMTQSPDSLAVSLGERVTMNCKSSQSLLNSGNQKNYLTWYQQKPGQP PKLLIYGASTRESGVPDRFSGSGSGTDFTLTISSLQAEDVAVYYCQNDYSY PFTFGGGTKLEIKRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC |
| 220 | Humanized antibody FS002-A153-H5L 2 heavy chain | QVQLVQSGAEVKKPGASVKVSCKASGYTFTDYEMHWVRQAPGQGLEW MGTIDPETGGTTYAQKFQGRVTMTADTSISTAYMELSRLRSDDTAVYYC ARSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 221 | Humanized antibody FS002-A153-H5L 4 light chain | DIVMTQSPPTLSVSPGERVTMSCKSSQSLLNSGNQKNYLTWYQQKPGQA PRLLIYGASTRESGVPDRFSGSGSGTDFTLTISSLQPEDFAVYYCQNDYSY PFTFGGGTKLEIKRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC |
| 222 | Humanized antibody FS002-A153-H5L 4 heavy chain | QVQLVQSGAEVKKPGASVKVSCKASGYTFTDYEMHWVRQAPGQGLEW MGTIDPETGGTTYAQKFQGRVTMTADTSISTAYMELSRLRSDDTAVYYC ARSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 223 | Humanized antibody FS002-A153-H5L 5 light chain | EIVMTQSPPTLSLSPGERVTMSCRSSQSLLNSGNQKNYLTWYQQKPGQAP RLLIYGASTRESGVPARFSGSGSGTDFTLTISSLQPEDFAVYYCQNDYSYP FTFGGGTKLEIKRTVAAPSVFIFPPSDEQLKSGTASVVCLLNNFYPREAKV QWKVDNALQSGNSQESVTEQDSKDSTYSLSSTLTLSKADYEKHKVYACE VTHQGLSSPVTKSFNRGEC |
| 224 | Humanized antibody FS002-A153-H5L 5 heavy chain | QVQLVQSGAEVKKPGASVKVSCKASGYTFTDYEMHWVRQAPGQGLEW MGTIDPETGGTTYAQKFQGRVTMTADTSISTAYMELSRLRSDDTAVYYC ARSGYYVSSYESYAMDYWGQGTTVTVSSASTKGPSVFPLAPSSKSTSGGT AALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 225 | Chimeric mouse antibody FS002-A208 light chain | DIVMTQSQKFMSTSVGDRVSVTC*KASQNVGTNVA*WYQQKPGQSPKTLIY *SASYRFS*GVPDRFTGSGSGTDFTLTINNVQSEDLADYFC*QQYNIYPLS*FGA GTKLELKRTVAAPSVFIFPPSDEQLKSGTASVVCLLNNFYPREAKVQWK VDNALQSGNSQESVTEQDSKDSTYSLSSTLTLSKADYEKHKVYACEVTH QGLSSPVTKSFNRGEC |
| 226 | Chimeric mouse antibody FS002-A208 heavy chain | QVQLQQSGAELVRPGASVTLSCKASGYTFTDYEIHWVKQTPVHGLEWIG AIDPETGGTNYNQKFNDKATLTADKSSSTAYMDLRSLTSEDSAVYYCSR SGYYVSNYESYAMDYWGQGTSVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKA |
| | | KGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYT QKSLSLSPGK |
| 227 | Humanized antibody FS002-A208-H3L 4 light chain | DIQMTQSPSSLSASVGDRVTITCRASQNVGTNVAWYQQKPEKAPKSLIYS ASYRFSGVPSRFSGSGSGTDFTLTISSLQPEDFATYYCQQYNIYPLSFGGGT KLELKRTVAAPSVFIFPPSDEQLKSGTASVVCLLNNFYPREAKVQWKVD NALQSGNSQESVTEQDSKDSTYSLSSTLTLSKADYEKHKVYACEVTHQG LSSPVTKSFNRGEC |
| 228 | Humanized antibody FS002-A208-H3L 4 heavy chain | QVQLVQSGAEVKKPGASVKVSCKASGYTFTDYEIHWVRQAPGQGLEWI GAIDPETGGTNYNQKFNDRVTLTADKSISTAYMELSRLRSDDTAVYYCS RSGYYVSNYESYAMDYWGQGTTVTVSSASTKGPSVFPLAPSSKSTSGGTA ALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGK |
| 229 | Humanized antibody FS002-A208-H4L 4 light chain | DIQMTQSPSSLSASVGDRVTITCRASQNVGTNVAWYQQKPEKAPKSLIYS ASYRFSGVPSRFSGSGSGTDFTLTISSLQPEDFATYYCQQYNIYPLSFGGGT KLELKRTVAAPSVFIFPPSDEQLKSGTASVVCLLNNFYPREAKVQWKVD NALQSGNSQESVTEQDSKDSTYSLSSTLTLSKADYEKHKVYACEVTHQG LSSPVTKSFNRGEC |
| 230 | Humanized antibody FS002-A208-H4L 4 heavy chain | QVQLVQSGAEVKKPGASVKVSCKASGYTFTDYEIHWVRQAPGQGLEWI GAIDPETGGTNYNQKFQGRVTLTADTSISTAYMELSRLRSDDTAVYYCS RSGYYVSNYESYAMDYWGQGTTVTVSSASTKGPSVFPLAPSSKSTSGGTA ALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGK |
| 231 | Humanized antibody FS002-A208-H5L 4 light chain | DIQMTQSPSSLSASVGDRVTITCRASQNVGTNVAWYQQKPEKAPKSLIYS ASYRFSGVPSRFSGSGSGTDFTLTISSLQPEDFATYYCQQYNIYPLSFGGGT KLELKRTVAAPSVFIFPPSDEQLKSGTASVVCLLNNFYPREAKVQWKVD NALQSGNSQESVTEQDSKDSTYSLSSTLTLSKADYEKHKVYACEVTHQG LSSPVTKSFNRGEC |
| 232 | Humanized antibody FS002-A208-H5L 4 heavy chain | QVQLVQSGAEVKKPGASVKVSCKASGYTFTDYEIHWVRQAPGQGLEW MGAIDPETGGTNYAQKFQGRVTMTADTSISTAYMELSRLRSDDTAVYY CSRSGYYVSNYESYAMDYWGQGTTVTVSSASTKGPSVFPLAPSSKSTSGG TAALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKT ISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 233 | Chimeric mouse antibody FS002-A226 light chain | DIKITQSPSSMYASLGERVTITCKASQDIKSYLSWYQQKPGSSPRLLIYDTS NLASGVPVRFSGRGSGTSYSLTISRMEAEDAATYYCQQWSSYPFTFGSGT KLEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGL SSPVTKSFNRGEC |
| 234 | Chimeric mouse antibody FS002-A226 heavy chain | QLQLQQSGAELVRPGASVTLSCKASGYTFTDYEIHWVKQTPVHGLEWIG AIDPETGGTNYNQKFNDKATLTADKSSSTAYMDLRSLTSEDSAVYYCSR SGYYVSNYESYAMDYWGQGTSVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYT QKSLSLSPGK |
| 235 | Humanized antibody FS002-A226-H3L 4 light chain | DIQLTQSPSSLSASVGDRVTITCRASQDIKSYLSWYQQKPGKAPKLLIYDT SNLASGVPSRFSGSGSGTDFTLTISSLQPEDFATYYCQQWSSYPFTFGGGT KLEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGL SSPVTKSFNRGEC |
| 236 | Humanized antibody FS002-A226-H3L 4 heavy chain | QLQLVQSGAEVKKPGASVKVSCKASGYTFTDYEIHWVRQAPGQGLEWI GAIDPETGGTNYNQKFNDRVTLTADTSISTAYMELSRLRSDDTAVYYCS RSGYYVSNYESYAMDYWGQGTTVTVSSASTKGPSVFPLAPSSKSTSGGTA ALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGK |
| 237 | Humanized antibody FS002-A226-H4L 3 light chain | DIQITQSPSSMSASVGDRVTITCKASQDIKSYLSWYQQKPGKAPKLLIYDT SNLASGVPSRFSGSGSGTDYTLTISSLQPEDFATYYCQQWSSYPFTFGGGT KLEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGL SSPVTKSFNRGEC |
| 238 | Humanized antibody FS002-A226-H4L 3 heavy chain | QVQLVQSGAEVKKPGASVKVSCKASGYTFTDYEIHWVRQAPGQGLEWI GAIDPETGGTNYNQKFQGRVTLTADTSISTAYMELSRLRSDDTAVYYCS RSGYYVSNYESYAMDYWGQGTTVTVSSASTKGPSVFPLAPSSKSTSGGTA ALGCLVKDYFPEPVTVSWNSGALTSGVHTFPAVLQSSGLYSLSSVVTVPS SSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHY TQKSLSLSPGK |
| 239 | Humanized antibody FS002-A226-H5L 3 light chain | DIQITQSPSSMSASVGDRVTITCKASQDIKSYLSWYQQKPGKAPKLLIYDT SNLASGVPSRFSGSGSGTDYTLTISSLQPEDFATYYCQQWSSYPFTFGGGT KLEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGL SSPVTKSFNRGEC |
| 240 | Humanized antibody FS002-A226-H5L 3 heavy chain | QVQLVQSGAEVKKPGASVKVSCKASGYTFTDYEIHWVRQAPGQGLEW MGAIDPETGGTNYAQKFQGRVTMTADTSISTAYMELSRLRSDDTAVYY CSRSGYYVSNYESYAMDYWGQGTTVTVSSASTKGPSVFPLAPSSKSTSGG TAALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKT ISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |
| 241 | Humanized antibody FS002-A226-H5L 4 light chain | DIQLTQSPSSLSASVGDRVTITCRASQDIKSYLSWYQQKPGKAPKLLIYDT SNLASGVPSRFSGSGSGTDFTLTISSLQPEDFATYYCQQWSSYPFTFGGGT KLEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGL SSPVTKSFNRGEC |
| 242 | Humanized antibody FS002-A226-H5L 4 heavy chain | QVQLVQSGAEVKKPGASVKVSCKASGYTFTDYEIHWVRQAPGQGLEW MGAIDPETGGTNYAQKFQGRVTMTADTSISTAYMELSRLRSDDTAVYY CSRSGYYVSNYESYAMDYWGQGTTVTVSSASTKGPSVFPLAPSSKSTSGG TAALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKT ISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK |

| | | |
|---|---|---|
| Note: The bold and underlined parts of amino acid sequences in the table are the corresponding CDR sequences. | | |

### Detailed Description of the Invention

### I. General Definitions

In the present invention, the scientific and technical terms used herein have the meanings commonly understood by those skilled in the art, unless otherwise stated. Also, terms, and laboratory procedures associated to the protein and nucleic acid chemistry, molecular biology, cell and tissue culture, microbiology, immunology used herein are terms and common procedures widely used in the corresponding fields. Also, the following definitions and explanations of related terms are provided for a better understanding of the present invention.

As used herein, "antibody" refers to immunoglobulin and immunoglobulin fragments, naturally or partially or fully synthetically produced (e.g., recombinantly produced), comprising any fragment comprising at least a portion of the variable region of an immunoglobulin molecule and retaining the binding specificity of the full length immunoglobulin. Thus, an antibody includes any protein having a binding structural domain homologous or substantially identical to an immunoglobulin antigen-binding structural domain (antibody-binding site). An antibody includes an antibody fragment, such as an antibody fragment against tumor stem cells. As used herein, the term "antibody" thus includes a synthetic antibody, a recombinantly produced antibody, a multispecific antibody (e.g., a bispecific antibody), a human antibody, a non-human antibody, a humanized antibody, a chimeric antibody, an intracellular antibody, and antibody fragments, for example, but not limited to, Fab fragment, Fab' fragment, F(ab')₂ fragment, Fv fragment, disulfide-linked Fv (dsFv), Fd fragment, Fd' fragment, single-chain Fv (scFv), single-chain Fab (scFab), a double antibody, an anti-idiotypic (anti-Id) antibody, or an antigen-binding fragment of any of the above antibodies. The antibody provided herein includes members of any immunoglobulin class (e.g., IgG, IgM, IgD, IgE, IgA, and IgY), any class (e.g., IgG1, IgG2, IgG3, IgG4, IgA1, and IgA2) or subclass (e.g., IgG2a and IgG2b).

As used herein, an "antibody fragment" or "antigen-binding fragment" of an antibody refers to any portion of a full length antibody that is less than the full length, but includes at least a portion of the variable region of the antibody that binds to the antigen (e.g., one or more CDRs and/or one or more antibody-binding sites), and thus retains the binding specificity as well as at least part of the specific binding capability of the full length antibody. Thus, an antigen-binding fragment refers to an antibody fragment comprising an antigen-binding portion that binds to the same antigen as the antibody deriving the antibody fragment. The antibody fragment includes an antibody derivative produced by enzymatic treatment of the full length antibody, as well as a synthetically produced derivative, such as a recombinantly produced derivative. The antibody includes an antibody fragment. Examples of the antibody fragment include, but are not limited to, Fab, Fab', F(ab')₂, single chain Fv (scFv), Fv, dsFv, double antibody, Fd and Fd' fragments, and other fragments, comprising modified fragments (see, for example, Methods in Molecular Biology, Vol 207: Recombinant Antibodies for Cancer Therapy Methods and Protocols (2003); Chapter 1; p 3-25, Kipriyanov). The fragment may include a plurality of chains joined together, for example by a disulfide bond and/or by a peptide linker. The antibody fragment typically includes at least or about 50 amino acids, and typically at least or about 200 amino acids. The antigen-binding fragment includes any antibody fragment that, when being inserted into an antibody framework (e.g., by substituting a corresponding region), obtains an antibody that immunospecifically binds to the antigen (i.e., exhibits at least or at least about 10⁷-10⁸ M⁻¹ Ka).

As used herein, a "monoclonal antibody" refers to a population of identical antibodies, where each antibody molecule is identical to other antibody molecules in the population of monoclonal antibodies. In contrast to the properties of a polyclonal population of antibodies, the polyclonal population of antibodies include antibodies having a plurality of different sequences. The monoclonal antibody may be prepared by a number of well-known methods (Smith et al. (2004) J. Clin. Pathol. 57, 912-917; and Nelson et al., J Clin Pathol (2000), 53, 165-175). For example, the monoclonal antibody may be prepared by immortalized B cells, for example by fusing with myeloma cells to produce a hybridoma cell line or by infecting B cells with a virus such as EBV. Recombinant techniques may also be used to prepare an antibody from a clonal population of host cells in vitro by transforming the host cells with a plasmid carrying an artificial sequence of a nucleotide encoding the antibody.

As used herein, the term "hybridoma" or "hybridoma cell" refers to a cell or cell line (typically a myeloma or lymphoma cell) produced by fusing antibody-producing lymphocytes and non-antibody-producing cancer cells. As known to those of ordinary skill in the art, hybridoma may proliferate and continue to be supplied to produce specific monoclonal antibodies. Methods for producing a hybridoma are known in the art (see, for example, Harlow & Lane, 1988). When referring to the term "hybridoma" or "hybridoma cell", it also includes subcloned and progeny cells of the hybridoma.

As used herein, a "conventional antibody" refers to an antibody comprising two heavy chains (which may be marked as H and H'), two light chains (which may be marked as L and L'), and two antigen-binding sites, where each heavy chain may be a full length immunoglobulin heavy chain or any of functional regions thereof that retains the antigen binding ability (e.g., heavy chains comprising, but are not limited to, V_{H} chains, V_{H}-C_{H}1 chains, and V_{H}-C_{H}1-C_{H}2-C_{H}3 chains), and each light chain may be a full length light chain or any functional region (e.g., light chains comprising, but not limited to, V_{L} chain and V_{L}-C_{L} chain). Each heavy chain (H and H') is paired with a light chain (L and L', respectively).

As used herein, a full length antibody is an antibody having two full length heavy chains (e.g., V_{H}-C_{H}1-C_{H}2-C_{H}3 or V_{H}-C_{H}1-C_{H}2-C_{H}3-C_{H}4), two full length light chains (V_{L}-C_{L}), and a hinge region, for example an antibody naturally produced by secretion of B cells by an antibody, and an synthetically produced antibody with the same structural domain.

As used herein, dsFv refers to an Fv having an engineered intermolecular disulfide bond that stabilizes a V_{H}-V_{L} pair.

As used herein, a Fab fragment is an antibody fragment obtained by papain digestion of a full length immunoglobulin, or a fragment having the same structure produced, for example, by recombinant methods. The Fab fragment includes a light chain (comprising V_{L} and C_{L}) and another chain comprising a variable structural domain (V_{H}) of the heavy chain and a constant region structural domain (C_{H}1) of the heavy chain.

As used herein, an F(ab')₂ fragment is an antibody fragment resulting from pepsin digestion of immunoglobulin at pH 4.0-4.5, or a fragment of the same structure produced, for example, by recombinant methods. The F(ab')₂ fragment substantially includes two Fab fragments, where each heavy chain moiety includes an additional several amino acids, comprising a cysteine that forms a disulfide bond linking the two fragments.

As used herein, a Fab' fragment is a fragment comprising a half of a F(ab')₂ fragment (one heavy chain and one light chain).

As used herein, a scFv fragment refers to an antibody fragment comprising a variable light chain (V_{L}) and a variable heavy chain (V_{H}) covalently linked in any sequence by a polypeptide linker. The length of the linker makes the two variable structural domains bridged substantially without interference. An exemplary linker is a (Gly-Ser)ₙ residue with some Glu or Lys residues dispersed to increase solubility.

The term "chimeric antibody" refers to such an antibody wherein the variable region sequence is derived from one species and the constant region sequence is derived from another species, such as an antibody wherein the variable region sequence is derived from a mouse antibody and the constant region sequence is derived from a human antibody.

A "humanized" antibody refers to a non-human (e.g., mouse) antibody form that is a chimeric immunoglobulin, immunoglobulin chain or fragment thereof (e.g., Fv, Fab, Fab', F(ab')₂ or other antigen-binding subsequences of the antibody), comprising minimal sequences derived from non-human immunoglobulins. Preferably, the humanized antibody is a human immunoglobulin (recipient antibody) where residues of complementarity determining regions (CDRs) of the recipient antibody are substituted by CDR residues of non-human species (donor antibody) having the expected specificity, affinity and ability, such as a mouse, rat or rabbit.

Furthermore, in humanization, it is also possible to mutate the amino acid residues within the CDR1, CDR2 and/or CDR3 regions of VH and/or VL, thereby improving one or more binding properties (e.g., affinity) of the antibody. A mutation, such as a PCR-mediated mutation, can be introduced, wherein the effect of the mutation on antibody binding or other functional properties can be assessed using in vitro or in vivo tests as described herein. Typically, a conservative mutation is introduced. Such mutation can be substitution, addition or deletion of an amino acid.

In addition, there are typically a maximum of one or two mutations within the CDRs. Thus, the humanized antibody according to the present invention also include the antibody comprising one or two amino acid mutations within the CDRs.

As used herein, a variable structural domain or variable region is a specific Ig structural domain of an antibody heavy or light chain that comprises an amino acid sequence that varies between different antibodies. Each light chain and each heavy chain has variable region structural domains V_{L} and V_{H}, respectively. The variable structural domain provides antigen specificity and is therefore responsible for antigen recognition. Each variable region comprises a CDR and a framework region (FR), wherein the CDR is the moiety of an antigen-binding site structural domain.

As used herein, an "antigen binding structural domain" and "antigen-binding site" are used synonymously to refer to a structural domain within an antibody that recognizes and physically interacts with a cognate antigen. Native common full length antibody molecules have two common antigen-binding sites, each of which includes a heavy chain variable region moiety and a light chain variable region moiety. Common antigen binding sites include a loop joining the antiparallel beta (β) chains within the variable region structural domain. The antigen-binding site may include other moieties of the variable region structural domain. Each common antigen-binding site includes 3 hypervariable regions from the heavy chain and 3 hypervariable regions from the light chain. The hypervariable region is also referred to as a complementarity determining region (CDR).

As used herein, the "hypervariable region", "HV", "complementarity determining region", "CDR", and "antibody CDR" may be used interchangeably to refer to one of the multiple moieties of each variable region of an antigen-binding site that together form an antibody. Each variable region structural domain includes three CDRs, named as CDR1, CDR2, and CDR3. For example, the light chain variable region structural domain includes three CDRs named as VL CDR1, VL CDR2, and VL CDR3; the heavy chain variable region structural domain includes three CDRs named as VH CDR1, VH CDR2, and VH CDR3. The three CDRs in the variable region are discontinuous along the linear amino acid sequence, but are close in the folded polypeptide. The CDRs are located within the loop joining the parallel chains within a β-sheet of the variable structural domain. As described herein, those skilled in the art are aware of and can identify CDRs based on Kabat or Chothia numbers (see, e.g., Kabat, E.A. et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). In the present invention, the abbreviations "VH CDR", "HCDR", "VL CDR", and "LCDR" have the same meaning.

As used herein, a framework region (FR) is a structural domain within an antibody variable region structural domain within a β-sheet. In terms of amino acid sequence, the FR region is relatively more conservative than the hypervariable region.

As used herein, a "constant region" structural domain is a structural domain in an antibody heavy or light chain that comprises an amino acid sequence that is relatively more conservative than the amino acid sequence of the variable region structural domain. In a common full length antibody molecule, each light chain has a single light chain constant region (C_{L}) structural domain, and each heavy chain includes one or more heavy chain constant region (C_{H}) structural domains, comprising C_{H}1, C_{H}2, C_{H}3, and C_{H}4. The full length IgA, IgD, and IgG isotypes include C_{H}1, C_{H}2, C_{H}3, and a hinge region, while IgE and IgM include C_{H}1, C_{H}2, C_{H}3, and C_{H}4. The C_{H}1 and C_{L} structural domains extend the Fab arm of the antibody molecule, thus facilitating interaction with the antigen and rotation of the antibody arm. The antibody constant region may serve to an effector function such as, but not limited to, clearing the antigens, pathogens, and toxins to which the antibody specifically binds, such as by interaction with various cells, biomolecules, and tissues.

As used herein, a functional region of an antibody is an antibody moiety comprising at least V_{H}, V_{L}, C_{H} (e.g., C_{H}1, C_{H}2 or C_{H}3), C_{L} or a hinge region structural domain or at least a functional region thereof of the antibody.

As used herein, a functional region of a V_{H} structural domain is at least a portion of a complete V_{H} structural domain that retains at least a part of the binding specificity of the complete V_{H} structural domain (e.g., by retaining one or more CDRs of the complete V_{H} structural domain) such that the functional region of the V_{H} structural domain binds to the antigen either alone or in combination with another antibody structural domain (e.g., a V_{L} structural domain) or a region thereof. A functional region of an exemplary V_{H} structural domain is a region that contains CDR1, CDR2, and/or CDR3 of the V_{H} structural domain.

As used herein, a functional region of a V_{L} structural domain is at least a portion of a complete V_{L} structural domain that retains at least a part of the binding specificity of the complete V_{L} structural domain (e.g., by retaining one or more CDRs of the complete V_{L} structural domain) such that the functional region of the V_{L} structural domain binds to the antigen either alone or in combination with another antibody structural domain (e.g., a V_{H} structural domain) or a region thereof. A functional region of an exemplary V_{L} structural domain is a region that contains CDR1, CDR2 and/or CDR3 of the V_{L} structural domain.

As used herein, the "specific binding" or "immunospecific binding" with respect to an antibody or an antigen-binding fragment thereof is used interchangeably herein and refers to the capability of an antibody or an antigen-binding fragment thereof to form one or more non-covalent bonds with an alloantigen through the non-covalent interaction between antibody binding sites of the antibody and the antigen. The antigen may be an isolated antigen or present in a tumor cell.

The term "Kassoc" or "Ka" used herein is intended to indicate the binding rate of a specific antibody-antigen interaction, while the term "Kdis" or "Kd" is intended to indicate the dissociation rate of a specific antibody-antigen interaction. The term "KD" used herein is intended to represent a dissociation constant, which is derived from the ratio of Kd to Ka (i.e., Kd/Ka) and is expressed as a molar concentration (M). The KD value of an antibody may be determined using methods known in the art. The KD of an antibody may be determined by for example surface plasmon resonance (SPR, Rich and Myszka (2000) Curr. Opin. Biotechnol 11:54; Englebienne (1998) Analyst. 189:1599), isothermal titration calorimetry (ITC) or other kinetic interaction determination methods known in the art (see, for example, Paul, ed., Fundamental Immunology, 2nd ed., Raven Press, New York, pages 332-336 (1989); see also US Patent No. 7,229,619 that describes exemplary SPR and ITC methods for calculating the binding affinity of antibodies). Instruments and methods for detecting and monitoring the binding rate in real time are known and commercially available (see BiaCore 2000, Biacore AB, Upsala, Sweden and GE Healthcare Life Sciences; Malmqvist (2000) Biochem. Soc. Trans. 27:335).

The terms "isolated protein", "isolated polypeptide" or "isolated antibody" means that the protein, polypeptide or antibody (1) is not associated with its naturally associated components in its natural state, (2) is free of other proteins from the same species, (3) is expressed by cells from different species, or (4) is not occurring in nature. Thus, a chemically synthesized polypeptide or a polypeptide synthesized in a cellular system other than a naturally derived cell of the polypeptide will be "isolated" from its naturally associated components. The isolation may also be employed to render the protein substantially free of naturally associated components, i.e., using protein purification techniques well known in the art.

The "substitution", "deletion", or "addition" of amino acid residues refers to the conserved amino acid modification in a peptide or protein that does not alter the function and/or biological activity of the peptide or protein. For example, the substitution of amino acid residues may be a conserved amino acid substitution. Suitable conservative amino acid substitutions are known to those skilled in the art and may generally be carried out without altering the biological activity of the resulting molecule. In general, one skilled in the art knows that a single amino acid substitution in a non-essential region of a polypeptide does not substantially alter the biological activity (see, for example, Watson et al., Molecular Biology of the Gene, 4th Edition, 1987, The Benjamin/Cummings Pub. co., p.224).

As used herein, the terms "polynucleotide" and "nucleic acid molecule" refer to an oligomer or polymer comprising at least two linked nucleotides or nucleotide derivatives, comprising deoxyribonucleic acid (DNA) and ribonucleic acid (RNA) typically linked together by phosphodiester bonds.

As used herein, an isolated nucleic acid molecule is a nucleic acid molecule isolated from other nucleic acid molecules present in a natural nucleic acid molecule source. An "isolated" nucleic acid molecule, such as a cDNA molecule, may be substantially free of other cellular material or culture medium when prepared by recombinant techniques, or substantially free of chemical precursor or other chemical components in chemical synthesis. An exemplary isolated nucleic acid molecule provided herein includes an isolated nucleic acid molecule encoding the provided antibody or antigen-binding fragment.

The "sequence identity" has the meaning well known in the art, and the percentage of sequence identity between two nucleic acid or polypeptide molecules or regions may be calculated using the disclosed technique. The sequence identity may be measured along the full length of a polynucleotide or polypeptide or along a region of the molecule. (See, for example: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods for measuring the identity between two polynucleotides or polypeptides, the term "sequence identity" is well known to those skilled in the art (Carrillo, H. & Lipman, D., SIAM J Applied Math 48:1073 (1988)).

As used herein, "operably linked" with respect to a nucleic acid sequence, region, element or structural domain means that the nucleic acid regions are functionally related to each other. For example, a promoter may be operably linked to a nucleic acid encoding a polypeptide such that the promoter regulates or mediates transcription of the nucleic acid.

As used herein, "expression" refers to the process by which a polypeptide is produced by transcription and translation of a polynucleotide. The level of expression of the polypeptide may be assessed using any method known in the art, comprising, for example, methods for determining the amount of polypeptide produced from a host cell. Such methods may include, but are not limited to, quantification of polypeptides in cell lysates by ELISA, Coomassie blue staining after gel electrophoresis, Lowry protein determination, and Bradford protein determination.

As used herein, a "host cell" is a cell used to receive, maintain, replicate, and amplify a vector. A host cell may also be used to express the polypeptide encoded by a vector. When the host cell divides, the nucleic acid contained in the vector replicates, thereby amplifying the nucleic acid. The host cell may be an eukaryotic cell or a prokaryotic cell. Suitable host cells include, but are not limited to, CHO cells, COS cells, HeLa cells, HEK cells such as HEK 293 cells.

As used herein, a "vector" is a replicable nucleic acid from which one or more heterologous proteins may be expressed when the vector is transformed into a suitable host cell. The vectors include those into which a nucleic acid encoding a polypeptide or a fragment thereof may be introduced, typically by restricting enzyme digestion and ligation. Also included in the vectors are those vectors comprising a nucleic acid encoding a polypeptide. A vector is used to introduce a nucleic acid encoding a polypeptide into a host cell, to amplify the nucleic acid, or to express/display the polypeptide encoded by the nucleic acid. The vector typically remains free, but can be designed to integrate a gene or a part thereof into the chromosome of a genome. Vectors of artificial chromosomes, such as yeast artificial vectors and mammalian artificial chromosomes, are also contemplated. The selection and use of such vectors are well known to those skilled in the art. As used herein, an "expression vector" includes a vector capable of expressing DNA operably linked to a regulatory sequence, such as a regulatory sequence in a promoter region that is capable of affecting the expression of such a DNA fragment. Such additional fragments may include promoter and terminator sequences, and may optionally include one or more replication origins, one or more selective markers, enhancers, polyadenylation signals, and the like. An expression vector is typically derived from plasmid or viral DNA, or may include elements of both. Thus, an expression vector refers to a recombinant DNA or RNA construct, such as a plasmid, phage, recombinant virus, or other vector that, when introduced into a suitable host cell, results in expression of the cloned DNA. Suitable expression vectors are well known to those skilled in the art and include expression vectors that are replicable in eukaryotic cells and/or prokaryotic cells, as well as expression vectors that retain the free expression vector or integrate into the genome of the host cell.

As used herein, "treating" an individual having a disease or condition indicates partial or total relief of the symptoms of the individual, or no change after treatment. Thus, treatment includes prevention, therapy, and/or cure. Prevention refers to the prevention of underlying disease and/or prevention of worsening symptoms or disease progression. Treatment also includes any of the antibody or antigen-binding fragment thereof provided, as well as any pharmaceutical use of the composition provided herein.

As used herein, "therapeutic effect" refers to an effect caused by the treatment of an individual that alters, generally ameliorates or relieves the symptoms of a disease or condition, or cures the disease or condition.

As used herein, "therapeutically effective amount" or "therapeutically effective dosage" refers to an amount of a substance, compound, material, or composition comprising a compound that is at least sufficient to produce a therapeutic effect upon administration to a subject. Thus, it is an amount necessary to prevent, cure, ameliorate, block or partially block the symptoms of a disease or condition.

As used herein, "prophylactically effective amount" or "prophylactically effective dosage" refers to an amount of a substance, compound, material or a composition comprising a compound that has the desired prophylactic effect upon administration to a subject, for example, preventing or delaying the occurrence or relapse of a disease or condition, and reducing the likelihood of the occurrence or relapse of a disease or condition. The complete prophylactically effective dosage does not have to occur by administrating one dose and can occur only after administrating a series of doses. Thus, a prophylactically effective amount can be administered in one or more administrations.

As used herein, the term "patient" refers to a mammal, such as a human being.

### II. Antibody targeting the ITGA2 protein

The full name of ITGA2 is Integrin alpha-2, and the human ITGA2 protein is numbered as P17301 in the Uniprot database. See https://www.uniprot.org/uniprot/P17301.

The inventor found that ITGA2 is a tumor-associated antigen that is specifically overexpressed by tumor cells, especially in patients with cancers such as colorectal cancer, bile duct cancer, pancreatic cancer, and skin cancer. Based on this, the present inventor has designed a variety of different humanized antibodies targeting the ITGA2 protein. The experiments in examples prove that the antibody according to the present invention has excellent binding ability for the ITGA2 protein (EC50<1 nM), and also has excellent binding ability to HuCCT1 cells that highly express ITGA2 (EC50<5 nM). With the excellent binding ability of the monoclonal antibody according to the present invention, partner molecules such as cytotoxins can be effectively delivered to tumor cells.

Therefore, in the first aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 42, (b) LCDR2 as set forth in SEQ ID NO: 43, and (c) LCDR3 as set forth in SEQ ID NO: 44, and a heavy chain variable region that comprises (d) HCDR1 as set forth in SEQ ID NO: 46, (e) HCDR2 as set forth in SEQ ID NO: 47, and (f) HCDR3 as set forth in SEQ ID NO: 48;
2) a light chain variable region that comprises (a) LCDR1 as set forth in SEQ ID NO: 50, (b) LCDR2 as set forth in SEQ ID NO: 51, and (c) LCDR3 as set forth in SEQ ID NO: 52, and a heavy chain variable region that comprises (d) HCDR1 as set forth in SEQ ID NO: 54, (e) HCDR2 as set forth in SEQ ID NO: 55, and (f) HCDR3 as set forth in SEQ ID NO:56;
3) a light chain variable region that comprises (a) LCDR1 as set forth in SEQ ID NO: 58, (b) LCDR2 as set forth in SEQ ID NO: 59, and (c) LCDR3 as set forth in SEQ ID NO: 60, and a heavy chain variable region that comprises (d) HCDR1 as set forth in SEQ ID NO: 62, (e) HCDR2 as set forth in SEQ ID NO: 63, and (f) HCDR3 as set forth in SEQ ID NO: 54; and
4) a light chain variable region that comprises (a) LCDR1 as set forth in SEQ ID NO: 66, (b) LCDR2 as set forth in SEQ ID NO: 67, and (c) LCDR3 as set forth in SEQ ID NO: 68, and a heavy chain variable region that comprises (d) HCDR1 as set forth in SEQ ID NO: 70, (e) HCDR2 as set forth in SEQ ID NO: 71, and (f) HCDR3 as set forth in SEQ ID NO: 72.

In some embodiments, the isolated antibody or the antigen binding fragment thereof comprises a light chain variable region and a heavy chain variable region selected from at least one of the following groups:
1) a light chain variable region that comprises an amino acid sequence as set forth in SEQ ID NO:41, or an amino acid sequence that is at least 85%, at least 90%, at least 95%, at least 96%, at least 97%, at least 98%, or at least 99% identical to the amino acid sequence as set forth in SEQ ID NO:41, and a heavy chain variable region that comprises an amino acid sequence as set forth in SEQ ID NO:45, or an amino acid sequence that is at least 85%, at least 90%, at least 95%, at least 96%, at least 97%, at least 98%, or at least 99% identical to the amino acid sequence as set forth in SEQ ID NO:45;
2) a light chain variable region that comprises an amino acid sequence as set forth in SEQ ID NO:49, or an amino acid sequence that is at least 85%, at least 90%, at least 95%, at least 96%, at least 97%, at least 98%, or at least 99% identical to the amino acid sequence as set forth in SEQ ID NO:49, and a heavy chain variable region that comprises an amino acid sequence as set forth in SEQ ID NO:53, or an amino acid sequence that is at least 85%, at least 90%, at least 95%, at least 96%, at least 97%, at least 98%, or at least 99% identical to the amino acid sequence as set forth in SEQ ID NO:53;
3) a light chain variable region that comprises an amino acid sequence as set forth in SEQ ID NO:57, or an amino acid sequence that is at least 85%, at least 90%, at least 95%, at least 96%, at least 97%, at least 98%, or at least 99% identical to the amino acid sequence as set forth in SEQ ID NO:57, and a heavy chain variable region that comprises an amino acid sequence as set forth in SEQ ID NO:61, or an amino acid sequence that is at least 85%, at least 90%, at least 95%, at least 96%, at least 97%, at least 98%, or at least 99% identical to the amino acid sequence as set forth in SEQ ID NO:61; and
4) a light chain variable region that comprises an amino acid sequence as set forth in SEQ ID NO:65, or an amino acid sequence that is at least 85%, at least 90%, at least 95%, at least 96%, at least 97%, at least 98%, or at least 99% identical to the amino acid sequence as set forth in SEQ ID NO:65, and a heavy chain variable region that comprises an amino acid sequence as set forth in SEQ ID NO:69, or an amino acid sequence that is at least 85%, at least 90%, at least 95%, at least 96%, at least 97%, at least 98%, or at least 99% identical to the amino acid sequence as set forth in SEQ ID NO:69.

In some embodiments, an antibody that is at least 85%, at least 90%, at least 95%, at least 96%, at least 97%, at least 98%, or at least 99% identical to the amino acid sequences of the light chain variable region (SEQ ID NOs: 41, 49, 57 and 65) and amino acid sequences of the heavy chain variable region (SEQ ID NOs: 45, 53, 61 and 69) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the light chain variable region (SEQ ID NOs: 41, 49, 57 and 65) and amino acid sequences of the heavy chain variable region (SEQ ID NOs: 45, 53, 61 and 69) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain variable region (SEQ ID NOs: 41, 49, 57 and 65) and amino acid sequences of the heavy chain variable region (SEQ ID NOs: 45, 53, 61 and 69) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof further comprises a light chain constant region and a heavy chain constant region,
the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201, and
the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.

In some embodiments, the isolated antibody or the antigen binding fragment thereof comprises a light chain and a heavy chain selected from at least one of the following groups:
1) a light chain that comprises an amino acid sequence as set forth in SEQ ID NO:203, or an amino acid sequence that is at least 85%, at least 90%, at least 95%, at least 96%, at least 97%, at least 98%, or at least 99% identical to the amino acid sequence as set forth in SEQ ID NO:203, and a heavy chain that comprises an amino acid sequence as set forth in SEQ ID NO:204, or an amino acid sequence that is at least 85%, at least 90%, at least 95%, at least 96%, at least 97%, at least 98%, or at least 99% identical to the amino acid sequence as set forth in SEQ ID NO:204;
2) a light chain that comprises an amino acid sequence as set forth in SEQ ID NO:205, or an amino acid sequence that is at least 85%, at least 90%, at least 95%, at least 96%, at least 97%, at least 98%, or at least 99% identical to the amino acid sequence as set forth in SEQ ID NO:205, and a heavy chain that comprises an amino acid sequence as set forth in SEQ ID NO:206, or an amino acid sequence that is at least 85%, at least 90%, at least 95%, at least 96%, at least 97%, at least 98%, or at least 99% identical to the amino acid sequence as set forth in SEQ ID NO:206;
3) a light chain that comprises an amino acid sequence as set forth in SEQ ID NO:207, or an amino acid sequence that is at least 85%, at least 90%, at least 95%, at least 96%, at least 97%, at least 98%, or at least 99% identical to the amino acid sequence as set forth in SEQ ID NO:207, and a heavy chain that comprises an amino acid sequence as set forth in SEQ ID NO:208, or an amino acid sequence that is at least 85%, at least 90%, at least 95%, at least 96%, at least 97%, at least 98%, or at least 99% identical to the amino acid sequence as set forth in SEQ ID NO:208;
4) a light chain that comprises an amino acid sequence as set forth in SEQ ID NO:209, or an amino acid sequence that is at least 85%, at least 90%, at least 95%, at least 96%, at least 97%, at least 98%, or at least 99% identical to the amino acid sequence as set forth in SEQ ID NO:209, and a heavy chain that comprises an amino acid sequence as set forth in SEQ ID NO:210, or an amino acid sequence that is at least 85%, at least 90%, at least 95%, at least 96%, at least 97%, at least 98%, or at least 99% identical to the amino acid sequence as set forth in SEQ ID NO:210.

In some embodiments, an antibody that is at least 85%, at least 90%, at least 95%, at least 96%, at least 97%, at least 98%, or at least 99% identical to the amino acid sequences of the light chain (SEQ ID NOs: 203, 205, 207 and 209) and amino acid sequences of the heavy chain (SEQ ID NOs: 204, 206, 208 and 210) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the light chain (SEQ ID NOs: 203, 205, 207 and 209) and amino acid sequences of the heavy chain (SEQ ID NOs: 204, 206, 208 and 210) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain (SEQ ID NOs: 203, 205, 207 and 209) and amino acid sequences of the heavy chain (SEQ ID NOs: 204, 206, 208 and 210) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof consists of a light chain and a heavy chain selected from at least one of the following groups:
1) a light chain that consists of an amino acid sequence as set forth in SEQ ID NO:203, and a heavy chain that consists of an amino acid sequence as set forth in SEQ ID NO:204;
2) a light chain that consists of an amino acid sequence as set forth in SEQ ID NO:205, and a heavy chain that consists of an amino acid sequence as set forth in SEQ ID NO:206;
3) a light chain that consists of an amino acid sequence as set forth in SEQ ID NO:207, and a heavy chain that consists of an amino acid sequence as set forth in SEQ ID NO:208;
4) a light chain that consists of an amino acid sequence as set forth in SEQ ID NO:209, and a heavy chain that consists of an amino acid sequence as set forth in SEQ ID NO:210.

In the second aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 74, (b) LCDR2 as set forth in SEQ ID NO: 75, and (c) LCDR3 as set forth in SEQ ID NO: 76, and a heavy chain variable region that comprises (d) HCDR1 as set forth in SEQ ID NO: 78, (e) HCDR2 as set forth in SEQ ID NO: 79, and (f) HCDR3 as set forth in SEQ ID NO: 80;
2) a light chain variable region that comprises (a) LCDR1 as set forth in SEQ ID NO: 82, (b) LCDR2 as set forth in SEQ ID NO: 83, and (c) LCDR3 as set forth in SEQ ID NO: 84, and a heavy chain variable region that comprises (d) HCDR1 as set forth in SEQ ID NO: 86, (e) HCDR2 as set forth in SEQ ID NO: 87, and (f) HCDR3 as set forth in SEQ ID NO:88;
3) a light chain variable region that comprises (a) LCDR1 as set forth in SEQ ID NO: 90, (b) LCDR2 as set forth in SEQ ID NO: 91, and (c) LCDR3 as set forth in SEQ ID NO: 92, and a heavy chain variable region that comprises (d) HCDR1 as set forth in SEQ ID NO: 94, (e) HCDR2 as set forth in SEQ ID NO: 95, and (f) HCDR3 as set forth in SEQ ID NO: 96;
4) a light chain variable region that comprises (a) LCDR1 as set forth in SEQ ID NO: 98, (b) LCDR2 as set forth in SEQ ID NO: 99, and (c) LCDR3 as set forth in SEQ ID NO: 100, and a heavy chain variable region that comprises (d) HCDR1 as set forth in SEQ ID NO: 102, (e) HCDR2 as set forth in SEQ ID NO: 103, and (f) HCDR3 as set forth in SEQ ID NO: 104;
5) a light chain variable region that comprises (a) LCDR1 as set forth in SEQ ID NO: 106, (b) LCDR2 as set forth in SEQ ID NO: 107, and (c) LCDR3 as set forth in SEQ ID NO: 108, and a heavy chain variable region that comprises (d) HCDR1 as set forth in SEQ ID NO: 110, (e) HCDR2 as set forth in SEQ ID NO: 111, and (f) HCDR3 as set forth in SEQ ID NO: 112;
6) a light chain variable region that comprises (a) LCDR1 as set forth in SEQ ID NO: 114, (b) LCDR2 as set forth in SEQ ID NO: 115, and (c) LCDR3 as set forth in SEQ ID NO: 116, and a heavy chain variable region that comprises (d) HCDR1 as set forth in SEQ ID NO: 118, (e) HCDR2 as set forth in SEQ ID NO: 119, and (f) HCDR3 as set forth in SEQ ID NO: 120; and
7) a light chain variable region that comprises (a) LCDR1 as set forth in SEQ ID NO: 122, (b) LCDR2 as set forth in SEQ ID NO: 123, and (c) LCDR3 as set forth in SEQ ID NO: 124, and a heavy chain variable region that comprises (d) HCDR1 as set forth in SEQ ID NO: 126, (e) HCDR2 as set forth in SEQ ID NO: 127, and (f) HCDR3 as set forth in SEQ ID NO: 128.

In some embodiments, the isolated antibody or the antigen binding fragment thereof comprises a light chain variable region and a heavy chain variable region selected from at least one of the following groups:
1) a light chain variable region that comprises an amino acid sequence as set forth in SEQ ID NO:73, or an amino acid sequence that is at least 85%, at least 90%, at least 95%, at least 96%, at least 97%, at least 98%, or at least 99% identical to the amino acid sequence as set forth in SEQ ID NO:73, and a heavy chain variable region that comprises an amino acid sequence as set forth in SEQ ID NO:77, or an amino acid sequence that is at least 85%, at least 90%, at least 95%, at least 96%, at least 97%, at least 98%, or at least 99% identical to the amino acid sequence as set forth in SEQ ID NO:77;
2) a light chain variable region that comprises an amino acid sequence as set forth in SEQ ID NO:81, or an amino acid sequence that is at least 85%, at least 90%, at least 95%, at least 96%, at least 97%, at least 98%, or at least 99% identical to the amino acid sequence as set forth in SEQ ID NO:81, and a heavy chain variable region that comprises an amino acid sequence as set forth in SEQ ID NO:85, or an amino acid sequence that is at least 85%, at least 90%, at least 95%, at least 96%, at least 97%, at least 98%, or at least 99% identical to the amino acid sequence as set forth in SEQ ID NO:85;
3) a light chain variable region that comprises an amino acid sequence as set forth in SEQ ID NO:89, or an amino acid sequence that is at least 85%, at least 90%, at least 95%, at least 96%, at least 97%, at least 98%, or at least 99% identical to the amino acid sequence as set forth in SEQ ID NO:89, and a heavy chain variable region that comprises an amino acid sequence as set forth in SEQ ID NO:93, or an amino acid sequence that is at least 85%, at least 90%, at least 95%, at least 96%, at least 97%, at least 98%, or at least 99% identical to the amino acid sequence as set forth in SEQ ID NO:93;
4) a light chain variable region that comprises an amino acid sequence as set forth in SEQ ID NO:97, or an amino acid sequence that is at least 85%, at least 90%, at least 95%, at least 96%, at least 97%, at least 98%, or at least 99% identical to the amino acid sequence as set forth in SEQ ID NO:97, and a heavy chain variable region that comprises an amino acid sequence as set forth in SEQ ID NO:101, or an amino acid sequence that is at least 85%, at least 90%, at least 95%, at least 96%, at least 97%, at least 98%, or at least 99% identical to the amino acid sequence as set forth in SEQ ID NO:101;
5) a light chain variable region that comprises an amino acid sequence as set forth in SEQ ID NO:105, or an amino acid sequence that is at least 85%, at least 90%, at least 95%, at least 96%, at least 97%, at least 98%, or at least 99% identical to the amino acid sequence as set forth in SEQ ID NO:105, and a heavy chain variable region that comprises an amino acid sequence as set forth in SEQ ID NO:109, or an amino acid sequence that is at least 85%, at least 90%, at least 95%, at least 96%, at least 97%, at least 98%, or at least 99% identical to the amino acid sequence as set forth in SEQ ID NO:109;
6) a light chain variable region that comprises an amino acid sequence as set forth in SEQ ID NO:113, or an amino acid sequence that is at least 85%, at least 90%, at least 95%, at least 96%, at least 97%, at least 98%, or at least 99% identical to the amino acid sequence as set forth in SEQ ID NO:113, and a heavy chain variable region that comprises an amino acid sequence as set forth in SEQ ID NO:117, or an amino acid sequence that is at least 85%, at least 90%, at least 95%, at least 96%, at least 97%, at least 98%, or at least 99% identical to the amino acid sequence as set forth in SEQ ID NO:117; and
7) a light chain variable region that comprises an amino acid sequence as set forth in SEQ ID NO:121, or an amino acid sequence that is at least 85%, at least 90%, at least 95%, at least 96%, at least 97%, at least 98%, or at least 99% identical to the amino acid sequence as set forth in SEQ ID NO:121, and a heavy chain variable region that comprises an amino acid sequence as set forth in SEQ ID NO:125, or an amino acid sequence that is at least 85%, at least 90%, at least 95%, at least 96%, at least 97%, at least 98%, or at least 99% identical to the amino acid sequence as set forth in SEQ ID NO:125.

In some embodiments, an antibody that is at least 85%, at least 90%, at least 95%, at least 96%, at least 97%, at least 98%, or at least 99% identical to the amino acid sequences of the light chain variable region (SEQ ID NOs: 73, 81, 89, 97, 105, 113 and 121) and amino acid sequences of the heavy chain variable region (SEQ ID NOs: 77, 85, 93, 101, 109, 117 and 125) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the light chain variable region (SEQ ID NOs: 73, 81, 89, 97, 105, 113 and 121) and amino acid sequences of the heavy chain variable region (SEQ ID NOs: 77, 85, 93, 101, 109, 117 and 125) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain variable region (SEQ ID NOs: 73, 81, 89, 97, 105, 113 and 121) and amino acid sequences of the heavy chain variable region (SEQ ID NOs: 77, 85, 93, 101, 109, 117 and 125) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof further comprises a light chain constant region and a heavy chain constant region,
the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201, and
the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.

In some embodiments, the isolated antibody or the antigen binding fragment thereof comprises a light chain and a heavy chain selected from at least one of the following groups:
1) a light chain that comprises an amino acid sequence as set forth in SEQ ID NO:211, or an amino acid sequence that is at least 85%, at least 90%, at least 95%, at least 96%, at least 97%, at least 98%, or at least 99% identical to the amino acid sequence as set forth in SEQ ID NO:211, and a heavy chain that comprises an amino acid sequence as set forth in SEQ ID NO:212, or an amino acid sequence that is at least 85%, at least 90%, at least 95%, at least 96%, at least 97%, at least 98%, or at least 99% identical to the amino acid sequence as set forth in SEQ ID NO:212;
2) a light chain that comprises an amino acid sequence as set forth in SEQ ID NO:213, or an amino acid sequence that is at least 85%, at least 90%, at least 95%, at least 96%, at least 97%, at least 98%, or at least 99% identical to the amino acid sequence as set forth in SEQ ID NO:213, and a heavy chain that comprises an amino acid sequence as set forth in SEQ ID NO:214, or an amino acid sequence that is at least 85%, at least 90%, at least 95%, at least 96%, at least 97%, at least 98%, or at least 99% identical to the amino acid sequence as set forth in SEQ ID NO:214;
3) a light chain that comprises an amino acid sequence as set forth in SEQ ID NO:215, or an amino acid sequence that is at least 85%, at least 90%, at least 95%, at least 96%, at least 97%, at least 98%, or at least 99% identical to the amino acid sequence as set forth in SEQ ID NO:215, and a heavy chain that comprises an amino acid sequence as set forth in SEQ ID NO:216, or an amino acid sequence that is at least 85%, at least 90%, at least 95%, at least 96%, at least 97%, at least 98%, or at least 99% identical to the amino acid sequence as set forth in SEQ ID NO:216;
4) a light chain that comprises an amino acid sequence as set forth in SEQ ID NO:217, or an amino acid sequence that is at least 85%, at least 90%, at least 95%, at least 96%, at least 97%, at least 98%, or at least 99% identical to the amino acid sequence as set forth in SEQ ID NO:217, and a heavy chain that comprises an amino acid sequence as set forth in SEQ ID NO:218, or an amino acid sequence that is at least 85%, at least 90%, at least 95%, at least 96%, at least 97%, at least 98%, or at least 99% identical to the amino acid sequence as set forth in SEQ ID NO:218;
5) a light chain that comprises an amino acid sequence as set forth in SEQ ID NO:219, or an amino acid sequence that is at least 85%, at least 90%, at least 95%, at least 96%, at least 97%, at least 98%, or at least 99% identical to the amino acid sequence as set forth in SEQ ID NO:219, and a heavy chain that comprises an amino acid sequence as set forth in SEQ ID NO:220, or an amino acid sequence that is at least 85%, at least 90%, at least 95%, at least 96%, at least 97%, at least 98%, or at least 99% identical to the amino acid sequence as set forth in SEQ ID NO:220;
6) a light chain that comprises an amino acid sequence as set forth in SEQ ID NO:221, or an amino acid sequence that is at least 85%, at least 90%, at least 95%, at least 96%, at least 97%, at least 98%, or at least 99% identical to the amino acid sequence as set forth in SEQ ID NO:221, and a heavy chain that comprises an amino acid sequence as set forth in SEQ ID NO:222, or an amino acid sequence that is at least 85%, at least 90%, at least 95%, at least 96%, at least 97%, at least 98%, or at least 99% identical to the amino acid sequence as set forth in SEQ ID NO:222; and
7) a light chain that comprises an amino acid sequence as set forth in SEQ ID NO:223, or an amino acid sequence that is at least 85%, at least 90%, at least 95%, at least 96%, at least 97%, at least 98%, or at least 99% identical to the amino acid sequence as set forth in SEQ ID NO:223, and a heavy chain that comprises an amino acid sequence as set forth in SEQ ID NO:224, or an amino acid sequence that is at least 85%, at least 90%, at least 95%, at least 96%, at least 97%, at least 98%, or at least 99% identical to the amino acid sequence as set forth in SEQ ID NO:224.

In some embodiments, an antibody that is at least 85%, at least 90%, at least 95%, at least 96%, at least 97%, at least 98%, or at least 99% identical to the amino acid sequences of the light chain (SEQ ID NOs: 211, 213, 215, 217, 219, 221 and 223) and amino acid sequences of the heavy chain (SEQ ID NOs: 212, 214, 216, 218, 220, 222 and 224) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the light chain (SEQ ID NOs: 211, 213, 215, 217, 219, 221 and 223) and amino acid sequences of the heavy chain (SEQ ID NOs: 212, 214, 216, 218, 220, 222 and 224) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain (SEQ ID NOs: 211, 213, 215, 217, 219, 221 and 223) and amino acid sequences of the heavy chain (SEQ ID NOs: 212, 214, 216, 218, 220, 222 and 224) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof consists of a light chain and a heavy chain selected from at least one of the following groups:
1) a light chain that consists of an amino acid sequence as set forth in SEQ ID NO:211, and a heavy chain that consists of an amino acid sequence as set forth in SEQ ID NO:212;
2) a light chain that consists of an amino acid sequence as set forth in SEQ ID NO:213, and a heavy chain that consists of an amino acid sequence as set forth in SEQ ID NO:214;
3) a light chain that consists of an amino acid sequence as set forth in SEQ ID NO:215, and a heavy chain that consists of an amino acid sequence as set forth in SEQ ID NO:216;
4) a light chain that consists of an amino acid sequence as set forth in SEQ ID NO:217, and a heavy chain that consists of an amino acid sequence as set forth in SEQ ID NO:218;
5) a light chain that consists of an amino acid sequence as set forth in SEQ ID NO:219, and a heavy chain that consists of an amino acid sequence as set forth in SEQ ID NO:220;
6) a light chain that consists of an amino acid sequence as set forth in SEQ ID NO:221, and a heavy chain that consists of an amino acid sequence as set forth in SEQ ID NO:222; and
7) a light chain that consists of an amino acid sequence as set forth in SEQ ID NO:223, and a heavy chain that consists of an amino acid sequence as set forth in SEQ ID NO:224.

In the third aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 130, (b) LCDR2 as set forth in SEQ ID NO: 131, and (c) LCDR3 as set forth in SEQ ID NO: 132, and a heavy chain variable region that comprises (d) HCDR1 as set forth in SEQ ID NO: 134, (e) HCDR2 as set forth in SEQ ID NO: 135, and (f) HCDR3 as set forth in SEQ ID NO: 136;
2) a light chain variable region that comprises (a) LCDR1 as set forth in SEQ ID NO: 138, (b) LCDR2 as set forth in SEQ ID NO: 139, and (c) LCDR3 as set forth in SEQ ID NO: 140, and a heavy chain variable region that comprises (d) HCDR1 as set forth in SEQ ID NO: 142, (e) HCDR2 as set forth in SEQ ID NO: 143, and (f) HCDR3 as set forth in SEQ ID NO: 144;
3) a light chain variable region that comprises (a) LCDR1 as set forth in SEQ ID NO: 146, (b) LCDR2 as set forth in SEQ ID NO: 147, and (c) LCDR3 as set forth in SEQ ID NO: 148, and a heavy chain variable region that comprises (d) HCDR1 as set forth in SEQ ID NO: 150, (e) HCDR2 as set forth in SEQ ID NO: 151, and (f) HCDR3 as set forth in SEQ ID NO: 152; and
4) a light chain variable region that comprises (a) LCDR1 as set forth in SEQ ID NO: 154, (b) LCDR2 as set forth in SEQ ID NO: 155, and (c) LCDR3 as set forth in SEQ ID NO: 156, and a heavy chain variable region that comprises (d) HCDR1 as set forth in SEQ ID NO: 158, (e) HCDR2 as set forth in SEQ ID NO: 159, and (f) HCDR3 as set forth in SEQ ID NO: 160.

In some embodiments, the isolated antibody or the antigen binding fragment thereof comprises a light chain variable region and a heavy chain variable region selected from at least one of the following groups:
1) a light chain variable region that comprises an amino acid sequence as set forth in SEQ ID NO:129, or an amino acid sequence that is at least 85%, at least 90%, at least 95%, at least 96%, at least 97%, at least 98%, or at least 99% identical to the amino acid sequence as set forth in SEQ ID NO:129, and a heavy chain variable region that comprises an amino acid sequence as set forth in SEQ ID NO:133, or an amino acid sequence that is at least 85%, at least 90%, at least 95%, at least 96%, at least 97%, at least 98%, or at least 99% identical to the amino acid sequence as set forth in SEQ ID NO:133;
2) a light chain variable region that comprises an amino acid sequence as set forth in SEQ ID NO:137, or an amino acid sequence that is at least 85%, at least 90%, at least 95%, at least 96%, at least 97%, at least 98%, or at least 99% identical to the amino acid sequence as set forth in SEQ ID NO:137, and a heavy chain variable region that comprises an amino acid sequence as set forth in SEQ ID NO:141, or an amino acid sequence that is at least 85%, at least 90%, at least 95%, at least 96%, at least 97%, at least 98%, or at least 99% identical to the amino acid sequence as set forth in SEQ ID NO:141;
3) a light chain variable region that comprises an amino acid sequence as set forth in SEQ ID NO:145, or an amino acid sequence that is at least 85%, at least 90%, at least 95%, at least 96%, at least 97%, at least 98%, or at least 99% identical to the amino acid sequence as set forth in SEQ ID NO:145, and a heavy chain variable region that comprises an amino acid sequence as set forth in SEQ ID NO:149, or an amino acid sequence that is at least 85%, at least 90%, at least 95%, at least 96%, at least 97%, at least 98%, or at least 99% identical to the amino acid sequence as set forth in SEQ ID NO:149; and
4) a light chain variable region that comprises an amino acid sequence as set forth in SEQ ID NO:153, or an amino acid sequence that is at least 85%, at least 90%, at least 95%, at least 96%, at least 97%, at least 98%, or at least 99% identical to the amino acid sequence as set forth in SEQ ID NO:153, and a heavy chain variable region that comprises an amino acid sequence as set forth in SEQ ID NO:157, or an amino acid sequence that is at least 85%, at least 90%, at least 95%, at least 96%, at least 97%, at least 98%, or at least 99% identical to the amino acid sequence as set forth in SEQ ID NO:157.

In some embodiments, an antibody that is at least 85%, at least 90%, at least 95%, at least 96%, at least 97%, at least 98%, or at least 99% identical to the amino acid sequences of the light chain variable region (SEQ ID NOs: 129, 137, 145 and 153) and amino acid sequences of the heavy chain variable region (SEQ ID NOs: 133, 141, 149 and 157) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the light chain variable region (SEQ ID NOs: 129, 137, 145 and 153) and amino acid sequences of the heavy chain variable region (SEQ ID NOs: 133, 141, 149 and 157) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain variable region (SEQ ID NOs: 129, 137, 145 and 153) and amino acid sequences of the heavy chain variable region (SEQ ID NOs: 133, 141, 149 and 157) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof further comprises a light chain constant region and a heavy chain constant region,
the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201, and
the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.

In some embodiments, the isolated antibody or the antigen binding fragment thereof comprises a light chain and a heavy chain selected from at least one of the following groups:
1) a light chain that comprises an amino acid sequence as set forth in SEQ ID NO:225, or an amino acid sequence that is at least 85%, at least 90%, at least 95%, at least 96%, at least 97%, at least 98%, or at least 99% identical to the amino acid sequence as set forth in SEQ ID NO:225, and a heavy chain that comprises an amino acid sequence as set forth in SEQ ID NO:226, or an amino acid sequence that is at least 85%, at least 90%, at least 95%, at least 96%, at least 97%, at least 98%, or at least 99% identical to the amino acid sequence as set forth in SEQ ID NO:226;
2) a light chain that comprises an amino acid sequence as set forth in SEQ ID NO:227, or an amino acid sequence that is at least 85%, at least 90%, at least 95%, at least 96%, at least 97%, at least 98%, or at least 99% identical to the amino acid sequence as set forth in SEQ ID NO:227, and a heavy chain that comprises an amino acid sequence as set forth in SEQ ID NO:228, or an amino acid sequence that is at least 85%, at least 90%, at least 95%, at least 96%, at least 97%, at least 98%, or at least 99% identical to the amino acid sequence as set forth in SEQ ID NO:228;
3) a light chain that comprises an amino acid sequence as set forth in SEQ ID NO:229, or an amino acid sequence that is at least 85%, at least 90%, at least 95%, at least 96%, at least 97%, at least 98%, or at least 99% identical to the amino acid sequence as set forth in SEQ ID NO:229, and a heavy chain that comprises an amino acid sequence as set forth in SEQ ID NO:230, or an amino acid sequence that is at least 85%, at least 90%, at least 95%, at least 96%, at least 97%, at least 98%, or at least 99% identical to the amino acid sequence as set forth in SEQ ID NO:230;
4) a light chain that comprises an amino acid sequence as set forth in SEQ ID NO:231, or an amino acid sequence that is at least 85%, at least 90%, at least 95%, at least 96%, at least 97%, at least 98%, or at least 99% identical to the amino acid sequence as set forth in SEQ ID NO:231, and a heavy chain that comprises an amino acid sequence as set forth in SEQ ID NO:232, or an amino acid sequence that is at least 85%, at least 90%, at least 95%, at least 96%, at least 97%, at least 98%, or at least 99% identical to the amino acid sequence as set forth in SEQ ID NO:232.

In some embodiments, an antibody that is at least 85%, at least 90%, at least 95%, at least 96%, at least 97%, at least 98%, or at least 99% identical to the amino acid sequences of the light chain (SEQ ID NOs: 225, 227, 229 and 231) and amino acid sequences of the heavy chain (SEQ ID NOs: 226, 228, 230 and 232) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the light chain (SEQ ID NOs: 225, 227, 229 and 231) and amino acid sequences of the heavy chain (SEQ ID NOs: 226, 228, 230 and 232) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain (SEQ ID NOs: 225, 227, 229 and 231) and amino acid sequences of the heavy chain (SEQ ID NOs: 226, 228, 230 and 232) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof consists of a light chain and a heavy chain selected from at least one of the following groups:
1) a light chain that consists of an amino acid sequence as set forth in SEQ ID NO:225, and a heavy chain that consists of an amino acid sequence as set forth in SEQ ID NO:226;
2) a light chain that consists of an amino acid sequence as set forth in SEQ ID NO:227, and a heavy chain that consists of an amino acid sequence as set forth in SEQ ID NO:228;
3) a light chain that consists of an amino acid sequence as set forth in SEQ ID NO:229, and a heavy chain that consists of an amino acid sequence as set forth in SEQ ID NO:230;
4) a light chain that consists of an amino acid sequence as set forth in SEQ ID NO:231, and a heavy chain that consists of an amino acid sequence as set forth in SEQ ID NO:232.

In the fourth aspect, the present invention provides an isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 162, (b) LCDR2 as set forth in SEQ ID NO: 163, and (c) LCDR3 as set forth in SEQ ID NO: 164, and a heavy chain variable region that comprises (d) HCDR1 as set forth in SEQ ID NO: 166, (e) HCDR2 as set forth in SEQ ID NO: 167, and (f) HCDR3 as set forth in SEQ ID NO: 168;
2) a light chain variable region that comprises (a) LCDR1 as set forth in SEQ ID NO: 170, (b) LCDR2 as set forth in SEQ ID NO: 171, and (c) LCDR3 as set forth in SEQ ID NO: 172, and a heavy chain variable region that comprises (d) HCDR1 as set forth in SEQ ID NO: 174, (e) HCDR2 as set forth in SEQ ID NO: 175, and (f) HCDR3 as set forth in SEQ ID NO:176;
3) a light chain variable region that comprises (a) LCDR1 as set forth in SEQ ID NO: 178, (b) LCDR2 as set forth in SEQ ID NO: 179, and (c) LCDR3 as set forth in SEQ ID NO: 180, and a heavy chain variable region that comprises (d) HCDR1 as set forth in SEQ ID NO: 182, (e) HCDR2 as set forth in SEQ ID NO: 183, and (f) HCDR3 as set forth in SEQ ID NO: 184;
4) a light chain variable region that comprises (a) LCDR1 as set forth in SEQ ID NO: 186, (b) LCDR2 as set forth in SEQ ID NO: 187, and (c) LCDR3 as set forth in SEQ ID NO: 188, and a heavy chain variable region that comprises (d) HCDR1 as set forth in SEQ ID NO: 190, (e) HCDR2 as set forth in SEQ ID NO: 191, and (f) HCDR3 as set forth in SEQ ID NO: 192; and
5) a light chain variable region that comprises (a) LCDR1 as set forth in SEQ ID NO: 194, (b) LCDR2 as set forth in SEQ ID NO: 195, and (c) LCDR3 as set forth in SEQ ID NO: 196, and a heavy chain variable region that comprises (d) HCDR1 as set forth in SEQ ID NO: 198, (e) HCDR2 as set forth in SEQ ID NO: 199, and (f) HCDR3 as set forth in SEQ ID NO: 200.

In some embodiments, the isolated antibody or the antigen binding fragment thereof comprises a light chain variable region and a heavy chain variable region selected from at least one of the following groups:
1) a light chain variable region that comprises an amino acid sequence as set forth in SEQ ID NO:161, or an amino acid sequence that is at least 85%, at least 90%, at least 95%, at least 96%, at least 97%, at least 98%, or at least 99% identical to the amino acid sequence as set forth in SEQ ID NO:161, and a heavy chain variable region that comprises an amino acid sequence as set forth in SEQ ID NO:165, or an amino acid sequence that is at least 85%, at least 90%, at least 95%, at least 96%, at least 97%, at least 98%, or at least 99% identical to the amino acid sequence as set forth in SEQ ID NO:165;
2) a light chain variable region that comprises an amino acid sequence as set forth in SEQ ID NO:169, or an amino acid sequence that is at least 85%, at least 90%, at least 95%, at least 96%, at least 97%, at least 98%, or at least 99% identical to the amino acid sequence as set forth in SEQ ID NO:169, and a heavy chain variable region that comprises an amino acid sequence as set forth in SEQ ID NO:173, or an amino acid sequence that is at least 85%, at least 90%, at least 95%, at least 96%, at least 97%, at least 98%, or at least 99% identical to the amino acid sequence as set forth in SEQ ID NO:173;
3) a light chain variable region that comprises an amino acid sequence as set forth in SEQ ID NO:177, or an amino acid sequence that is at least 85%, at least 90%, at least 95%, at least 96%, at least 97%, at least 98%, or at least 99% identical to the amino acid sequence as set forth in SEQ ID NO:177, and a heavy chain variable region that comprises an amino acid sequence as set forth in SEQ ID NO:181, or an amino acid sequence that is at least 85%, at least 90%, at least 95%, at least 96%, at least 97%, at least 98%, or at least 99% identical to the amino acid sequence as set forth in SEQ ID NO:181;
4) a light chain variable region that comprises an amino acid sequence as set forth in SEQ ID NO:185, or an amino acid sequence that is at least 85%, at least 90%, at least 95%, at least 96%, at least 97%, at least 98%, or at least 99% identical to the amino acid sequence as set forth in SEQ ID NO:185, and a heavy chain variable region that comprises an amino acid sequence as set forth in SEQ ID NO:189, or an amino acid sequence that is at least 85%, at least 90%, at least 95%, at least 96%, at least 97%, at least 98%, or at least 99% identical to the amino acid sequence as set forth in SEQ ID NO:189; and
5) a light chain variable region that comprises an amino acid sequence as set forth in SEQ ID NO: 193, or an amino acid sequence that is at least 85%, at least 90%, at least 95%, at least 96%, at least 97%, at least 98%, or at least 99% identical to the amino acid sequence as set forth in SEQ ID NO: 193, and a heavy chain variable region that comprises an amino acid sequence as set forth in SEQ ID NO:197, or an amino acid sequence that is at least 85%, at least 90%, at least 95%, at least 96%, at least 97%, at least 98%, or at least 99% identical to the amino acid sequence as set forth in SEQ ID NO:197.

In some embodiments, an antibody that is at least 85%, at least 90%, at least 95%, at least 96%, at least 97%, at least 98%, or at least 99% identical to the amino acid sequences of the light chain variable region (SEQ ID NOs: 161, 169, 177, 185 and 193) and amino acid sequences of the heavy chain variable region (SEQ ID NOs: 165, 173, 181, 189 and 197) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the light chain variable region (SEQ ID NOs: 161, 169, 177, 185 and 193) and amino acid sequences of the heavy chain variable region (SEQ ID NOs: 165, 173, 181, 189 and 197) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain variable region (SEQ ID NOs: 161, 169, 177, 185 and 193) and amino acid sequences of the heavy chain variable region (SEQ ID NOs: 165, 173, 181, 189 and 197) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof further comprises a light chain constant region and a heavy chain constant region,
the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201, and
the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.

In some embodiments, the isolated antibody or the antigen binding fragment thereof comprises a light chain and a heavy chain selected from at least one of the following groups:
1) a light chain that comprises an amino acid sequence as set forth in SEQ ID NO:233, or an amino acid sequence that is at least 85%, at least 90%, at least 95%, at least 96%, at least 97%, at least 98%, or at least 99% identical to the amino acid sequence as set forth in SEQ ID NO:233, and a heavy chain that comprises an amino acid sequence as set forth in SEQ ID NO:234, or an amino acid sequence that is at least 85%, at least 90%, at least 95%, at least 96%, at least 97%, at least 98%, or at least 99% identical to the amino acid sequence as set forth in SEQ ID NO:234;
2) a light chain that comprises an amino acid sequence as set forth in SEQ ID NO:235, or an amino acid sequence that is at least 85%, at least 90%, at least 95%, at least 96%, at least 97%, at least 98%, or at least 99% identical to the amino acid sequence as set forth in SEQ ID NO:235, and a heavy chain that comprises an amino acid sequence as set forth in SEQ ID NO:236, or an amino acid sequence that is at least 85%, at least 90%, at least 95%, at least 96%, at least 97%, at least 98%, or at least 99% identical to the amino acid sequence as set forth in SEQ ID NO:236;
3) a light chain that comprises an amino acid sequence as set forth in SEQ ID NO:237, or an amino acid sequence that is at least 85%, at least 90%, at least 95%, at least 96%, at least 97%, at least 98%, or at least 99% identical to the amino acid sequence as set forth in SEQ ID NO:237, and a heavy chain that comprises an amino acid sequence as set forth in SEQ ID NO:238, or an amino acid sequence that is at least 85%, at least 90%, at least 95%, at least 96%, at least 97%, at least 98%, or at least 99% identical to the amino acid sequence as set forth in SEQ ID NO:238;
4) a light chain that comprises an amino acid sequence as set forth in SEQ ID NO:239, or an amino acid sequence that is at least 85%, at least 90%, at least 95%, at least 96%, at least 97%, at least 98%, or at least 99% identical to the amino acid sequence as set forth in SEQ ID NO:239, and a heavy chain that comprises an amino acid sequence as set forth in SEQ ID NO:240, or an amino acid sequence that is at least 85%, at least 90%, at least 95%, at least 96%, at least 97%, at least 98%, or at least 99% identical to the amino acid sequence as set forth in SEQ ID NO:240; and
5) a light chain that comprises an amino acid sequence as set forth in SEQ ID NO:241, or an amino acid sequence that is at least 85%, at least 90%, at least 95%, at least 96%, at least 97%, at least 98%, or at least 99% identical to the amino acid sequence as set forth in SEQ ID NO:241, and a heavy chain that comprises an amino acid sequence as set forth in SEQ ID NO:242, or an amino acid sequence that is at least 85%, at least 90%, at least 95%, at least 96%, at least 97%, at least 98%, or at least 99% identical to the amino acid sequence as set forth in SEQ ID NO:242.

In some embodiments, an antibody that is at least 85%, at least 90%, at least 95%, at least 96%, at least 97%, at least 98%, or at least 99% identical to the amino acid sequences of the light chain (SEQ ID NOs: 233, 235, 237, 239 and 241) and amino acid sequences of the heavy chain (SEQ ID NOs: 234, 236, 238, 240 and 242) of the antibody according to the present invention has the same or similar biological functions and/or activity as the antibody according to the present invention. In some embodiments, an antibody that is at least 85%, at least 90%, at least 95%, at least 96%, at least 97%, at least 98%, or at least 99% identical to the amino acid sequences of the light chain (SEQ ID NOs: 233, 235, 237, 239 and 241) and amino acid sequences of the heavy chain (SEQ ID NOs: 234, 236, 238, 240 and 242) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the light chain (SEQ ID NOs: 233, 235, 237, 239 and 241) and amino acid sequences of the heavy chain (SEQ ID NOs: 234, 236, 238, 240 and 242) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody (for example, not changing the function and/or activity of specifically binding to the ITGA2 protein).

In some embodiments, the isolated antibody or the antigen binding fragment thereof consists of a light chain and a heavy chain selected from at least one of the following groups:
1) a light chain that consists of an amino acid sequence as set forth in SEQ ID NO:233, and a heavy chain that consists of an amino acid sequence as set forth in SEQ ID NO:234;
2) a light chain that consists of an amino acid sequence as set forth in SEQ ID NO:235, and a heavy chain that consists of an amino acid sequence as set forth in SEQ ID NO:236;
3) a light chain that consists of an amino acid sequence as set forth in SEQ ID NO:237, and a heavy chain that consists of an amino acid sequence as set forth in SEQ ID NO:238;
4) a light chain that consists of an amino acid sequence as set forth in SEQ ID NO:239, and a heavy chain that consists of an amino acid sequence as set forth in SEQ ID NO:240; and
5) a light chain that consists of an amino acid sequence as set forth in SEQ ID NO:241, and a heavy chain that consists of an amino acid sequence as set forth in SEQ ID NO:242.

In some embodiments, the antibody is capable of binding to an ITGA2 protein in vitro at an EC50 of less than 1 nM.

In some embodiments, the antibody is capable of binding to an ITGA2 protein on the surface of HuCCT1 cells with an EC50 of less than 5 nM.

In some embodiments, wherein the antibody is a monoclonal antibody, a polyclonal antibody, a chimeric antibody, or a humanized antibody.

The amino acid sequence numberings (SEQ ID NO:) of the antibody according to the present invention and the corresponding structure thereof are summarized in Table 2 below.

**Table 2a - Chimeric mouse antibody FS002-A103 and the corresponding humanized antibody thereof**

| Antibody | Light Chain Variable Region | VL CDR1-3 | Light Chain Constant Region | Light Chain Amino Acid Sequence | Heavy Chain Variable Region | VH CDR1-3 | Heavy Chain Constant Region | Heavy Chain Amino Acid Sequence |
|---|---|---|---|---|---|---|---|---|
| FS002-A103 | 41 | 42-44 | 201 | 203 | 45 | 46-48 | 202 | 204 |
| FS002-A103-H4L2 | 49 | 50-52 | 201 | 205 | 53 | 54-56 | 202 | 206 |
| FS002-A103-H4L3 | 57 | 58-60 | 201 | 207 | 61 | 62-64 | 202 | 208 |
| FS002-A103-H4L4 | 65 | 66-68 | 201 | 209 | 69 | 70-72 | 202 | 210 |

**Table 2b - Chimeric mouse antibody FS002-A153 and the corresponding humanized antibody thereof**

| Antibody | Light Chain Variable Region | VL CDR1-3 | Light Chain Constant Region | Light Chain Amino Acid Sequence | Heavy Chain Variable Region | VH CDR1-3 | Heavy Chain Constant Region | Heavy Chain Amino Acid Sequence |
|---|---|---|---|---|---|---|---|---|
| FS002-A153 | 73 | 74-76 | 201 | 211 | 77 | 78-80 | 202 | 212 |
| FS002-A153-H3L3 | 81 | 82-84 | 201 | 213 | 85 | 86-88 | 202 | 214 |
| FS002-A153-H4L2 | 89 | 90-92 | 201 | 215 | 93 | 94-96 | 202 | 216 |
| FS002-A153-H5L1 | 97 | 98-100 | 201 | 217 | 101 | 102-104 | 202 | 218 |
| FS002-A153-H5L2 | 105 | 106-108 | 201 | 219 | 109 | 110-112 | 202 | 220 |
| FS002-A153-H5L4 | 113 | 114-116 | 201 | 221 | 117 | 118-120 | 202 | 222 |
| FS002-A153-H5L5 | 121 | 122-124 | 201 | 223 | 125 | 126-128 | 202 | 224 |

**Table 2c - Chimeric mouse antibody FS002-A208 and the corresponding humanized antibody thereof**

| Antibody | Light Chain Variable Region | VL CDR1-3 | Light Chain Constant Region | Light Chain Amino Acid Sequence | Heavy Chain Variable Region | VH CDR1-3 | Heavy Chain Constant Region | Heavy Chain Amino Acid Sequence |
|---|---|---|---|---|---|---|---|---|
| FS002-A208 | 129 | 130-132 | 201 | 225 | 133 | 134-136 | 202 | 226 |
| FS002-A208-H3L4 | 137 | 138-140 | 201 | 227 | 141 | 142-144 | 202 | 228 |
| FS002-A208-H4L4 | 145 | 146-148 | 201 | 229 | 149 | 150-152 | 202 | 230 |
| FS002-A208-H5L4 | 153 | 154-156 | 201 | 231 | 157 | 158-160 | 202 | 232 |

**Table 2d - Chimeric mouse antibody FS002-A226 and the corresponding humanized antibody thereof**

| Antibody | Light Chain Variable Region | VL CDR1-3 | Light Chain Constant Region | Light Chain Amino Acid Sequence | Heavy Chain Variable Region | VH CDR1-3 | Heavy Chain Constant Region | Heavy Chain Amino Acid Sequence |
|---|---|---|---|---|---|---|---|---|
| FS002-A226 | 161 | 162-164 | 201 | 233 | 165 | 166-168 | 202 | 234 |
| FS002-A226-H3L4 | 169 | 170-172 | 201 | 235 | 173 | 174-176 | 202 | 236 |
| FS002-A226-H4L3 | 177 | 178-180 | 201 | 237 | 181 | 182-184 | 202 | 238 |
| FS002-A226-H5L3 | 185 | 186-188 | 201 | 239 | 189 | 190-192 | 202 | 240 |
| FS002-A226-H5L4 | 193 | 194-196 | 201 | 241 | 197 | 198-200 | 202 | 242 |

In another aspect, the present invention provides an isolated antibody specifically binding to an ITGA2 protein or an antigen-binding fragment thereof, wherein the isolated antibody or the antigen-binding fragment thereof comprises a light chain and a heavy chain selected from at least one of the following groups:
1) a light chain that comprises an amino acid sequence encoded by a nucleotide sequence as set forth in SEQ ID NO:1, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:1, or consists of an amino acid sequence encoded by the nucleotide sequence as set forth in SEQ ID NO:1, and
   a heavy chain that comprises an amino acid sequence encoded by a nucleotide sequence as set forth in SEQ ID NO:2, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:2, or consists of an amino acid sequence encoded by the nucleotide sequence as set forth in SEQ ID NO:2;
2) a light chain that comprises an amino acid sequence encoded by a nucleotide sequence as set forth in SEQ ID NO:3, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:3, or consists of an amino acid sequence encoded by the nucleotide sequence as set forth in SEQ ID NO:3, and
   a heavy chain that comprises an amino acid sequence encoded by a nucleotide sequence as set forth in SEQ ID NO:4, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:4, or consists of an amino acid sequence encoded by the nucleotide sequence as set forth in SEQ ID NO:4;
3) a light chain that comprises an amino acid sequence encoded by a nucleotide sequence as set forth in SEQ ID NO:5, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:5, or consists of an amino acid sequence encoded by the nucleotide sequence as set forth in SEQ ID NO:5, and
   a heavy chain that comprises an amino acid sequence encoded by a nucleotide sequence as set forth in SEQ ID NO:6, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:6, or consists of an amino acid sequence encoded by the nucleotide sequence as set forth in SEQ ID NO:6; and
4) a light chain that comprises an amino acid sequence encoded by a nucleotide sequence as set forth in SEQ ID NO:7, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:7, or consists of an amino acid sequence encoded by the nucleotide sequence as set forth in SEQ ID NO:7, and
   a heavy chain that comprises an amino acid sequence encoded by a nucleotide sequence as set forth in SEQ ID NO:8, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:8, or consists of an amino acid sequence encoded by the nucleotide sequence as set forth in SEQ ID NO:8.

In another aspect, the present invention provides an isolated antibody specifically binding to an ITGA2 protein or an antigen-binding fragment thereof, wherein the isolated antibody or the antigen-binding fragment thereof comprises a light chain and a heavy chain selected from at least one of the following groups:
1) a light chain that comprises an amino acid sequence encoded by a nucleotide sequence as set forth in SEQ ID NO:9, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:9, or consists of an amino acid sequence encoded by the nucleotide sequence as set forth in SEQ ID NO:9, and
   a heavy chain that comprises an amino acid sequence encoded by a nucleotide sequence as set forth in SEQ ID NO:10, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:10, or consists of an amino acid sequence encoded by the nucleotide sequence as set forth in SEQ ID NO:10;
2) a light chain that comprises an amino acid sequence encoded by a nucleotide sequence as set forth in SEQ ID NO:11, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:11, or consists of an amino acid sequence encoded by the nucleotide sequence as set forth in SEQ ID NO:11, and
   a heavy chain that comprises an amino acid sequence encoded by a nucleotide sequence as set forth in SEQ ID NO:12, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:12, or consists of an amino acid sequence encoded by the nucleotide sequence as set forth in SEQ ID NO:12;
3) a light chain that comprises an amino acid sequence encoded by a nucleotide sequence as set forth in SEQ ID NO:13, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:13, or consists of an amino acid sequence encoded by the nucleotide sequence as set forth in SEQ ID NO:13, and
   a heavy chain that comprises an amino acid sequence encoded by a nucleotide sequence as set forth in SEQ ID NO:14, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:14, or consists of an amino acid sequence encoded by the nucleotide sequence as set forth in SEQ ID NO:14;
4) a light chain that comprises an amino acid sequence encoded by a nucleotide sequence as set forth in SEQ ID NO:15, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:15, or consists of an amino acid sequence encoded by the nucleotide sequence as set forth in SEQ ID NO:15, and
   a heavy chain that comprises an amino acid sequence encoded by a nucleotide sequence as set forth in SEQ ID NO:16, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:16, or consists of an amino acid sequence encoded by the nucleotide sequence as set forth in SEQ ID NO:16;
5) a light chain that comprises an amino acid sequence encoded by a nucleotide sequence as set forth in SEQ ID NO:17, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:17, or consists of an amino acid sequence encoded by the nucleotide sequence as set forth in SEQ ID NO:17, and
   a heavy chain that comprises an amino acid sequence encoded by a nucleotide sequence as set forth in SEQ ID NO:18, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:18, or consists of an amino acid sequence encoded by the nucleotide sequence as set forth in SEQ ID NO:18;
6) a light chain that comprises an amino acid sequence encoded by a nucleotide sequence as set forth in SEQ ID NO: 19, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:19, or consists of an amino acid sequence encoded by the nucleotide sequence as set forth in SEQ ID NO: 19, and
   a heavy chain that comprises an amino acid sequence encoded by a nucleotide sequence as set forth in SEQ ID NO:20, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:20, or consists of an amino acid sequence encoded by the nucleotide sequence as set forth in SEQ ID NO:20; and
7) a light chain that comprises an amino acid sequence encoded by a nucleotide sequence as set forth in SEQ ID NO:21, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:21, or consists of an amino acid sequence encoded by the nucleotide sequence as set forth in SEQ ID NO:21, and
   a heavy chain that comprises an amino acid sequence encoded by a nucleotide sequence as set forth in SEQ ID NO:22, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:22, or consists of an amino acid sequence encoded by the nucleotide sequence as set forth in SEQ ID NO:22.

In another aspect, the present invention provides an isolated antibody specifically binding to an ITGA2 protein or an antigen-binding fragment thereof, wherein the isolated antibody or the antigen-binding fragment thereof comprises a light chain and a heavy chain selected from at least one of the following groups:
1) a light chain that comprises an amino acid sequence encoded by a nucleotide sequence as set forth in SEQ ID NO:23, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:23, or consists of an amino acid sequence encoded by the nucleotide sequence as set forth in SEQ ID NO:23, and
   a heavy chain that comprises an amino acid sequence encoded by a nucleotide sequence as set forth in SEQ ID NO:24, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:24, or consists of an amino acid sequence encoded by the nucleotide sequence as set forth in SEQ ID NO:24;
2) a light chain that comprises an amino acid sequence encoded by a nucleotide sequence as set forth in SEQ ID NO:25, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:25, or consists of an amino acid sequence encoded by the nucleotide sequence as set forth in SEQ ID NO:25, and
   a heavy chain that comprises an amino acid sequence encoded by a nucleotide sequence as set forth in SEQ ID NO:26, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:26, or consists of an amino acid sequence encoded by the nucleotide sequence as set forth in SEQ ID NO:26;
3) a light chain that comprises an amino acid sequence encoded by a nucleotide sequence as set forth in SEQ ID NO:27, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:27, or consists of an amino acid sequence encoded by the nucleotide sequence as set forth in SEQ ID NO:27, and
   a heavy chain that comprises an amino acid sequence encoded by a nucleotide sequence as set forth in SEQ ID NO:28, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:28, or consists of an amino acid sequence encoded by the nucleotide sequence as set forth in SEQ ID NO:28; and
4) a light chain that comprises an amino acid sequence encoded by a nucleotide sequence as set forth in SEQ ID NO:29, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:29, or consists of an amino acid sequence encoded by the nucleotide sequence as set forth in SEQ ID NO:29, and
a heavy chain that comprises an amino acid sequence encoded by a nucleotide sequence as set forth in SEQ ID NO:30, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:30, or consists of an amino acid sequence encoded by the nucleotide sequence as set forth in SEQ ID NO:30.

In another aspect, the present invention provides an isolated antibody specifically binding to an ITGA2 protein or an antigen-binding fragment thereof, wherein the isolated antibody or the antigen-binding fragment thereof comprises a light chain and a heavy chain selected from at least one of the following groups:
1) a light chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31, and
   a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32;
2) a light chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33, and
   a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34;
3) a light chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35, and
   a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36;
4) a light chain that comprises an amino acid sequence encoded by a nucleotide sequence as set forth in SEQ ID NO:37, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:37, or consists of an amino acid sequence encoded by the nucleotide sequence as set forth in SEQ ID NO:37, and
   a heavy chain that comprises an amino acid sequence encoded by a nucleotide sequence as set forth in SEQ ID NO:38, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:38, or consists of an amino acid sequence encoded by the nucleotide sequence as set forth in SEQ ID NO:38; and
5) a light chain that comprises an amino acid sequence encoded by a nucleotide sequence as set forth in SEQ ID NO:39, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:39, or consists of an amino acid sequence encoded by the nucleotide sequence as set forth in SEQ ID NO:39, and
   a heavy chain that comprises an amino acid sequence encoded by a nucleotide sequence as set forth in SEQ ID NO:40, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:40, or consists of an amino acid sequence encoded by the nucleotide sequence as set forth in SEQ ID NO:40.

In some embodiments, the antibody or the antigen-binding fragment thereof is capable of binding the ITGA2 protein in vitro at an EC50 of less than 1 nM.

In some embodiments, the antibody or the antigen-binding fragment thereof is capable of binding to an ITGA2 protein on the surface of HuCCT1 cells with an EC50 of less than 5 nM.

In some embodiments, the antibody is a monoclonal antibody, a polyclonal antibody, a chimeric antibody, or a humanized antibody.

### III. Nucleic Acid, Vector, Host Cell, Hybridoma, and Method for Preparing Antibody

In one aspect, the invention provides an isolated nucleic acid molecule encoding the antibody or the antigen-binding fragment thereof according to the present invention described above. In some embodiments, the nucleic acid molecules according to the present invention encode the heavy chain and/or light chain variable region, each CDR in the heavy chain and/or light chain variable region, and the heavy chain and/or light chain constant region of the antibody according to the present invention described above. In some embodiments, the nucleotide sequence of the nucleic acid molecule is codon optimized for a host cell for expression. In some embodiments, the nucleic acid molecule according to the invention is operably linked to an expression regulatory sequence. The present invention further provides an isolated nucleic acid molecule encoding the antibody or the antigen-binding fragment thereof according to the present invention.

In one aspect, the present invention provides an isolated nucleic acid molecule, wherein the nucleic acid molecule comprises or consists of a nucleotide sequence combination selected from any one of the following nucleotide sequences: SEQ ID NO:1 and SEQ ID NO:2; SEQ ID NO:3 and SEQ ID NO:4; SEQ ID NO:5 and SEQ ID NO:6; and SEQ ID NO:7 and SEQ ID NO:8.

In one aspect, the present invention provides an isolated nucleic acid molecule, wherein the nucleic acid molecule comprises or consists of a nucleotide sequence combination selected from any one of the following nucleotide sequences: SEQ ID NO:9 and SEQ ID NO:10; SEQ ID NO:11 and SEQ ID NO:12; SEQ ID NO:13 and SEQ ID NO:14; SEQ ID NO:15 and SEQ ID NO:16; SEQ ID NO:17 and SEQ ID NO:18; SEQ ID NO:19 and SEQ ID NO:20; and SEQ ID NO:21 and SEQ ID NO:22.

In one aspect, the present invention provides an isolated nucleic acid molecule, wherein the nucleic acid molecule comprises or consists of a nucleotide sequence combination selected from any one of the following nucleotide sequences: SEQ ID NO:23 and SEQ ID NO:24; SEQ ID NO:25 and SEQ ID NO:26; SEQ ID NO:27 and SEQ ID NO:28; and SEQ ID NO:29 and SEQ ID NO:30.

In one aspect, the present invention provides an isolated nucleic acid molecule, wherein the nucleic acid molecule comprises or consists of a nucleotide sequence combination selected from any one of the following nucleotide sequences: SEQ ID NO:31 and SEQ ID NO:32; SEQ ID NO:33 and SEQ ID NO:34; SEQ ID NO:35 and SEQ ID NO:36; SEQ ID NO:37 and SEQ ID NO:38; and SEQ ID NO:39 and SEQ ID NO:40.

In some embodiments, the nucleic acid molecule is operably linked to an expression regulatory sequence.

The nucleotide sequence numberings (SEQ ID NO:) of the antibody according to the present invention and the corresponding structure thereof are summarized in Table 3 below:

**Table 3a - Nucleotide sequences encoding the light chains and heavy chains of the chimeric mouse antibody FS002-A103 and the corresponding humanized antibody thereof**

| Antibody | Light Chain | Nucleotide Sequence Encoding Light Chain | Heavy Chain | Nucleotide Sequence Encoding Heavy Chain |
|---|---|---|---|---|
| FS002-A103 | FS002-A103-LC(Kappa) | 1 | FS002-A103-HC(IgG1) | 2 |
| FS002-A103-H4L2 | FS002-A103-L2-LC(Kappa) | 3 | FS002-A103-H4-HC(IgG1) | 4 |
| FS002-A103-H4L3 | FS002-A103-L3-LC(Kappa) | 5 | FS002-A103-H4-HC(IgG1) | 6 |
| FS002-A103-H4L4 | FS002-A103-L4-LC(Kappa) | 7 | FS002-A103-H4-HC(IgG1) | 8 |

**Table 3b - Nucleotide sequences encoding the light chains and heavy chains of the chimeric mouse antibody FS002-A153 and the corresponding humanized antibody thereof**

| Antibody | Light Chain | Nucleotide Sequence Encoding Light Chain | Heavy Chain | Nucleotide Sequence Encoding Heavy Chain |
|---|---|---|---|---|
| FS002-A153 | FS002-A153-LC(Kappa) | 9 | FS002-A153-HC(IgG1) | 10 |
| FS002-A153-H3L3 | FS002-A153-L3-LC(Kappa) | 11 | FS002-A153-H3-HC(IgG1) | 12 |
| FS002-A153-H4L2 | FS002-A153-L2-LC(Kappa) | 13 | FS002-A153-H4-HC(IgG1) | 14 |
| FS002-A153-H5L1 | FS002-A153-L1-LC(Kappa) | 15 | FS002-A153-H5-HC(IgG1) | 16 |
| FS002-A153-H5L2 | FS002-A153-L2-LC(Kappa) | 17 | FS002-A153-H5-HC(IgG1) | 18 |
| FS002-A153-H5L4 | FS002-A153-L4-LC(Kappa) | 19 | FS002-A153-H5-HC(IgG1) | 20 |
| FS002-A153-H5L5 | FS002-A153-L5-LC(Kappa) | 21 | FS002-A153-H5-HC(IgG1) | 22 |

**Table 3c - Nucleotide sequences encoding the light chains and heavy chains of the chimeric mouse antibody FS002-A208 and the corresponding humanized antibody thereof**

| Antibody | Light Chain | Nucleotide Sequence Encoding Light Chain | Heavy Chain | Nucleotide Sequence Encoding Heavy Chain |
|---|---|---|---|---|
| FS002-A208 | FS002-A208-LC(Kappa) | 23 | FS002-A208-HC(IgG1) | 24 |
| FS002-A208-H3L4 | FS002-A208-L4-LC(Kappa) | 25 | FS002-A208-H3-HC(IgG1) | 26 |
| FS002-A208-H4L4 | FS002-A208-L4-LC(Kappa) | 27 | FS002-A208-H4-HC(IgG1) | 28 |
| FS002-A208-H5L4 | FS002-A208-L4-LC(Kappa) | 29 | FS002-A208-H5-HC(IgG1) | 30 |

**Table 3d - Nucleotide sequences encoding the light chains and heavy chains of the chimeric mouse antibody FS002-A226 and the corresponding humanized antibody thereof**

| Antibody | Light Chain | Nucleotide Sequence Encoding Light Chain | Heavy Chain | Nucleotide Sequence Encoding Heavy Chain |
|---|---|---|---|---|
| FS002-A226 | FS002-A226-LC(Kappa) | 31 | FS002-A226-HC(IgG1) | 32 |
| FS002-A226-H3L4 | FS002-A226-L4-LC(Kappa) | 33 | FS002-A226-H3-HC(IgG1) | 34 |
| FS002-A226-H4L3 | FS002-A226-L3-LC(Kappa) | 35 | FS002-A226-H4-HC(IgG1) | 36 |
| FS002-A226-H5L3 | FS002-A226-L3-LC(Kappa) | 37 | FS002-A226-H5-HC(IgG1) | 38 |
| FS002-A226-H5L4 | FS002-A226-L4-LC(Kappa) | 39 | FS002-A226-H5-HC(IgG1) | 40 |

The invention further provides an expression vector comprising at least one of the nucleic acid molecules according to the invention described above.

The invention further provides a host cell transformed with at least one of the nucleic acid molecules or expression vectors according the invention described above.

The present invention further provides a hybridoma cell expressing at least one of the antibody or the antigen-binding fragment thereof according to the present invention described above.

The invention further provides a method for producing the antibody or the antigen-binding fragment thereof according to the invention, comprising:
(i) culturing the host cell of the present invention in a condition suitable for expressing the nucleic acid molecule or the expression vector, and
(ii) isolating and purifying the antibody or antigen-binding fragment thereof expressed by the host cell.

In some embodiments, the nucleotide sequence of the nucleic acid molecule is codon optimized for a host cell for expression. In some embodiments, the nucleic acid molecule is operably linked to an expression regulatory sequence.

The invention further provides a method for producing the antibody or the antigen-binding fragment thereof according to the invention, comprising:
(i) preparing a hybridoma cell capable of expressing the monoclonal antibody or the antigen-binding fragment thereof according to the present invention, and
(ii) isolating and purifying the antibody or antigen-binding fragment thereof expressed by the hybridoma cell.

### IV. Antibody-drug Conjugate

In one aspect, the invention provides an antibody-drug conjugate (ADC) prepared by the antibody or the antigen-binding fragment thereof according to the invention.

Specifically, the invention provides an antibody-drug conjugate of the formula Ab-L-D, where:
"Ab" represents at least one monoclonal antibody or the antigen-binding fragment thereof according to the present invention described above;
"L" represents the linker that connects the antibody moiety to the partner molecule; and
"D" represents the partner molecule.

As used herein, the ADC is prepared by a linker that link a partner molecule to the antibody or the antigen-binding fragment thereof. In the ADC according to the present invention, the antibody performs a targeting function: by binding to a target tissue or cell of the antigen in which it is found, and the antibody directs the conjugate to the target tissue or cell. In that place, the linker is cleaved, for example, releasing a partner molecule to perform its desired biological function.

The partner molecule may be a drug payload or a marker molecule. The drug payload may be a therapeutic agent, where for example, the therapeutic agent may be a cytotoxic agent, a chemotherapeutic agent, a cytostatic agent, an immunomodulator, and the like. In some embodiments, the therapeutic agent is a cytotoxic agent, i.e., a cytotoxin. As used herein, the terms "cytotoxic agent" and "cytotoxin" are used interchangeably.

A marker molecule may be any marker that produces a detectable signal, such as a radioactive label, a fluorescent label, or a detectable modification enzyme for a catalytic substrate.

As used herein, the terms "drug", "payload" and "drug payload" are used interchangeably. As used herein, the antibody-drug conjugate (ADC) covers a conjugate formed by connecting the antibody or the antigen-binding fragment thereof according to the present invention to a drug payload, and a conjugate formed by connecting the antibody or the antigen-binding fragment thereof according to the present invention to a marker molecule.

The proportion of partner molecules linked to the antibody may vary depending on factors such as the amount of partner molecules used during the conjugation reaction and the experimental conditions. The ratio of the partner molecule to the antibody may be 1 to 6, 1 to 5, 1 to 4, 1 to 3, 1 to 2, or 1 to 1.5.

Therefore, the present invention provides an antibody-drug conjugate of the formula Ab-(L-D)p, where:
"Ab" represents at least one monoclonal antibody or the antigen-binding fragment thereof according to the present invention described above;
"L" represents the linker that connects the antibody moiety to the partner molecule;
"D" represents the partner molecule; and
"p" represents the number of linker/partner molecules (preferably drug payloads) conjugated to the monoclonal antibody or the antigen-binding fragment thereof according to the present invention described above.

In some embodiments, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.

In some embodiments, the linker according to the present invention is selected from one of the following: vc, mc-GGFG, AcLysvc, mc, MalPeg6, m(H20)c and m(H20)cvc; and/or the linker is cleavable. In some embodiments, the linker according to the present invention is vc.

In some embodiments, the partner molecule according to the present invention is a drug payload, wherein the drug payload is a therapeutic molecule. Optionally, the drug payload is selected from: a cytotoxic agent, a cytostatic agent, an immunomodulator and a chemotherapeutic agent. Optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD.

In some embodiments, the drug payload is MMAE. "MMAE" refers to monomethyl auristatin E, as shown in the figure below, wherein the wavy lines indicate that it is covalently connected to the linker (L) of the antibody-drug conjugate:

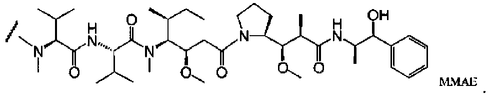

In some embodiments, the drug payload is MMAF. "MMAF" refers to monomethyl auristatin F, wherein the wavy lines indicate that it is covalently connected to the linker (L) of the antibody-drug conjugate (US2005/0238649):

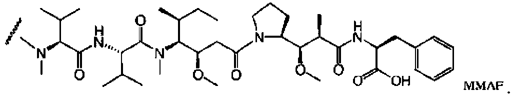

In some embodiments, the linker-partner molecule is vcMMAE. The drug linker part and conjugation method for vcMMAE are published in WO2004010957, US7659241, US7829531, US7851437, and US 11/833,028 (SeattleGenetics, Inc.), which are incorporated herein by reference. Using a method similar to that disclosed in the foregoing literatures, the drug linker of vcMMAE is conjugated with the antibody according to the present invention.

For further discussion of types of cytotoxins, linkers and methods for conjugating therapeutic agents to antibodies, see further Saito, G. et al. (2003) Adv. Drug Deliv. Rev. 55:199-215; Trail, PA et al. (2003) Cancer. Immunol. Immunother. 52:328-337; Payne, G. (2003) Cancer Cell 3: 207-212; Allen, T. M. (2002) Nat. Rev. Cancer 2: 750-763; Pastan, I. and Kreitman, R. J. (2002) Curr. Opin. Investig. Drugs 3: 1089-1091; Senter, P. D. and Springer, C. J. (2001) Adv. Drug Deliv. Rev. 53: 247-264, all of which are incorporated herein by reference.

In some embodiments, the drug payload may radioisotope, resulting in a cytotoxic radioactive drug (also known as an antibody-radioisotope conjugate). Examples of radioisotopes that kill tumor cells include, but are not limited to, iodine-131, indium-111, yttrium-90, and lutetium-177. Methods for preparing the antibody-radioisotope conjugate are known in the art.

In some embodiments, the partner molecule according to the present invention is a marker molecule. Optionally, the marker molecule is selected from one of: a radioactive label, a fluorescent label, and an enzyme that can detect a modification. In some embodiments, the radioactive label is radioisotope, such as iodine-131, indium-111, yttrium-90, and lutetium-177. Experiments have shown that the ITGA2 protein may be used as an effective target for the antibody-drug conjugate. The antibody-drug conjugate obtained by conjugating the antibody to micromolecule toxins can produce excellent killing effect on tumors expressing the ITGA2 protein.

### V. Pharmaceutical composition, Therapeutic Use and Method

The present invention provides a pharmaceutical composition comprising a therapeutic effective amount of at least one monoclonal antibody or antigen-binding fragment thereof according to the present invention described above or at least one antibody-drug conjugate according to the present invention described above, a therapeutic use and a therapeutic method of the pharmaceutical composition. In the antibody-drug conjugate according to the present invention, the antibody according to the present invention is conjugated with a drug payload.

In one aspect, the present invention provides a pharmaceutical composition comprising a therapeutic effective amount of at least one monoclonal antibody or antigen-binding fragment thereof according to the present invention described above or at least one antibody-drug conjugate according to the present invention described above. In some embodiments, the pharmaceutical composition according to the present invention may further include a pharmaceutically acceptable carrier.

A "pharmaceutical amount" of at least one monoclonal antibody or antigen-binding fragment thereof according to the present invention described above or at least one antibody-drug conjugate according to the present invention described above preferably results in decreased severity of the symptoms of a disease, an increased frequency and duration of the asymptomatic phase of a disease, or prevention of damage or disability caused by the pain of a disease. For example, for treating a tumor, a "therapeutic effective amount" of at least one monoclonal antibody or antigen-binding fragment thereof according to the present invention described above or at least one antibody-drug conjugate according to the present invention described above preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, or more preferably at least about 80%, compared to a subject not receiving treatment. The ability to inhibit tumor growth may be evaluated in an animal model system that predicts the therapeutic efficacy against a human tumor. Alternatively, it may also be evaluated by examining the ability to inhibit cell growth, which may be determined in vitro by tests well known to those skilled in the art. An effective amount of the antibody or antigen-binding fragment thereof according to the invention is capable of reducing the size of a tumor, or otherwise alleviating a subject's symptoms, such as prevention and/or treatment of metastasis or relapse. One skilled in the art may determine such an amount based on factors such as the size of the subject, the severity of the subject's symptoms, and the selected particular composition or route of administration.

In another aspect, the present invention provides use of at least one monoclonal antibody or antigen-binding fragment thereof according to the present invention described above or at least one antibody-drug conjugate according to the present invention described above in preparing a pharmaceutical composition. In some embodiments, the pharmaceutical composition is used for treating a malignant tumor, preventing and/or treating metastasis or relapse of a malignant tumor in a patient.

In another aspect, the present invention provides a pharmaceutical composition comprising at least one monoclonal antibody or the antigen-binding fragment thereof according to the present invention described above or the antibody-drug conjugate according to the present invention described above, wherein the pharmaceutical composition is used for a method of treating a malignant tumor, or preventing and/or treating metastasis or relapse of a malignant tumor in a patient.

In another aspect, the present invention provides a method for treating a malignant tumor, preventing and/or treating metastasis or relapse of a malignant tumor in a patient, wherein the method comprises administering to the patient an effective amount of at least one monoclonal antibody or the antigen-binding fragment thereof according to the present invention described above, at least one antibody-drug conjugate according to the present invention described above, and/or at least one pharmaceutical composition according to the present invention described above.

In some embodiments, the malignant tumor is a tumor expressing the ITGA2 protein. The malignant tumor is selected from a group consisting of the following: breast cancer, colorectal cancer, large intestine cancer, skin cancer, pancreatic cancer, prostate cancer, liver cancer, lung cancer, gastric cancer, leukemia, bile duct cancer, cervical cancer, endometrial cancer, esophageal cancer, head and neck cancer, lymphoma, medullary thyroid cancer, Non-Hodgkin's lymphoma, ovarian cancer, glioma, melanoma, and bladder cancer. In some embodiments, the malignant tumor is selected from a group consisting of the following: colorectal cancer, bile duct cancer, pancreatic cancer, and skin cancer. In some embodiments, the malignant tumor is colorectal cancer. In some embodiments, the malignant tumor is bile duct cancer. In some embodiments, the malignant tumor is pancreatic cancer. In some embodiments, the malignant tumor is skin cancer.

### VI. Diagnostic/Prognostic Composition, Diagnostic/Prognostic Use and Method

As described herein, ITGA2 is a tumor-associated antigen that is specifically overexpressed by tumor cells, especially in patients with cancers such as colorectal cancer, bile duct cancer, pancreatic cancer, and skin cancer, and is expressed on the surface of tumor cells.

The present invention provides a diagnostic/prognostic composition comprising at least one monoclonal antibody or antigen-binding fragment thereof according to the present invention described above or at least one antibody-drug conjugate according to the present invention described above, a diagnostic/prognostic use and a diagnostic/prognostic method of the composition. In the diagnostic/prognostic drug conjugate according to the present invention, the antibody according to the present invention is conjugated with a marker molecule. For the diagnostic/prognostic composition, the diagnostic/prognostic use and the diagnostic/prognostic method, the antibody-drug conjugate according to the present invention described above refers to a conjugate formed by connecting the antibody of the present invention or the antigen-binding fragment thereof to a marker molecule.

In one aspect, the present invention provides use of at least one monoclonal antibody or antigen-binding fragment thereof according to the present invention described above or at least one antibody-drug conjugate according to the present invention described above in preparing a diagnostic/prognostic composition. In some embodiments, the diagnostic composition is used for detecting presence of ITGA2a malignant tumor in a patient. In some embodiments, the prognostic composition is used for prognosticating relapse or progression of a malignant tumor in a patient.

In another aspect, the present invention provides a method for detecting/diagnosing presence of a malignant tumor in a patient, wherein the method comprises administering to the patient an effective amount of at least one monoclonal antibody or antigen-binding fragment thereof according to the present invention described above, at least one antibody-drug conjugate according to the present invention described above, and/or at least one diagnostic/prognostic composition according to the present invention described above.

In some embodiments, the present invention provides a method for detecting/diagnosing presence of a malignant tumor in a patient, comprising:
a) contacting a biological sample obtained from the patient with at least one monoclonal antibody or antigen-binding fragment thereof according to the present invention described above, at least one antibody-drug conjugate according to the present invention described above, and/or at least one diagnostic/prognostic composition according to the present invention described above;
b) detecting binding of at least one monoclonal antibody or antigen-binding fragment thereof according to the present invention described above, at least one antibody-drug conjugate according to the present invention described above, and/or at least one diagnostic/prognostic composition according to the present invention described above with a target antigen in the biological sample, wherein the detected binding of the antibody to the target antigen indicates presence of the malignant tumor in the patient.

In another aspect, the present invention provides a method for prognosticating relapse or progression of a malignant tumor in a patient, wherein the method comprises administering to the patient an effective amount of at least one monoclonal antibody or antigen-binding fragment thereof according to the present invention described above, at least one antibody-drug conjugate according to the present invention described above, and/or at least one diagnostic/prognostic composition according to the present invention described above.

In some embodiments, the invention provides a method for prognosticating relapse or progression of a malignant tumor in a patient, wherein the method comprises:
(a) isolating a biological sample comprising tumor cells from the patient;
(b) contacting the biological sample comprising tumor cells with at least one monoclonal antibody or antigen-binding fragment thereof according to the present invention described above, at least one antibody-drug conjugate according to the present invention described above, and/or at least one diagnostic/prognostic composition according to the present invention described above; and
(c) identifying the binding of the antibody to the tumor cells, which represents the presence of tumor cells,
thereby prognosticating the relapse or progression of the malignant tumor in the patient.

In some embodiments, the progression of the malignant tumor includes metastasis of the malignant tumor in the patient.

In some embodiments, the detected binding of tumor cells to at least one monoclonal antibody or antigen-binding fragment thereof according to the present invention described above, at least one antibody-drug conjugate according to the present invention described above, and/or at least one diagnostic/prognostic composition according to the present invention described above indicates high risk of relapse or progression of the malignant tumor in the patient.

In some embodiments, the partner molecule according to the present invention is a marker molecule. Optionally, the marker molecule is selected from one of: a radioactive label (for example, an isotope), a fluorescent label (for example, a fluorescent dye), a chemical substance, and an enzyme or label that can detect a modification.

In some embodiments, the biological sample is selected from one of: a blood sample, a tissue sample, and a lymph sample.

Methods for detecting binding of an antibody to an antigen are known in the art, such as ELISA and the like.

### VII. Kit

The scope of the present invention further comprises a kit for the method according to the present invention, wherein the kit includes at least one monoclonal antibody or antigen-binding fragment thereof according to the present invention described above, at least one antibody-drug conjugate according to the present invention described above, at least one pharmaceutical composition according to the present invention described above, or a diagnostic/prognostic composition according to the present invention described above, and a direction for use.

In some embodiments, the kit may further include at least one additional detection reagent used to detect presence of at least one monoclonal antibody or antigen-binding fragment thereof according to the present invention described above, at least one antibody-drug conjugate according to the present invention described above, at least one pharmaceutical composition according to the present invention described above, or a diagnostic/prognostic composition according to the present invention described above. The kit generally includes a label indicating the intended use and/or method of use of the contents of the kit. The term "label" includes any written or recorded material provided on or with the kit or otherwise provided with the kit.

### Examples

The present invention may be further understood by reference to the specific examples set forth herein, which are only intended to illustrate the invention, and are not intended to limit the scope of the invention. It is apparent that various modifications and variations may be made to the present invention without departing from the spirit of the invention, and such modifications and variations are therefore also within the patentable scope of the present invention.

### Example 1 - Specific overexpression of the ITGA2 protein on the surface of a variety of cancer cells

HuCC-T1 (human bile duct cancer cells; MeisenCTCC: CTCC-003-0103), HCCC-9810 (human bile duct cancer cells; Procell Life Science&Technology Co.,Ltd. (Wuhan): CL-0095), AsPC-1 (human metastatic pancreatic cancer cells; Procell Life Science&Technology Co.,Ltd. (Wuhan): CL-0027), BxPC-3 (human orthotopic pancreatic adenocarcinoma cells; MeisenCTCC: CTCC-001-0360), A431 (human epidermal cancer cells; Procell Life Science&Technology Co.,Ltd. (Wuhan): CL-0015), HT-29 (human colorectal adenocarcinoma cell line; Procell Life Science&Technology Co.,Ltd. (Wuhan): CL-0176) and CHO (hamster ovary cells; Procell Life Science&Technology Co.,Ltd. (Wuhan): CL-0061) cells were seeded into a deep 96-well plate. The cells were seeded into each well at 50,000/100 µL, and each cell was seeded in three wells. It was then blown evenly with PBS and washed twice. Each well was added with a PBS solution comprising 1% FBS per at an amount of 100 µL/well and the plate was then incubated in a 4°C freezer for 30 min. A FITC-labeled mouse anti-human ITGA2 antibody solution (BD Pharmingen:555498) was diluted to a working concentration of 20 µL/well, and incubated in a 4°C freezer for 30 min in the dark. After incubation, cells were washed twice with 500 ul of 1% FBS and then added with 250 ul of PBS and suspended again. The blank reference solution was a 250 µL PBS solution comprising the reference control solution. Flow cytometry was used to collect and analyze the number of ITGA2 protein copies of each cell line, in the molecules of equivalent soluble fluorochrome (MESF). The results are shown in the table 4 below.

**Table 4**

| **Cell Line** | **ITGA2 Protein Copy Number (MESF)** |
|---|---|
| Human bile duct cancer cells HuCCT1 | 2,013,058 |
| Human orthotopic pancreatic adenocarcinoma cells BxPC-3 | 1,633,773 |
| Human bile duct cancer cells HCCC-9810 | 961,428 |
| Human metastatic pancreatic cancer cells ASPC-1 | 746,355 |
| Human epidermal carcinoma cells A431 | 721,588 |
| Human colorectal adenocarcinoma cells HT-29 | 151,268 |
| CHO | 959 |

As shown in Table 4, ITGA2 protein had higher expression (MESF>150,000) on the surface of HuCCT1, BxPC-3, HCCC-9810, ASPC-1, and A431 cells, and almost no expression on the surface of the negative control cell CHO (MESF<1000, which can be regarded as the background value at the experimental technical level).

### Example 2 - Preparation of ITGA2 Antibody

### 1. Constructing a Vector

The design, vector construction, expression, purification and identification of the ITGA2 antibody sequence of the present invention were entrusted to Sanyou Biopharmaceuticals Co., Ltd. The construction process mainly includes constructing the sequence of the ITGA2 antibody on a pcDNA3.4 vector, and obtaining a plasmid through PCR, enzyme digestion, ligation, transformation, identification, sequencing, alignment, and extraction.

The vector comprising the light chain and heavy chain sequence of the ITGA2 antibody according to the present invention is shown in Table 5 below:

| **Antibody** | **Vector Comprising Light Chain** | **Vector Comprising Heavy Chain** |
|---|---|---|
| Chimeric mouse antibody FS002-A103 | pcDNA3.4-FS002-A103-LC(Kappa) | pcDNA3.4-FS002-A103-HC(IgG1) |
| Humanized antibody FS002-A103-H4L2 | pcDNA3.4-FS002-A103-L2-LC(Kappa) | pcDNA3.4-F5002-A103-H4-HC(IgG1) |
| Humanized antibody FS002-A103-H4L3 | pcDNA3.4-FS002-A103-L3-LC(Kappa) | pcDNA3.4-FS002-A103-H4-HC(IgG1) |
| Humanized antibody FS002-A103-H4L4 | pcDNA3.4-FS002-A103-L4-LC(Kappa) | pcDNA3.4-FS002-A103-H4-HC(IgG1) |
| Chimeric mouse antibody FS002-A153 | pcDNA3.4-FS002-A153-LC(Kappa) | pcDNA3.4-FS002-A153-HC(IgG1) |
| Humanized antibody FS002-A153-H3L3 | pcDNA3.4-FS002-A153-L3-LC(Kappa) | pcDNA3.4-FS002-A153-H3-HC(IgG1) |
| Humanized antibody FS002-A153-H4L2 | pcDNA3.4-FS002-A153-L2-LC(Kappa) | pcDNA3.4-FS002-A153-H4-HC(IgG1) |
| Humanized antibody FS002-A153-H5L1 | pcDNA3.4-FS002-A153-L1-LC(Kappa) | pcDNA3.4-FS002-A153-H5-HC(IgG1) |
| Humanized antibody FS002-A153-H5L2 | pcDNA3.4-FS002-A153-L2-LC(Kappa) | pcDNA3.4-FS002-A153-H5-HC(IgG1) |
| Humanized antibody FS002-A153-H5L4 | pcDNA3.4-FS002-A153-L4-LC(Kappa) | pcDNA3.4-FS002-A153-H5-HC(IgG1) |
| Humanized antibody FS002-A153-H5L5 | pcDNA3.4-FS002-A153-L5-LC(Kappa) | pcDNA3.4-FS002-A153-H5-HC(IgG1) |
| Chimeric mouse antibody FS002-A208 | pcDNA3.4-FS002-A208-LC(Kappa) | pcDNA3.4-FS002-A208-HC(IgG1) |
| Humanized antibody FS002-A208-H3L4 | pcDNA3.4-FS002-A208-L4-LC(Kappa) | pcDNA3.4-FS002-A208-H3-HC(IgG1) |
| Humanized antibody FS002-A208-H4L4 | pcDNA3.4-FS002-A208-L4-LC(Kappa) | pcDNA3.4-FS002-A208-H4-HC(IgG1) |
| Humanized antibody FS002-A208-H5L4 | pcDNA3.4-FS002-A208-L4-LC(Kappa) | pcDNA3.4-FS002-A208-H5-HC(IgG1) |
| Chimeric mouse antibody FS002-A226 | pcDNA3.4-FS002-A226-LC(Kappa) | pcDNA3.4-FS002-A226-HC(IgG1) |
| Humanized antibody FS002-A226-H3L4 | pcDNA3.4-FS002-A226-L4-LC(Kappa) | pcDNA3.4-FS002-A226-H3-HC(IgG1) |
| Humanized antibody FS002-A226-H4L3 | pcDNA3.4-FS002-A226-L3-LC(Kappa) | pcDNA3.4-FS002-A226-H4-HC(IgG1) |
| Humanized antibody FS002-A226-H5L3 | pcDNA3.4-FS002-A226-L3-LC(Kappa) | pcDNA3.4-FS002-A226-H5-HC(IgG1) |
| Humanized antibody FS002-A226-H5L4 | pcDNA3.4-FS002-A226-L4-LC(Kappa) | pcDNA3.4-FS002-A226-H5-HC(IgG1) |

The coding sequences for the light and heavy chains in each plasmid vector in the table above are set forth in SEQ ID NO: 1-40 of the table 1 (the shaded parts correspond to the constant region sequences). The amino acid sequences of light and heavy chain variable regions, constant regions, and light and heavy chains of the antibodies expressed from the above plasmid vectors are set forth in SEQ ID NO:41-242 of the table 2-4.

Table 6: Summary of the sequence numberings of the heavy chains, light chains, constant regions, variable regions and CDRs of the above antibodies of the present invention (in the following tables, the number values on the columns 3-7 and 9-13 refer to numbers of SEQ ID NO:, for example, number "1" refers to SEQ ID NO:1).

**Table 6a - Chimeric mouse antibody FS002-A103 and the corresponding humanized antibody thereof**

| **Antibody** | **Light Chain** | **Light Chain Coding Sequence** | **Light Chain Amino Acid Sequence** | **VL CDR 1-3** | **Light Chain Constant Region** | **Light Chain Variable Region** | **Heavy Chain** | **Heavy Chain Coding Sequence** | **Heavy Chain Amino Acid Sequence** | **VH CDR 1-3** | **Heavy Chain Constant Region** | **Heavy Chain Variable Region** |
|---|---|---|---|---|---|---|---|---|---|---|---|---|
| FS002-A103 | FS002-A103-LC(Kappa) | 1 | 203 | 42-44 | 201 | 41 | FS002-A103-HC(IgG1) | 2 | 204 | 46-48 | 202 | 45 |
| FS002-A103-H4L2 | FS002-A103-L2-LC(Kappa) | 3 | 205 | 50-52 | 201 | 49 | FS002-A103-H4-HC(IgG1) | 4 | 206 | 54-56 | 202 | 53 |
| FS002-A103-H4L3 | FS002-A103-L3-LC(Kappa) | 5 | 207 | 58-60 | 201 | 57 | FS002-A103-H4-HC(IgG1) | 6 | 208 | 62-64 | 202 | 61 |
| FS002-A103-H4L4 | FS002-A103-L4-LC(Kappa) | 7 | 209 | 66-68 | 201 | 65 | FS002-A103-H4-HC(IgG1) | 8 | 210 | 70-72 | 202 | 69 |

**Table 6b - Chimeric mouse antibody FS002-A153 and the corresponding humanized antibody thereof**

| **Antibody** | **Light Chain** | **Light Chain Coding Sequence** | **Light Chain Amino Acid Sequence** | **VL CDR 1-3** | **Light Chain Constant Region** | **Light Chain Variable Region** | **Heavy Chain** | **Heavy Chain Coding Sequence** | **Heavy Chain Amino Acid Sequence** | **VH CDR 1-3** | **Heavy Chain Constant Region** | **Heavy Chain Variable Region** |
|---|---|---|---|---|---|---|---|---|---|---|---|---|
| FS002-A153 | FS002-A153-LC(Kappa) | 9 | 211 | 74-76 | 201 | 73 | FS002-A153-HC(IgG1) | 10 | 212 | 78-80 | 202 | 77 |
| FS002-A153-H3L3 | FS002-A153-13-LC(Kappa) | 11 | 213 | 82-84 | 201 | 81 | FS002-A153-H3-HC(IgG1) | 12 | 214 | 86-88 | 202 | 85 |
| FS002-A153-H4L2 | FS002-A153-L2-LC(Kappa) | 13 | 215 | 90-92 | 201 | 89 | FS002-A153-H4-HC(IgG1) | 14 | 216 | 94-96 | 202 | 93 |
| FS002-A153-H511 | FS002-A153-L1-LC(Kappa) | 15 | 217 | 98-100 | 201 | 97 | FS002-A153-H5-HC(IgG1) | 16 | 218 | 102-104 | 202 | 101 |
| FS002-A153-H51.2 | FS002-A153-1.2-LC(Kappa) | 17 | 219 | 106-108 | 201 | 105 | FS002-A153-H5-HC(IgG1) | 18 | 220 | 110-112 | 202 | 109 |
| FS002-A153-H5L4 | FS002-A153-L4-LC(Kappa) | 19 | 221 | 114-116 | 201 | 113 | FS002-A153-H5-HC(IgG1) | 20 | 222 | 118-120 | 202 | 117 |
| FS002-A153-H5L5 | FS002-A153-L5-LC(Kappa) | 21 | 223 | 122-124 | 201 | 121 | FS002-A153-H5-HC(IgG1) | 22 | 224 | 126-128 | 202 | 125 |

**Table 6c - Chimeric mouse antibody FS002-A208 and the corresponding humanized antibody thereof**

| **Antibody** | **Light Chain** | **Light Chain Coding Sequence** | **Light Chain Amino Acid Sequence** | **VL CDR 1-3** | **Light Chain Constant Region** | **Light Chain Variable Region** | **Heavy Chain** | **Heavy Chain Coding Sequence** | **Heavy Chain Amino Acid Sequence** | **VH CDR 1-3** | **Heavy Chain Constant Region** | **Heavy Chain Variable Region** |
|---|---|---|---|---|---|---|---|---|---|---|---|---|
| FS002-A208 | FS002-A208-LC(Kappa) | 23 | 225 | 130-132 | 201 | 129 | FS002-A208-HC(IgG1) | 24 | 226 | 134-136 | 202 | 133 |
| FS002-A208-H3L4 | FS002-A208-L4-LC(Kappa) | 25 | 227 | 138-140 | 201 | 137 | FS002-A208-H3-HC(IgG1) | 26 | 228 | 142-144 | 202 | 141 |
| FS002-A208-H4L4 | FS002-A208-L4-LC(Kappa) | 27 | 229 | 146-148 | 201 | 145 | FS002-A208-H4-HC(IgG1) | 28 | 230 | 150-152 | 202 | 149 |
| FS002-A208-H5L4 | FS002-A208-L4-LC(Kappa) | 29 | 231 | 154-156 | 201 | 153 | FS002-A208-H5-HC(IgG1) | 30 | 232 | 158-160 | 202 | 157 |

**Table 6d - Chimeric mouse antibody FS002-A226 and the corresponding humanized antibody thereof**

| **Antibody** | **Light Chain** | **Light Chain Coding Sequence** | **Light Chain Amino Acid Sequence** | **VL CDR 1-3** | **Light Chain Constant Region** | **Light Chain Variable Region** | **Heavy Chain** | **Heavy Chain Coding Sequence** | **Heavy Chain Amino Acid Sequence** | **VH CDR 1-3** | **Heavy Chain Constant Region** | **Heavy Chain Variable Region** |
|---|---|---|---|---|---|---|---|---|---|---|---|---|
| FS002-A226 | FS002-A226-LC(Kappa) | 31 | 233 | 162-164 | 201 | 161 | FS002-A226-HC(IgG1) | 32 | 234 | 166-168 | 202 | 165 |
| FS002-A226-H3L4 | FS002-A226-L4-LC(Kappa) | 33 | 235 | 170-172 | 201 | 169 | FS002-A226-H3-HC(IgG1) | 34 | 236 | 174-176 | 202 | 173 |
| FS002-A226-H4L3 | FS002-A226-L3-LC(Kappa) | 35 | 237 | 178-180 | 201 | 177 | FS002-A226-H4-HC(IgG1) | 36 | 238 | 182-184 | 202 | 181 |
| FS002-A226-H5L3 | FS002-A226-L3-LC(Kappa) | 37 | 239 | 186-188 | 201 | 185 | FS002-A226-H5-HC(IgG1) | 38 | 240 | 190-192 | 202 | 189 |
| FS002-A226-H5L4 | FS002-A226-L4-LC(Kappa) | 39 | 241 | 194-196 | 201 | 193 | FS002-A226-H5-HC(IgG1) | 40 | 242 | 198-200 | 202 | 197 |

### 2. Antibody expression and purification, and SDS-PAGE identification

The above plasmids were expressed in CHO cells for 7 days as required using the ExpiCHO-s expression system, and the antibody was obtained by purifying with Protein A affinity chromatography on the 6th day of expression.

Subsequently, the purity of the resulting antibody was measured by SDS-PAGE. In SDS-PAGE, the purity of the reduced band or the sum of the reduced heavy chain plus light chain was calculated with ImageJ through peak area normalization, wherein the non-reduced band of the reference IPI had a molecular weight of about 150 kDa, and a purity of greater than 90%, and the reduced heavy chain had a molecular weight of about 55 kDa, the light chain had a molecular weight of about 25 kDa, and the heavy chain plus light chain had a purity of greater than 90%.

### Example 3 - Evaluating the affinity of the anti-ITGA2 antibody to the ITGA2 protein at the protein level by ELISA method

The affinity of the ITGA2 antibody to the ITGA2 protein at the protein level was determined using the following method, comprising coating the ELISA plate with 2 ug/mL ITGA2-His. The plate was washed 3 times with PBST and blocked with 5% PBS-Milk for 2 h at room temperature. The plate was washed 3 times with PBST, then the ELISA plate was added with the diluted antibody at 30 µL per well, and left standing for 60 min at room temperature. The plate was washed 3 times with PBST, then the ELISA plate was added with anti-human IgG-Fc-HRP (abcam; ab97225) and left standing for 60 min at room temperature It was added with TMB. The reaction was stopped with 2 M stop solution, and the OD450 was read. Finally, EC50 was used to compare the binding capacity of each antibody to ITGA2-his. The antibody Vatelizumab (purchased from Biointron) was used as a positive control. The results are shown in Table 7 below.

**Table 7**

| Anti-ITGA2 antibody | EC50 of ITGA2 Determined by ELISA (nM) |
|---|---|
| FS002-A103 | 0.017 |
| FS002-A153 | 0.008 |
| FS002-A208 | 0.020 |
| FS002-A226 | 0.018 |
| FS002-A103-H4L2 | 0.006 |
| FS002-A103-H4L3 | 0.006 |
| FS002-A103-H4L4 | 0.010 |
| FS002-A153-H3L3 | 0.014 |
| FS002-A153-H4L2 | 0.011 |
| FS002-A153-H5L1 | 0.027 |
| FS002-A153-H5L2 | 0.009 |
| FS002-A153-H5L4 | 0.024 |
| FS002-A153-H5L5 | 0.022 |
| FS002-A208-H3L4 | 0.022 |
| FS002-A208-H4L4 | 0.017 |
| FS002-A208-H5L4 | 0.018 |
| FS002-A226-H3L4 | 0.023 |
| FS002-A226-H4L3 | 0.024 |
| FS002-A226-H5L3 | 0.023 |
| FS002-A226-H5L4 | 0.022 |
| hIgG1 | N/A |
| Vatelizumab | 0.008 |

As shown in the table above, 20 tested antibodies have good binding activity to ITGA2-His (EC50<1 nM).

### Example 4 - Determining affinity of anti-ITGA2 antibody to ITGA2 at the cellular level by flow cytometry

Human bile duct cancer HuCCT1 cells were seeded into a 96-well plate at 50,000 cells per well, and blown evenly and washed twice with PBS. The PBS was then drawn and discarded. The ITGA2 antibody to be tested was used as a primary antibody, and was prepared to a working concentration of 30 nM with 1% FBS/PBS solution. It was diluted by 3 folds in sequence, with a total of 5 specific concentration points. The prepared antibody dilution was added to the corresponding 96-well plate and incubated at 4°C for 30 min. It was then blown and washed twice with 1% FBS/PBS solution. After the PBS was drawn and discarded, the solution was centrifuged, and the antibody supernatant was drawn and discarded. A goat anti-human secondary antibody IgG (H+L) with cross-recognition ability from other species removed was prepared with a PBS solution comprising 10% FBS at 1:300. It was then added to a 96-well plate, and incubated at 4 °C for 30 min. Flow cytometry was used for collection and analysis, and finally the affinity of the antibody to the ITGA2 target was evaluated by EC50. The antibody Vatelizumab (purchased from Biointron) was used as a positive control antibody for binding to the ITGA2.

The results of the determination of EC50 are shown in Table 8. As shown in Table 8, 20 antibodies have EC50 of less than 1 nM and bind well to ITGA2-positive HuCCT1 cells.

**Table 8**

| Anti-ITGA2 antibody | EC50 of HuCCT1 Determined by Flow Cytometry (nM) |
|---|---|
| FS002-A103 | 0.633 |
| FS002-A153 | 0.333 |
| FS002-A208 | 0.760 |
| FS002-A226 | 0.580 |
| FS002-A103-H4L2 | 0.127 |
| FS002-A103-H4L3 | 0.080 |
| FS002-A103-H4L4 | 0.300 |
| FS002-A153-H3L3 | 0.420 |
| FS002-A153-H4L2 | 0.313 |
| FS002-A153-H5L1 | 0.580 |
| FS002-A153-H5L2 | 0.220 |
| FS002-A153-H5L4 | 0.487 |
| FS002-A153-H5L5 | 0.480 |
| FS002-A208-H3L4 | 0.587 |
| FS002-A208-H4L4 | 0.600 |
| FS002-A208-H5L4 | 0.627 |
| FS002-A226-H3L4 | 0.280 |
| FS002-A226-H4L3 | 0.533 |
| FS002-A226-H5L3 | 0.580 |
| FS002-A226-H5L4 | 0.647 |
| hIgG1 | N/A |
| Vatelizumab | 0.780 |

### Example 5 - Preparing an antibody-drug conjugate (ADC)

The antibody-drug conjugate was prepared by the following method.

### Materials and Methods:

Conjugating buffer: 25 mM Na₂B₄O₇, 25 mM NaCl, 1 mM DPTA, pH 7.4
Dialysis buffer: 1 × PBS, pH 7.4
Antibody reduction concentration: 5 mg/mL
Tris(2-chloroethyl)phosphate (TCEP): 5 mM, binding buffer
TCEP:antibody = 2.3:1
Payload: 10 mM linker-payload, dimethyl sulfoxide (DMSO)
DMSO in final reaction: 10%
Payload:antibody =10:1
Reduction temperature and time: 25°C and 2 hours
Conjugating temperature and time: 25°C and 4 hours

### Conjugating Steps:

Thaw and dialyze the antibody using a conjugation buffer at 4 °C for > 4 h;
If needed, concentrate the antibody solution to 2-5 mg/ml;
Add a TCEP working solution to reduce the antibody and incubate it in a water bath at 25°C;
Prepare a linker-payload solution in DMSO (10 mM);
Calculate the volume of the effective linker-payload solution for each conjugation based on molar concentration;
Mix the antibody with the linker-payload at the desired molar ratio and incubate it in a 25 °C water bath;
Dialyze the conjugate for more than 4 h at 4 °C using an ADC storage buffer, with the buffer changed at least every 4 h; and
Extract and filter the ADC using a 0.22 µm filter, and send the samples for SEC-HPLC and HIC-HPLC analysis.

Through the above method, the antibody-drug conjugates of the 12 antibodies of the present invention and the positive control antibody Vatelizumab were prepared in the present example using vc-MMAE (mc-vc-PAB-MMAE, C68H105N11O15, provided by ChemPartner) as the linker-payload.

**Table 9**

| ADC Name | Drug purity (%, SEC) | Drug-antibody ratio (DAR) |
|---|---|---|
| FS002-A103-H4L2-vcMMAE | 99.6 | 3.5 |
| FS002-A103-H4L3-vcMMAE | 99.4 | 3.5 |
| FS002-A103-H4L4-vcMMAE | 99.7 | 3.7 |
| FS002-A153-H3L3-vcMMAE | 99.2 | 3.8 |
| FS002-A153-H4L2-vcMMAE | 98.6 | 3.8 |
| FS002-A153-H5L1-vcMMAE | 99.0 | 3.7 |
| FS002-A153-H5L2-vcMMAE | 95.1 | 3.5 |
| FS002-A153-H5L5-vcMMAE | 99.6 | 4.0 |
| FS002-A208-H4L4-vcMMAE | 99.4 | 4.1 |
| FS002-A208-H5L4-vcMMAE | 99.7 | 4.0 |
| FS002-A226-H3L4-vcMMAE | 99.1 | 3.8 |
| FS002-A226-H5L4-vcMMAE | 99.1 | 3.8 |
| Vatelizumab-vc-MMAE | 98.9 | 3.9 |

### Example 6 - Biological activity of antibody-drug conjugate (ADC) for killing cancer cells

The example tests the biological activity of the ADC prepared from the antibody-conjugated toxin of the present invention for killing cancer cells.

Human epidermal cancer cells A431 available from Procell Life Science&Technology Co.,Ltd. (Wuhan) were seeded into a 96-well plate at 2500 cells/well. It was then incubated in a 5% CO2 incubator overnight at 37°C. Half of the medium was removed from the cell plate on the next day. A 2x hIgG1-vcMMAE (negative control ADC, provided by ChemPartner), a control antibody and the ADC prepared as the above were prepared with a complete culture medium, and then diluted at a 3-fold gradient with 11 specific concentration points in total. Serial dilutions of the above molecules were added to the appropriate wells, and it was then incubated in a 5% CO2 incubator for 5 days at 37°C. After the incubation, 100 uL of Cell-Titer-Glo 2.0 reagent was added to the 96-well plate. It was then well mixed on a plate shaker for 2 minutes, and finally equilibrated at room temperature for 10 minutes. Readings were taken using a microplate reader. Finally, IC50 was used to compare the biological activity of the ADC against each cell line.

The results are shown in Table 10. As shown in Table 10, the ADCs prepared from the 12 antibodies of the present invention and the positive control Vatelizumab-ve-MMAE had good killing effect on ITGA2-positive cells A431 (IC50<0.1nM). The ITGA2 antibody Vatelizumab not coupled to vc-MMAE and the negative control hIgG1-vc-MMAE cannot effectively kill the A431 cells.

**Table 10**

| ADC Name | A431 cell killing IC50 (nM) |
|---|---|
| Vatelizumab | N/A |
| hIgG1-vc-MMAE | N/A |
| FS002-A103-H4L2-vcMMAE | 0.061 |
| FS002-A103-H4L3-vcMMAE | 0.070 |
| FS002-A103-H4L4-vcMMAE | 0.052 |
| FS002-A153-H3L3-vcMMAE | 0.088 |
| FS002-A153-H4L2-vcMMAE | 0.057 |
| FS002-A153-H5L1-vcMMAE | 0.082 |
| FS002-A 153- H5L2-vcMMAE | 0.028 |
| FS002-A 153- H5L5-vcMMAE | 0.073 |
| FS002-A208-H4L4-vcMMAE | 0.051 |
| FS002-A208-H5L4-vcMMAE | 0.083 |
| FS002-A226-H3L4-vcMMAE | 0.063 |
| FS002-A226-H5L4-vcMMAE | 0.049 |
| Vatelizumab-vc-MMAE | 0.054 |

These results suggest that ITGA2 antibody cannot be used alone to kill tumor cells in vitro, but surprisingly, the ITGA2 protein has been found to be an effective target for the antibody-drug conjugate. The antibody-drug conjugate obtained by conjugating the antibody to micromolecule toxins can produce excellent killing effects on tumors expressing the ITGA2 protein.

### Sets of clauses

### Set A of clauses

1. An isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
   1) a light chain variable region that comprises (a) LCDR1 as set forth in SEQ ID NO: 42, (b) LCDR2 as set forth in SEQ ID NO: 43, and (c) LCDR3 as set forth in SEQ ID NO: 44, and a heavy chain variable region that comprises (d) HCDR1 as set forth in SEQ ID NO: 46, (e) HCDR2 as set forth in SEQ ID NO: 47, and (f) HCDR3 as set forth in SEQ ID NO: 48;
   2) a light chain variable region that comprises (a) LCDR1 as set forth in SEQ ID NO: 50, (b) LCDR2 as set forth in SEQ ID NO: 51, and (c) LCDR3 as set forth in SEQ ID NO: 52, and a heavy chain variable region that comprises (d) HCDR1 as set forth in SEQ ID NO: 54, (e) HCDR2 as set forth in SEQ ID NO: 55, and (f) HCDR3 as set forth in SEQ ID NO:56;
   3) a light chain variable region that comprises (a) LCDR1 as set forth in SEQ ID NO: 58, (b) LCDR2 as set forth in SEQ ID NO: 59, and (c) LCDR3 as set forth in SEQ ID NO: 60, and a heavy chain variable region that comprises (d) HCDR1 as set forth in SEQ ID NO: 62, (e) HCDR2 as set forth in SEQ ID NO: 63, and (f) HCDR3 as set forth in SEQ ID NO: 54; and
   4) a light chain variable region that comprises (a) LCDR1 as set forth in SEQ ID NO: 66, (b) LCDR2 as set forth in SEQ ID NO: 67, and (c) LCDR3 as set forth in SEQ ID NO: 68, and a heavy chain variable region that comprises (d) HCDR1 as set forth in SEQ ID NO: 70, (e) HCDR2 as set forth in SEQ ID NO: 71, and (f) HCDR3 as set forth in SEQ ID NO: 72.
2. The isolated antibody or the antigen binding fragment thereof according to clause 1, wherein the isolated antibody or the antigen binding fragment thereof comprises a light chain variable region and a heavy chain variable region selected from at least one of the following groups:
   1) a light chain variable region that comprises an amino acid sequence as set forth in SEQ ID NO:41, or an amino acid sequence that is at least 85%, at least 90%, at least 95%, at least 96%, at least 97%, at least 98%, or at least 99% identical to the amino acid sequence as set forth in SEQ ID NO:41, and a heavy chain variable region that comprises an amino acid sequence as set forth in SEQ ID NO:45, or an amino acid sequence that is at least 85%, at least 90%, at least 95%, at least 96%, at least 97%, at least 98%, or at least 99% identical to the amino acid sequence as set forth in SEQ ID NO:45;
   2) a light chain variable region that comprises an amino acid sequence as set forth in SEQ ID NO:49, or an amino acid sequence that is at least 85%, at least 90%, at least 95%, at least 96%, at least 97%, at least 98%, or at least 99% identical to the amino acid sequence as set forth in SEQ ID NO:49, and a heavy chain variable region that comprises an amino acid sequence as set forth in SEQ ID NO:53, or an amino acid sequence that is at least 85%, at least 90%, at least 95%, at least 96%, at least 97%, at least 98%, or at least 99% identical to the amino acid sequence as set forth in SEQ ID NO:53;
   3) a light chain variable region that comprises an amino acid sequence as set forth in SEQ ID NO:57, or an amino acid sequence that is at least 85%, at least 90%, at least 95%, at least 96%, at least 97%, at least 98%, or at least 99% identical to the amino acid sequence as set forth in SEQ ID NO:57, and a heavy chain variable region that comprises an amino acid sequence as set forth in SEQ ID NO:61, or an amino acid sequence that is at least 85%, at least 90%, at least 95%, at least 96%, at least 97%, at least 98%, or at least 99% identical to the amino acid sequence as set forth in SEQ ID NO:61; and
   4) a light chain variable region that comprises an amino acid sequence as set forth in SEQ ID NO:65, or an amino acid sequence that is at least 85%, at least 90%, at least 95%, at least 96%, at least 97%, at least 98%, or at least 99% identical to the amino acid sequence as set forth in SEQ ID NO:65, and a heavy chain variable region that comprises an amino acid sequence as set forth in SEQ ID NO:69, or an amino acid sequence that is at least 85%, at least 90%, at least 95%, at least 96%, at least 97%, at least 98%, or at least 99% identical to the amino acid sequence as set forth in SEQ ID NO:69.
3. The isolated antibody or the antigen binding fragment thereof according to clause 1 or 2, wherein the isolated antibody or the antigen binding fragment thereof further comprises a light chain constant region and a heavy chain constant region,
   the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201,
   the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.
4. The isolated antibody or the antigen binding fragment thereof according to any one of clauses 1 to 3, wherein the isolated antibody or the antigen binding fragment thereof comprises a light chain and a heavy chain selected from at least one of the following groups:
   1) a light chain that comprises an amino acid sequence as set forth in SEQ ID NO:203, or an amino acid sequence that is at least 85%, at least 90%, at least 95%, at least 96%, at least 97%, at least 98%, or at least 99% identical to the amino acid sequence as set forth in SEQ ID NO:203, and a heavy chain that comprises an amino acid sequence as set forth in SEQ ID NO:204, or an amino acid sequence that is at least 85%, at least 90%, at least 95%, at least 96%, at least 97%, at least 98%, or at least 99% identical to the amino acid sequence as set forth in SEQ ID NO:204;
   2) a light chain that comprises an amino acid sequence as set forth in SEQ ID NO:205, or an amino acid sequence that is at least 85%, at least 90%, at least 95%, at least 96%, at least 97%, at least 98%, or at least 99% identical to the amino acid sequence as set forth in SEQ ID NO:205, and a heavy chain that comprises an amino acid sequence as set forth in SEQ ID NO:206, or an amino acid sequence that is at least 85%, at least 90%, at least 95%, at least 96%, at least 97%, at least 98%, or at least 99% identical to the amino acid sequence as set forth in SEQ ID NO:206;
   3) a light chain that comprises an amino acid sequence as set forth in SEQ ID NO:207, or an amino acid sequence that is at least 85%, at least 90%, at least 95%, at least 96%, at least 97%, at least 98%, or at least 99% identical to the amino acid sequence as set forth in SEQ ID NO:207, and a heavy chain that comprises an amino acid sequence as set forth in SEQ ID NO:208, or an amino acid sequence that is at least 85%, at least 90%, at least 95%, at least 96%, at least 97%, at least 98%, or at least 99% identical to the amino acid sequence as set forth in SEQ ID NO:208;
   4) a light chain that comprises an amino acid sequence as set forth in SEQ ID NO:209, or an amino acid sequence that is at least 85%, at least 90%, at least 95%, at least 96%, at least 97%, at least 98%, or at least 99% identical to the amino acid sequence as set forth in SEQ ID NO:209, and a heavy chain that comprises an amino acid sequence as set forth in SEQ ID NO:210, or an amino acid sequence that is at least 85%, at least 90%, at least 95%, at least 96%, at least 97%, at least 98%, or at least 99% identical to the amino acid sequence as set forth in SEQ ID NO:210.
5. The isolated antibody or the antigen binding fragment thereof according to any one of clauses 1 to 4, wherein the isolated antibody or the antigen binding fragment thereof consists of a light chain and a heavy chain selected from at least one of the following groups:
   1) a light chain that consists of an amino acid sequence as set forth in SEQ ID NO:203, and a heavy chain that consists of an amino acid sequence as set forth in SEQ ID NO:204;
   2) a light chain that consists of an amino acid sequence as set forth in SEQ ID NO:205, and a heavy chain that consists of an amino acid sequence as set forth in SEQ ID NO:206;
   3) a light chain that consists of an amino acid sequence as set forth in SEQ ID NO:207, and a heavy chain that consists of an amino acid sequence as set forth in SEQ ID NO:208;
   4) a light chain that consists of an amino acid sequence as set forth in SEQ ID NO:209, and a heavy chain that consists of an amino acid sequence as set forth in SEQ ID NO:210.
6. An isolated antibody specifically binding to an ITGA2 protein or an antigen-binding fragment thereof, wherein the isolated antibody or the antigen-binding fragment thereof comprises a light chain and a heavy chain selected from at least one of the following groups:
   1) a light chain that comprises an amino acid sequence encoded by a nucleotide sequence as set forth in SEQ ID NO:1, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:1, or consists of an amino acid sequence encoded by the nucleotide sequence as set forth in SEQ ID NO:1, and
      a heavy chain that comprises an amino acid sequence encoded by a nucleotide sequence as set forth in SEQ ID NO:2, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:2, or consists of an amino acid sequence encoded by the nucleotide sequence as set forth in SEQ ID NO:2;
   2) a light chain that comprises an amino acid sequence encoded by a nucleotide sequence as set forth in SEQ ID NO:3, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:3, or consists of an amino acid sequence encoded by the nucleotide sequence as set forth in SEQ ID NO:3, and
      a heavy chain that comprises an amino acid sequence encoded by a nucleotide sequence as set forth in SEQ ID NO:4, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:4, or consists of an amino acid sequence encoded by the nucleotide sequence as set forth in SEQ ID NO:4;
   3) a light chain that comprises an amino acid sequence encoded by a nucleotide sequence as set forth in SEQ ID NO:5, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:5, or consists of an amino acid sequence encoded by the nucleotide sequence as set forth in SEQ ID NO:5, and
      a heavy chain that comprises an amino acid sequence encoded by a nucleotide sequence as set forth in SEQ ID NO:6, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:6, or consists of an amino acid sequence encoded by the nucleotide sequence as set forth in SEQ ID NO:6; and
   4) a light chain that comprises an amino acid sequence encoded by a nucleotide sequence as set forth in SEQ ID NO:7, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:7, or consists of an amino acid sequence encoded by the nucleotide sequence as set forth in SEQ ID NO:7, and
      a heavy chain that comprises an amino acid sequence encoded by a nucleotide sequence as set forth in SEQ ID NO:8, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:8, or consists of an amino acid sequence encoded by the nucleotide sequence as set forth in SEQ ID NO:8.
7. The isolated antibody or the antigen binding fragment thereof according to any one of clauses 1 to 6,
   wherein the antibody is capable of binding to an ITGA2 protein in vitro at an EC50 of less than 1 nM; and/or
   wherein the antibody is capable of binding to an ITGA2 protein on the surface of HuCCT1 cells with an EC50 of less than 5 nM;
   and/or wherein the antibody is a monoclonal antibody, a polyclonal antibody, a chimeric antibody, or a humanized antibody.
8. An isolated nucleic acid molecule encoding the antibody or the antigen-binding fragment thereof according to any one of clauses 1 to 7; or
   the nucleic acid molecule comprises or consists of a nucleotide sequence combination selected from any one of the following nucleotide sequences: SEQ ID NO:1 and SEQ ID NO:2; SEQ ID NO:3 and SEQ ID NO:4; SEQ ID NO:5 and SEQ ID NO:6; and SEQ ID NO:7 and SEQ ID NO:8.
9. The isolated nucleic acid molecule according to clause 8, wherein the nucleic acid molecule is operably linked to an expression regulatory sequence.
10. An expression vector comprising the nucleic acid molecule according to clause 8 or 9.
11. A host cell comprising the nucleic acid molecule according to clause 8 or 9 or the expression vector according to clause 10.
12. A hybridoma cell expressing the antibody or the antigen-binding fragment thereof according to any one of clauses 1 to 7.
13. An antibody-drug conjugate of the formula:
   Ab-L-D, wherein:
      "Ab" represents at least one antibody or antigen-binding fragment thereof according to the present invention described above;
      "L" represents the linker that connects the antibody moiety to the partner molecule; and
      "D" represents the partner molecule;
      or
   Ab-(L-D)p, wherein:
      "Ab" represents at least one antibody or antigen-binding fragment thereof according to the present invention described above;
      "L" represents the linker that connects the antibody moiety to the partner molecule;
      "D" represents the partner molecule; and
      "p" represents the number of linker/partner molecules (preferably drug payloads) conjugated to the antibody or the antigen-binding fragment thereof according to the present invention described above; optionally, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.
14. The antibody-drug conjugate according to clause 13, wherein the linker is selected from one of the following: vc, mc-GGFG, AcLysvc, mc, MalPeg6, m(H20)c and m(H20)cvc; and/or the linker is cleavable.
15. The antibody-drug conjugate according to clause 13 or 14, wherein
   the partner molecule is a drug payload, wherein the drug payload is a therapeutic molecule; optionally, the drug payload is selected from: a cytotoxic agent, a cytostatic agent, an immunomodulator and a chemotherapeutic agent; optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD; or
   the partner molecule is a marker molecule; optionally, the marker molecule is selected from one of: a radioactive label (for example, an isotope), a fluorescent label (for example, a fluorescent dye), a chemical substance, and an enzyme or label that can detect a modification.
16. A pharmaceutical composition comprising the antibody or the antigen-binding fragment thereof according to any one of clauses 1-7, or the antibody-drug conjugate according to any one of clauses 13 to 15, and optionally a pharmaceutically acceptable carrier.
17. Use of the antibody or the antigen-binding fragment thereof according to any one of the clauses 1-7 or the antibody-drug conjugate according to any one of the clauses 13 to 15 in preparing a pharmaceutical composition; optionally, the pharmaceutical composition is used for treating a malignant tumor, preventing and/or treating metastasis or relapse of a malignant tumor in a patient; optionally, the pharmaceutical composition is used for detecting presence of a malignant tumor in a patient; and optionally, the pharmaceutical composition is used for prognosticating relapse or progression of a malignant tumor in a patient.
18. A method for treating a malignant tumor, preventing and/or treating metastasis or relapse of a malignant tumor in a patient, comprising administering to the patient an effective amount of the antibody or the antigen-binding fragment thereof according to any one of clauses 1 to 7, the antibody-drug conjugate according to any one of clauses 13 to 15, or the pharmaceutical composition according to clause 16.
19. The method according to clause 18, wherein the malignant tumor is selected from a group consisting of the following: breast cancer, large intestine cancer, skin cancer, pancreatic cancer, prostate cancer, liver cancer, lung cancer, gastric cancer, leukemia, bile duct cancer, cervical cancer, colorectal cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
20. The method according to clause 18 or 19, further comprising administering to the patient other anti-tumor treatments, such as administering a chemotherapeutic agent, an antibody targeting other tumor-specific antigens, or a radiation therapy.
21. A method for detecting presence of tumor cells in a biological sample, comprising:
   a) contacting the biological sample with the antibody or antigen-binding fragment thereof according to any one of clauses 1 to 7;
   b) detecting binding of the antibody or antigen-binding fragment thereof to a target antigen in the biological sample,
   wherein the detected binding represents presence of the tumor cells in the biological sample.
22. A method for detecting presence of a malignant tumor in a patient, comprising:
   a) contacting the biological sample obtained from the patient with the antibody or antigen-binding fragment thereof according to any one of clauses 1 to 7;
   b) detecting binding of the antibody or the antigen-binding fragment thereof to a target antigen in the biological sample, wherein the detected binding represents presence of the malignant tumor in the patient.
23. A method for prognosticating relapse or progression of a malignant tumor in a patient, wherein the method comprises:
   (a) isolating a biological sample comprising tumor cells from the patient;
   (b) contacting the biological sample comprising tumor cells with the antibody or antigen-binding fragment thereof according to any one of clauses 1 to 7; and
   (c) identifying presence of the tumor cells that bind to the antibody or the antigen-binding fragment thereof,
   thereby prognosticating the relapse or progression of the malignant tumor in the patient.
24. The method according to clause 23, wherein the progression of the malignant tumor includes metastasis of the malignant tumor in the patient.
25. The method according to clause 23 or 24, wherein the biological sample is selected from one of the following: a blood sample, a tissue sample, and a lymph sample.
26. The method according to any one of clauses 23 to 25, wherein the malignant tumor is selected from a group consisting of the following: breast cancer, colorectal cancer, large intestine cancer, skin cancer, pancreatic cancer, prostate cancer, liver cancer, lung cancer, gastric cancer, leukemia, bile duct cancer, cervical cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
27. A method for producing an antibody or an antigen-binding fragment thereof targeting human tumor cells that express ITGA2, wherein the method comprises:
   (i) culturing the host cell according to clause 11 in a condition suitable for expressing the nucleic acid molecule or the expression vector, and
   (ii) isolating and purifying the antibody or the antigen-binding fragment thereof expressed by the nucleic acid molecule or the expression vector.

### Set B of clauses

1. An isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
   1) a light chain variable region that comprises (a) LCDR1 as set forth in SEQ ID NO: 74, (b) LCDR2 as set forth in SEQ ID NO: 75, and (c) LCDR3 as set forth in SEQ ID NO: 76, and a heavy chain variable region that comprises (d) HCDR1 as set forth in SEQ ID NO: 78, (e) HCDR2 as set forth in SEQ ID NO: 79, and (f) HCDR3 as set forth in SEQ ID NO: 80;
   2) a light chain variable region that comprises (a) LCDR1 as set forth in SEQ ID NO: 82, (b) LCDR2 as set forth in SEQ ID NO: 83, and (c) LCDR3 as set forth in SEQ ID NO: 84, and a heavy chain variable region that comprises (d) HCDR1 as set forth in SEQ ID NO: 86, (e) HCDR2 as set forth in SEQ ID NO: 87, and (f) HCDR3 as set forth in SEQ ID NO:88;
   3) a light chain variable region that comprises (a) LCDR1 as set forth in SEQ ID NO: 90, (b) LCDR2 as set forth in SEQ ID NO: 91, and (c) LCDR3 as set forth in SEQ ID NO: 92, and a heavy chain variable region that comprises (d) HCDR1 as set forth in SEQ ID NO: 94, (e) HCDR2 as set forth in SEQ ID NO: 95, and (f) HCDR3 as set forth in SEQ ID NO: 96;
   4) a light chain variable region that comprises (a) LCDR1 as set forth in SEQ ID NO: 98, (b) LCDR2 as set forth in SEQ ID NO: 99, and (c) LCDR3 as set forth in SEQ ID NO: 100, and a heavy chain variable region that comprises (d) HCDR1 as set forth in SEQ ID NO: 102, (e) HCDR2 as set forth in SEQ ID NO: 103, and (f) HCDR3 as set forth in SEQ ID NO: 104;
   5) a light chain variable region that comprises (a) LCDR1 as set forth in SEQ ID NO: 106, (b) LCDR2 as set forth in SEQ ID NO: 107, and (c) LCDR3 as set forth in SEQ ID NO: 108, and a heavy chain variable region that comprises (d) HCDR1 as set forth in SEQ ID NO: 110, (e) HCDR2 as set forth in SEQ ID NO: 111, and (f) HCDR3 as set forth in SEQ ID NO: 112;
   6) a light chain variable region that comprises (a) LCDR1 as set forth in SEQ ID NO: 114, (b) LCDR2 as set forth in SEQ ID NO: 115, and (c) LCDR3 as set forth in SEQ ID NO: 116, and a heavy chain variable region that comprises (d) HCDR1 as set forth in SEQ ID NO: 118, (e) HCDR2 as set forth in SEQ ID NO: 119, and (f) HCDR3 as set forth in SEQ ID NO: 120; and
   7) a light chain variable region that comprises (a) LCDR1 as set forth in SEQ ID NO: 122, (b) LCDR2 as set forth in SEQ ID NO: 123, and (c) LCDR3 as set forth in SEQ ID NO: 124, and a heavy chain variable region that comprises (d) HCDR1 as set forth in SEQ ID NO: 126, (e) HCDR2 as set forth in SEQ ID NO: 127, and (f) HCDR3 as set forth in SEQ ID NO: 128.
2. The isolated antibody or the antigen binding fragment thereof according to clause 1, wherein the isolated antibody or the antigen binding fragment thereof comprises a light chain variable region and a heavy chain variable region selected from at least one of the following groups:
   1) a light chain variable region that comprises an amino acid sequence as set forth in SEQ ID NO:73, or an amino acid sequence that is at least 85%, at least 90%, at least 95%, at least 96%, at least 97%, at least 98%, or at least 99% identical to the amino acid sequence as set forth in SEQ ID NO:73, and a heavy chain variable region that comprises an amino acid sequence as set forth in SEQ ID NO:77, or an amino acid sequence that is at least 85%, at least 90%, at least 95%, at least 96%, at least 97%, at least 98%, or at least 99% identical to the amino acid sequence as set forth in SEQ ID NO:77;
   2) a light chain variable region that comprises an amino acid sequence as set forth in SEQ ID NO:81, or an amino acid sequence that is at least 85%, at least 90%, at least 95%, at least 96%, at least 97%, at least 98%, or at least 99% identical to the amino acid sequence as set forth in SEQ ID NO:81, and a heavy chain variable region that comprises an amino acid sequence as set forth in SEQ ID NO:85, or an amino acid sequence that is at least 85%, at least 90%, at least 95%, at least 96%, at least 97%, at least 98%, or at least 99% identical to the amino acid sequence as set forth in SEQ ID NO:85;
   3) a light chain variable region that comprises an amino acid sequence as set forth in SEQ ID NO:89, or an amino acid sequence that is at least 85%, at least 90%, at least 95%, at least 96%, at least 97%, at least 98%, or at least 99% identical to the amino acid sequence as set forth in SEQ ID NO:89, and a heavy chain variable region that comprises an amino acid sequence as set forth in SEQ ID NO:93, or an amino acid sequence that is at least 85%, at least 90%, at least 95%, at least 96%, at least 97%, at least 98%, or at least 99% identical to the amino acid sequence as set forth in SEQ ID NO:93;
   4) a light chain variable region that comprises an amino acid sequence as set forth in SEQ ID NO:97, or an amino acid sequence that is at least 85%, at least 90%, at least 95%, at least 96%, at least 97%, at least 98%, or at least 99% identical to the amino acid sequence as set forth in SEQ ID NO:97, and a heavy chain variable region that comprises an amino acid sequence as set forth in SEQ ID NO:101, or an amino acid sequence that is at least 85%, at least 90%, at least 95%, at least 96%, at least 97%, at least 98%, or at least 99% identical to the amino acid sequence as set forth in SEQ ID NO:101;
   5) a light chain variable region that comprises an amino acid sequence as set forth in SEQ ID NO:105, or an amino acid sequence that is at least 85%, at least 90%, at least 95%, at least 96%, at least 97%, at least 98%, or at least 99% identical to the amino acid sequence as set forth in SEQ ID NO:105, and a heavy chain variable region that comprises an amino acid sequence as set forth in SEQ ID NO:109, or an amino acid sequence that is at least 85%, at least 90%, at least 95%, at least 96%, at least 97%, at least 98%, or at least 99% identical to the amino acid sequence as set forth in SEQ ID NO:109;
   6) a light chain variable region that comprises an amino acid sequence as set forth in SEQ ID NO:113, or an amino acid sequence that is at least 85%, at least 90%, at least 95%, at least 96%, at least 97%, at least 98%, or at least 99% identical to the amino acid sequence as set forth in SEQ ID NO:113, and a heavy chain variable region that comprises an amino acid sequence as set forth in SEQ ID NO:117, or an amino acid sequence that is at least 85%, at least 90%, at least 95%, at least 96%, at least 97%, at least 98%, or at least 99% identical to the amino acid sequence as set forth in SEQ ID NO:117; and
   7) a light chain variable region that comprises an amino acid sequence as set forth in SEQ ID NO:121, or an amino acid sequence that is at least 85%, at least 90%, at least 95%, at least 96%, at least 97%, at least 98%, or at least 99% identical to the amino acid sequence as set forth in SEQ ID NO:121, and a heavy chain variable region that comprises an amino acid sequence as set forth in SEQ ID NO:125, or an amino acid sequence that is at least 85%, at least 90%, at least 95%, at least 96%, at least 97%, at least 98%, or at least 99% identical to the amino acid sequence as set forth in SEQ ID NO:125.
3. The isolated antibody or the antigen binding fragment thereof according to clause 1 or 2, wherein the isolated antibody or the antigen binding fragment thereof further comprises a light chain constant region and a heavy chain constant region,
   the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201,
   the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.
4. The isolated antibody or the antigen binding fragment thereof according to any one of clauses 1 to 3, wherein the isolated antibody or the antigen binding fragment thereof comprises a light chain and a heavy chain selected from at least one of the following groups:
   1) a light chain that comprises an amino acid sequence as set forth in SEQ ID NO:211, or an amino acid sequence that is at least 85%, at least 90%, at least 95%, at least 96%, at least 97%, at least 98%, or at least 99% identical to the amino acid sequence as set forth in SEQ ID NO:211, and a heavy chain that comprises an amino acid sequence as set forth in SEQ ID NO:212, or an amino acid sequence that is at least 85%, at least 90%, at least 95%, at least 96%, at least 97%, at least 98%, or at least 99% identical to the amino acid sequence as set forth in SEQ ID NO:212;
   2) a light chain that comprises an amino acid sequence as set forth in SEQ ID NO:213, or an amino acid sequence that is at least 85%, at least 90%, at least 95%, at least 96%, at least 97%, at least 98%, or at least 99% identical to the amino acid sequence as set forth in SEQ ID NO:213, and a heavy chain that comprises an amino acid sequence as set forth in SEQ ID NO:214, or an amino acid sequence that is at least 85%, at least 90%, at least 95%, at least 96%, at least 97%, at least 98%, or at least 99% identical to the amino acid sequence as set forth in SEQ ID NO:214;
   3) a light chain that comprises an amino acid sequence as set forth in SEQ ID NO:215, or an amino acid sequence that is at least 85%, at least 90%, at least 95%, at least 96%, at least 97%, at least 98%, or at least 99% identical to the amino acid sequence as set forth in SEQ ID NO:215, and a heavy chain that comprises an amino acid sequence as set forth in SEQ ID NO:216, or an amino acid sequence that is at least 85%, at least 90%, at least 95%, at least 96%, at least 97%, at least 98%, or at least 99% identical to the amino acid sequence as set forth in SEQ ID NO:216;
   4) a light chain that comprises an amino acid sequence as set forth in SEQ ID NO:217, or an amino acid sequence that is at least 85%, at least 90%, at least 95%, at least 96%, at least 97%, at least 98%, or at least 99% identical to the amino acid sequence as set forth in SEQ ID NO:217, and a heavy chain that comprises an amino acid sequence as set forth in SEQ ID NO:218, or an amino acid sequence that is at least 85%, at least 90%, at least 95%, at least 96%, at least 97%, at least 98%, or at least 99% identical to the amino acid sequence as set forth in SEQ ID NO:218;
   5) a light chain that comprises an amino acid sequence as set forth in SEQ ID NO:219, or an amino acid sequence that is at least 85%, at least 90%, at least 95%, at least 96%, at least 97%, at least 98%, or at least 99% identical to the amino acid sequence as set forth in SEQ ID NO:219, and a heavy chain that comprises an amino acid sequence as set forth in SEQ ID NO:220, or an amino acid sequence that is at least 85%, at least 90%, at least 95%, at least 96%, at least 97%, at least 98%, or at least 99% identical to the amino acid sequence as set forth in SEQ ID NO:220;
   6) a light chain that comprises an amino acid sequence as set forth in SEQ ID NO:221, or an amino acid sequence that is at least 85%, at least 90%, at least 95%, at least 96%, at least 97%, at least 98%, or at least 99% identical to the amino acid sequence as set forth in SEQ ID NO:221, and a heavy chain that comprises an amino acid sequence as set forth in SEQ ID NO:222, or an amino acid sequence that is at least 85%, at least 90%, at least 95%, at least 96%, at least 97%, at least 98%, or at least 99% identical to the amino acid sequence as set forth in SEQ ID NO:222; and
   7) a light chain that comprises an amino acid sequence as set forth in SEQ ID NO:223, or an amino acid sequence that is at least 85%, at least 90%, at least 95%, at least 96%, at least 97%, at least 98%, or at least 99% identical to the amino acid sequence as set forth in SEQ ID NO:223, and a heavy chain that comprises an amino acid sequence as set forth in SEQ ID NO:224, or an amino acid sequence that is at least 85%, at least 90%, at least 95%, at least 96%, at least 97%, at least 98%, or at least 99% identical to the amino acid sequence as set forth in SEQ ID NO:224.
5. The isolated antibody or the antigen binding fragment thereof according to any one of clauses 1 to 4, wherein the isolated antibody or the antigen binding fragment thereof consists of a light chain and a heavy chain selected from at least one of the following groups:
   1) a light chain that consists of an amino acid sequence as set forth in SEQ ID NO:211, and a heavy chain that consists of an amino acid sequence as set forth in SEQ ID NO:212;
   2) a light chain that consists of an amino acid sequence as set forth in SEQ ID NO:213, and a heavy chain that consists of an amino acid sequence as set forth in SEQ ID NO:214;
   3) a light chain that consists of an amino acid sequence as set forth in SEQ ID NO:215, and a heavy chain that consists of an amino acid sequence as set forth in SEQ ID NO:216;
   4) a light chain that consists of an amino acid sequence as set forth in SEQ ID NO:217, and a heavy chain that consists of an amino acid sequence as set forth in SEQ ID NO:218;
   5) a light chain that consists of an amino acid sequence as set forth in SEQ ID NO:219, and a heavy chain that consists of an amino acid sequence as set forth in SEQ ID NO:220;
   6) a light chain that consists of an amino acid sequence as set forth in SEQ ID NO:221, and a heavy chain that consists of an amino acid sequence as set forth in SEQ ID NO:222; and
   7) a light chain that consists of an amino acid sequence as set forth in SEQ ID NO:223, and a heavy chain that consists of an amino acid sequence as set forth in SEQ ID NO:224.
6. An isolated antibody specifically binding to an ITGA2 protein or an antigen-binding fragment thereof, wherein the isolated antibody or the antigen-binding fragment thereof comprises a light chain and a heavy chain selected from at least one of the following groups:
   1) a light chain that comprises an amino acid sequence encoded by a nucleotide sequence as set forth in SEQ ID NO:9, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:9, or consists of an amino acid sequence encoded by the nucleotide sequence as set forth in SEQ ID NO:9, and
      a heavy chain that comprises an amino acid sequence encoded by a nucleotide sequence as set forth in SEQ ID NO: 10, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:10, or consists of an amino acid sequence encoded by the nucleotide sequence as set forth in SEQ ID NO:10;
   2) a light chain that comprises an amino acid sequence encoded by a nucleotide sequence as set forth in SEQ ID NO:11, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:11, or consists of an amino acid sequence encoded by the nucleotide sequence as set forth in SEQ ID NO:11, and
      a heavy chain that comprises an amino acid sequence encoded by a nucleotide sequence as set forth in SEQ ID NO:12, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:12, or consists of an amino acid sequence encoded by the nucleotide sequence as set forth in SEQ ID NO:12;
   3) a light chain that comprises an amino acid sequence encoded by a nucleotide sequence as set forth in SEQ ID NO:13, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:13, or consists of an amino acid sequence encoded by the nucleotide sequence as set forth in SEQ ID NO:13, and
      a heavy chain that comprises an amino acid sequence encoded by a nucleotide sequence as set forth in SEQ ID NO:14, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:14, or consists of an amino acid sequence encoded by the nucleotide sequence as set forth in SEQ ID NO:14;
   4) a light chain that comprises an amino acid sequence encoded by a nucleotide sequence as set forth in SEQ ID NO:15, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:15, or consists of an amino acid sequence encoded by the nucleotide sequence as set forth in SEQ ID NO:15, and
      a heavy chain that comprises an amino acid sequence encoded by a nucleotide sequence as set forth in SEQ ID NO:16, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:16, or consists of an amino acid sequence encoded by the nucleotide sequence as set forth in SEQ ID NO:16;
   5) a light chain that comprises an amino acid sequence encoded by a nucleotide sequence as set forth in SEQ ID NO:17, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:17, or consists of an amino acid sequence encoded by the nucleotide sequence as set forth in SEQ ID NO:17, and
      a heavy chain that comprises an amino acid sequence encoded by a nucleotide sequence as set forth in SEQ ID NO:18, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:18, or consists of an amino acid sequence encoded by the nucleotide sequence as set forth in SEQ ID NO:18;
   6) a light chain that comprises an amino acid sequence encoded by a nucleotide sequence as set forth in SEQ ID NO:19, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:19, or consists of an amino acid sequence encoded by the nucleotide sequence as set forth in SEQ ID NO:19, and
      a heavy chain that comprises an amino acid sequence encoded by a nucleotide sequence as set forth in SEQ ID NO:20, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:20, or consists of an amino acid sequence encoded by the nucleotide sequence as set forth in SEQ ID NO:20; and
   7) a light chain that comprises an amino acid sequence encoded by a nucleotide sequence as set forth in SEQ ID NO:21, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:21, or consists of an amino acid sequence encoded by the nucleotide sequence as set forth in SEQ ID NO:21, and
      a heavy chain that comprises an amino acid sequence encoded by a nucleotide sequence as set forth in SEQ ID NO:22, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:22, or consists of an amino acid sequence encoded by the nucleotide sequence as set forth in SEQ ID NO:22.
7. The isolated antibody or the antigen binding fragment thereof according to any one of clauses 1 to 6,
   wherein the antibody is capable of binding to an ITGA2 protein in vitro at an EC50 of less than 1 nM; and/or
   wherein the antibody is capable of binding to an ITGA2 protein on the surface of HuCCT1 cells with an EC50 of less than 5 nM;
   and/or wherein the antibody is a monoclonal antibody, a polyclonal antibody, a chimeric antibody, or a humanized antibody.
8. An isolated nucleic acid molecule encoding the antibody or the antigen-binding fragment thereof according to any one of clauses 1 to 7; or
   the nucleic acid molecule comprises or consists of a nucleotide sequence combination selected from any one of the following nucleotide sequences: SEQ ID NO:9 and SEQ ID NO:10; SEQ ID NO:11 and SEQ ID NO:12; SEQ ID NO:13 and SEQ ID NO:14; SEQ ID NO:15 and SEQ ID NO:16; SEQ ID NO:17 and SEQ ID NO:18; SEQ ID NO:19 and SEQ ID NO:20; and SEQ ID NO:21 and SEQ ID NO:22.
9. The isolated nucleic acid molecule according to clause 8, wherein the nucleic acid molecule is operably linked to an expression regulatory sequence.
10. An expression vector comprising the nucleic acid molecule according to clause 8 or 9.
11. A host cell comprising the nucleic acid molecule according to clause 8 or 9 or the expression vector according to clause 10.
12. A hybridoma cell expressing the antibody or the antigen-binding fragment thereof according to any one of clauses 1 to 7.
13. An antibody-drug conjugate of the formula:
   Ab-L-D, wherein:
      "Ab" represents at least one antibody or antigen-binding fragment thereof according to the present invention described above;
      "L" represents the linker that connects the antibody moiety to the partner molecule; and
      "D" represents the partner molecule;
      or
   Ab-(L-D)p, wherein:
      "Ab" represents at least one antibody or antigen-binding fragment thereof according to the present invention described above;
      "L" represents the linker that connects the antibody moiety to the partner molecule;
      "D" represents the partner molecule; and
      "p" represents the number of linker/partner molecules (preferably drug payloads) conjugated to the antibody or the antigen-binding fragment thereof according to the present invention described above; optionally, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.
14. The antibody-drug conjugate according to clause 13, wherein the linker is selected from one of the following: ve, mc-GGFG, AcLysvc, mc, MalPeg6, m(H20)c and m(H20)cvc; and/or the linker is cleavable.
15. The antibody-drug conjugate according to clause 13 or 14, wherein
   the partner molecule is a drug payload, wherein the drug payload is a therapeutic molecule; optionally, the drug payload is selected from: a cytotoxic agent, a cytostatic agent, an immunomodulator and a chemotherapeutic agent; optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD; or
   the partner molecule is a marker molecule; optionally, the marker molecule is selected from one of: a radioactive label (for example, an isotope), a fluorescent label (for example, a fluorescent dye), a chemical substance, and an enzyme or label that can detect a modification.
16. A pharmaceutical composition comprising the antibody or the antigen-binding fragment thereof according to any one of clauses 1-7, or the antibody-drug conjugate according to any one of clauses 13 to 15, and optionally a pharmaceutically acceptable carrier.
17. Use of the antibody or the antigen-binding fragment thereof according to any one of the clauses 1-7 or the antibody-drug conjugate according to any one of the clauses 13 to 15 in preparing a pharmaceutical composition; optionally, the pharmaceutical composition is used for treating a malignant tumor, preventing and/or treating metastasis or relapse of a malignant tumor in a patient; optionally, the pharmaceutical composition is used for detecting presence of a malignant tumor in a patient; and optionally, the pharmaceutical composition is used for prognosticating relapse or progression of a malignant tumor in a patient.
18. A method for treating a malignant tumor, preventing and/or treating metastasis or relapse of a malignant tumor in a patient, comprising administering to the patient an effective amount of the antibody or the antigen-binding fragment thereof according to any one of clauses 1 to 7, the antibody-drug conjugate according to any one of clauses 13 to 15, or the pharmaceutical composition according to clause 16.
19. The method according to clause 18, wherein the malignant tumor is selected from a group consisting of the following: breast cancer, large intestine cancer, skin cancer, pancreatic cancer, prostate cancer, liver cancer, lung cancer, gastric cancer, leukemia, bile duct cancer, cervical cancer, colorectal cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
20. The method according to clause 18 or 19, further comprising administering to the patient other anti-tumor treatments, such as administering a chemotherapeutic agent, an antibody targeting other tumor-specific antigens, or a radiation therapy.
21. A method for detecting presence of tumor cells in a biological sample, comprising:
   a) contacting the biological sample with the antibody or antigen-binding fragment thereof according to any one of clauses 1 to 7;
   b) detecting binding of the antibody or antigen-binding fragment thereof to a target antigen in the biological sample,
   wherein the detected binding represents presence of the tumor cells in the biological sample.
22. A method for detecting presence of a malignant tumor in a patient, comprising:
   a) contacting the biological sample obtained from the patient with the antibody or antigen-binding fragment thereof according to any one of clauses 1 to 7;
   b) detecting binding of the antibody or the antigen-binding fragment thereof to a target antigen in the biological sample, wherein the detected binding represents presence of the malignant tumor in the patient.
23. A method for prognosticating relapse or progression of a malignant tumor in a patient, wherein the method comprises:
   (a) isolating a biological sample comprising tumor cells from the patient;
   (b) contacting the biological sample comprising tumor cells with the antibody or antigen-binding fragment thereof according to any one of clauses 1 to 7; and
   (c) identifying presence of the tumor cells that bind to the antibody or the antigen-binding fragment thereof,
   thereby prognosticating the relapse or progression of the malignant tumor in the patient.
24. The method according to clause 23, wherein the progression of the malignant tumor includes metastasis of the malignant tumor in the patient.
25. The method according to clause 23 or 24, wherein the biological sample is selected from one of the following: a blood sample, a tissue sample, and a lymph sample.
26. The method according to any one of clauses 23 to 25, wherein the malignant tumor is selected from a group consisting of the following: breast cancer, colorectal cancer, large intestine cancer, skin cancer, pancreatic cancer, prostate cancer, liver cancer, lung cancer, gastric cancer, leukemia, bile duct cancer, cervical cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
27. A method for producing an antibody or an antigen-binding fragment thereof targeting human tumor cells that express ITGA2, wherein the method comprises:
   (i) culturing the host cell according to clause 11 in a condition suitable for expressing the nucleic acid molecule or the expression vector, and
   (ii) isolating and purifying the antibody or the antigen-binding fragment thereof expressed by the nucleic acid molecule or the expression vector.

### Set C of clauses

1. An isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
   1) a light chain variable region that comprises (a) LCDR1 as set forth in SEQ ID NO: 130, (b) LCDR2 as set forth in SEQ ID NO: 131, and (c) LCDR3 as set forth in SEQ ID NO: 132, and a heavy chain variable region that comprises (d) HCDR1 as set forth in SEQ ID NO: 134, (e) HCDR2 as set forth in SEQ ID NO: 135, and (f) HCDR3 as set forth in SEQ ID NO: 136;
   2) a light chain variable region that comprises (a) LCDR1 as set forth in SEQ ID NO: 138, (b) LCDR2 as set forth in SEQ ID NO: 139, and (c) LCDR3 as set forth in SEQ ID NO: 140, and a heavy chain variable region that comprises (d) HCDR1 as set forth in SEQ ID NO: 142, (e) HCDR2 as set forth in SEQ ID NO: 143, and (f) HCDR3 as set forth in SEQ ID NO:144;
   3) a light chain variable region that comprises (a) LCDR1 as set forth in SEQ ID NO: 146, (b) LCDR2 as set forth in SEQ ID NO: 147, and (c) LCDR3 as set forth in SEQ ID NO: 148, and a heavy chain variable region that comprises (d) HCDR1 as set forth in SEQ ID NO: 150, (e) HCDR2 as set forth in SEQ ID NO: 151, and (f) HCDR3 as set forth in SEQ ID NO: 152; and
   4) a light chain variable region that comprises (a) LCDR1 as set forth in SEQ ID NO: 154, (b) LCDR2 as set forth in SEQ ID NO: 155, and (c) LCDR3 as set forth in SEQ ID NO: 156, and a heavy chain variable region that comprises (d) HCDR1 as set forth in SEQ ID NO: 158, (e) HCDR2 as set forth in SEQ ID NO: 159, and (f) HCDR3 as set forth in SEQ ID NO: 160.
2. The isolated antibody or the antigen binding fragment thereof according to clause 1, wherein the isolated antibody or the antigen binding fragment thereof comprises a light chain variable region and a heavy chain variable region selected from at least one of the following groups:
   1) a light chain variable region that comprises an amino acid sequence as set forth in SEQ ID NO:129, or an amino acid sequence that is at least 85%, at least 90%, at least 95%, at least 96%, at least 97%, at least 98%, or at least 99% identical to the amino acid sequence as set forth in SEQ ID NO:129, and a heavy chain variable region that comprises an amino acid sequence as set forth in SEQ ID NO:133, or an amino acid sequence that is at least 85%, at least 90%, at least 95%, at least 96%, at least 97%, at least 98%, or at least 99% identical to the amino acid sequence as set forth in SEQ ID NO:133;
   2) a light chain variable region that comprises an amino acid sequence as set forth in SEQ ID NO:137, or an amino acid sequence that is at least 85%, at least 90%, at least 95%, at least 96%, at least 97%, at least 98%, or at least 99% identical to the amino acid sequence as set forth in SEQ ID NO:137, and a heavy chain variable region that comprises an amino acid sequence as set forth in SEQ ID NO:141, or an amino acid sequence that is at least 85%, at least 90%, at least 95%, at least 96%, at least 97%, at least 98%, or at least 99% identical to the amino acid sequence as set forth in SEQ ID NO:141;
   3) a light chain variable region that comprises an amino acid sequence as set forth in SEQ ID NO:145, or an amino acid sequence that is at least 85%, at least 90%, at least 95%, at least 96%, at least 97%, at least 98%, or at least 99% identical to the amino acid sequence as set forth in SEQ ID NO: 145, and a heavy chain variable region that comprises an amino acid sequence as set forth in SEQ ID NO:149, or an amino acid sequence that is at least 85%, at least 90%, at least 95%, at least 96%, at least 97%, at least 98%, or at least 99% identical to the amino acid sequence as set forth in SEQ ID NO:149; and
   4) a light chain variable region that comprises an amino acid sequence as set forth in SEQ ID NO:153, or an amino acid sequence that is at least 85%, at least 90%, at least 95%, at least 96%, at least 97%, at least 98%, or at least 99% identical to the amino acid sequence as set forth in SEQ ID NO: 153, and a heavy chain variable region that comprises an amino acid sequence as set forth in SEQ ID NO:157, or an amino acid sequence that is at least 85%, at least 90%, at least 95%, at least 96%, at least 97%, at least 98%, or at least 99% identical to the amino acid sequence as set forth in SEQ ID NO:157.
3. The isolated antibody or the antigen binding fragment thereof according to clause 1 or 2, wherein the isolated antibody or the antigen binding fragment thereof further comprises a light chain constant region and a heavy chain constant region,
   the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201,
   the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.
4. The isolated antibody or the antigen binding fragment thereof according to any one of clauses 1 to 3, wherein the isolated antibody or the antigen binding fragment thereof comprises a light chain and a heavy chain selected from at least one of the following groups:
   1) a light chain that comprises an amino acid sequence as set forth in SEQ ID NO:225, or an amino acid sequence that is at least 85%, at least 90%, at least 95%, at least 96%, at least 97%, at least 98%, or at least 99% identical to the amino acid sequence as set forth in SEQ ID NO:225, and a heavy chain that comprises an amino acid sequence as set forth in SEQ ID NO:226, or an amino acid sequence that is at least 85%, at least 90%, at least 95%, at least 96%, at least 97%, at least 98%, or at least 99% identical to the amino acid sequence as set forth in SEQ ID NO:226;
   2) a light chain that comprises an amino acid sequence as set forth in SEQ ID NO:227, or an amino acid sequence that is at least 85%, at least 90%, at least 95%, at least 96%, at least 97%, at least 98%, or at least 99% identical to the amino acid sequence as set forth in SEQ ID NO:227, and a heavy chain that comprises an amino acid sequence as set forth in SEQ ID NO:228, or an amino acid sequence that is at least 85%, at least 90%, at least 95%, at least 96%, at least 97%, at least 98%, or at least 99% identical to the amino acid sequence as set forth in SEQ ID NO:228;
   3) a light chain that comprises an amino acid sequence as set forth in SEQ ID NO:229, or an amino acid sequence that is at least 85%, at least 90%, at least 95%, at least 96%, at least 97%, at least 98%, or at least 99% identical to the amino acid sequence as set forth in SEQ ID NO:229, and a heavy chain that comprises an amino acid sequence as set forth in SEQ ID NO:230, or an amino acid sequence that is at least 85%, at least 90%, at least 95%, at least 96%, at least 97%, at least 98%, or at least 99% identical to the amino acid sequence as set forth in SEQ ID NO:230;
   4) a light chain that comprises an amino acid sequence as set forth in SEQ ID NO:231, or an amino acid sequence that is at least 85%, at least 90%, at least 95%, at least 96%, at least 97%, at least 98%, or at least 99% identical to the amino acid sequence as set forth in SEQ ID NO:231, and a heavy chain that comprises an amino acid sequence as set forth in SEQ ID NO:232, or an amino acid sequence that is at least 85%, at least 90%, at least 95%, at least 96%, at least 97%, at least 98%, or at least 99% identical to the amino acid sequence as set forth in SEQ ID NO:232.
5. The isolated antibody or the antigen binding fragment thereof according to any one of clauses 1 to 4, wherein the isolated antibody or the antigen binding fragment thereof consists of a light chain and a heavy chain selected from at least one of the following groups:
   1) a light chain that consists of an amino acid sequence as set forth in SEQ ID NO:225, and a heavy chain that consists of an amino acid sequence as set forth in SEQ ID NO:226;
   2) a light chain that consists of an amino acid sequence as set forth in SEQ ID NO:227, and a heavy chain that consists of an amino acid sequence as set forth in SEQ ID NO:228;
   3) a light chain that consists of an amino acid sequence as set forth in SEQ ID NO:229, and a heavy chain that consists of an amino acid sequence as set forth in SEQ ID NO:230;
   4) a light chain that consists of an amino acid sequence as set forth in SEQ ID NO:231, and a heavy chain that consists of an amino acid sequence as set forth in SEQ ID NO:232.
6. An isolated antibody specifically binding to an ITGA2 protein or an antigen-binding fragment thereof, wherein the isolated antibody or the antigen-binding fragment thereof comprises a light chain and a heavy chain selected from at least one of the following groups:
   1) a light chain that comprises an amino acid sequence encoded by a nucleotide sequence as set forth in SEQ ID NO:23, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:23, or consists of an amino acid sequence encoded by the nucleotide sequence as set forth in SEQ ID NO:23, and
      a heavy chain that comprises an amino acid sequence encoded by a nucleotide sequence as set forth in SEQ ID NO:24, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:24, or consists of an amino acid sequence encoded by the nucleotide sequence as set forth in SEQ ID NO:24;
   2) a light chain that comprises an amino acid sequence encoded by a nucleotide sequence as set forth in SEQ ID NO:25, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:25, or consists of an amino acid sequence encoded by the nucleotide sequence as set forth in SEQ ID NO:25, and
      a heavy chain that comprises an amino acid sequence encoded by a nucleotide sequence as set forth in SEQ ID NO:26, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:26, or consists of an amino acid sequence encoded by the nucleotide sequence as set forth in SEQ ID NO:26;
   3) a light chain that comprises an amino acid sequence encoded by a nucleotide sequence as set forth in SEQ ID NO:27, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:27, or consists of an amino acid sequence encoded by the nucleotide sequence as set forth in SEQ ID NO:27, and
      a heavy chain that comprises an amino acid sequence encoded by a nucleotide sequence as set forth in SEQ ID NO:28, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:28, or consists of an amino acid sequence encoded by the nucleotide sequence as set forth in SEQ ID NO:28; and
   4) a light chain that comprises an amino acid sequence encoded by a nucleotide sequence as set forth in SEQ ID NO:29, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:29, or consists of an amino acid sequence encoded by the nucleotide sequence as set forth in SEQ ID NO:29, and
      a heavy chain that comprises an amino acid sequence encoded by a nucleotide sequence as set forth in SEQ ID NO:30, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:30, or consists of an amino acid sequence encoded by the nucleotide sequence as set forth in SEQ ID NO:30.
7. The isolated antibody or the antigen binding fragment thereof according to any one of clauses 1 to 6,
   wherein the antibody is capable of binding to an ITGA2 protein in vitro at an EC50 of less than 1 nM; and/or
   wherein the antibody is capable of binding to an ITGA2 protein on the surface of HuCCT1 cells with an EC50 of less than 5 nM;
   and/or wherein the antibody is a monoclonal antibody, a polyclonal antibody, a chimeric antibody, or a humanized antibody.
8. An isolated nucleic acid molecule encoding the antibody or the antigen-binding fragment thereof according to any one of clauses 1 to 7; or
   the nucleic acid molecule comprises or consists of a nucleotide sequence combination selected from any one of the following nucleotide sequences: SEQ ID NO:23 and SEQ ID NO:24; SEQ ID NO:25 and SEQ ID NO:26; SEQ ID NO:27 and SEQ ID NO:28; and SEQ ID NO:29 and SEQ ID NO:30.
9. The isolated nucleic acid molecule according to clause 8, wherein the nucleic acid molecule is operably linked to an expression regulatory sequence.
10. An expression vector comprising the nucleic acid molecule according to clause 8 or 9.
11. A host cell comprising the nucleic acid molecule according to clause 8 or 9 or the expression vector according to clause 10.
12. A hybridoma cell expressing the antibody or the antigen-binding fragment thereof according to any one of clauses 1 to 7.
13. An antibody-drug conjugate of the formula:
   Ab-L-D, wherein:
      "Ab" represents at least one antibody or antigen-binding fragment thereof according to the present invention described above;
      "L" represents the linker that connects the antibody moiety to the partner molecule; and
      "D" represents the partner molecule;
      or
   Ab-(L-D)p, wherein:
      "Ab" represents at least one antibody or antigen-binding fragment thereof according to the present invention described above;
      "L" represents the linker that connects the antibody moiety to the partner molecule;
      "D" represents the partner molecule; and
      "p" represents the number of linker/partner molecules (preferably drug payloads) conjugated to the antibody or the antigen-binding fragment thereof according to the present invention described above; optionally, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.
14. The antibody-drug conjugate according to clause 13, wherein the linker is selected from one of the following: vc, mc-GGFG, AcLysvc, mc, MalPeg6, m(H20)c and m(H20)cvc; and/or the linker is cleavable.
15. The antibody-drug conjugate according to clause 13 or 14, wherein
   the partner molecule is a drug payload, wherein the drug payload is a therapeutic molecule; optionally, the drug payload is selected from: a cytotoxic agent, a cytostatic agent, an immunomodulator and a chemotherapeutic agent; optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD; or
   the partner molecule is a marker molecule; optionally, the marker molecule is selected from one of: a radioactive label (for example, an isotope), a fluorescent label (for example, a fluorescent dye), a chemical substance, and an enzyme or label that can detect a modification.
16. A pharmaceutical composition comprising the antibody or the antigen-binding fragment thereof according to any one of clauses 1-7, or the antibody-drug conjugate according to any one of clauses 13 to 15, and optionally a pharmaceutically acceptable carrier.
17. Use of the antibody or the antigen-binding fragment thereof according to any one of the clauses 1-7 or the antibody-drug conjugate according to any one of the clauses 13 to 15 in preparing a pharmaceutical composition; optionally, the pharmaceutical composition is used for treating a malignant tumor, preventing and/or treating metastasis or relapse of a malignant tumor in a patient; optionally, the pharmaceutical composition is used for detecting presence of a malignant tumor in a patient; and optionally, the pharmaceutical composition is used for prognosticating relapse or progression of a malignant tumor in a patient.
18. A method for treating a malignant tumor, preventing and/or treating metastasis or relapse of a malignant tumor in a patient, comprising administering to the patient an effective amount of the antibody or the antigen-binding fragment thereof according to any one of clauses 1 to 7, the antibody-drug conjugate according to any one of clauses 13 to 15, or the pharmaceutical composition according to clause 16.
19. The method according to clause 18, wherein the malignant tumor is selected from a group consisting of the following: breast cancer, large intestine cancer, skin cancer, pancreatic cancer, prostate cancer, liver cancer, lung cancer, gastric cancer, leukemia, bile duct cancer, cervical cancer, colorectal cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
20. The method according to clause 18 or 19, further comprising administering to the patient other anti-tumor treatments, such as administering a chemotherapeutic agent, an antibody targeting other tumor-specific antigens, or a radiation therapy.
21. A method for detecting presence of tumor cells in a biological sample, comprising:
   a) contacting the biological sample with the antibody or antigen-binding fragment thereof according to any one of clauses 1 to 7;
   b) detecting binding of the antibody or antigen-binding fragment thereof to a target antigen in the biological sample,
   wherein the detected binding represents presence of the tumor cells in the biological sample.
22. A method for detecting presence of a malignant tumor in a patient, comprising:
   a) contacting the biological sample obtained from the patient with the antibody or antigen-binding fragment thereof according to any one of clauses 1 to 7;
   b) detecting binding of the antibody or the antigen-binding fragment thereof to a target antigen in the biological sample, wherein the detected binding represents presence of the malignant tumor in the patient.
23. A method for prognosticating relapse or progression of a malignant tumor in a patient, wherein the method comprises:
   (a) isolating a biological sample comprising tumor cells from the patient;
   (b) contacting the biological sample comprising tumor cells with the antibody or antigen-binding fragment thereof according to any one of clauses 1 to 7; and
   (c) identifying presence of the tumor cells that bind to the antibody or the antigen-binding fragment thereof,
   thereby prognosticating the relapse or progression of the malignant tumor in the patient.
24. The method according to clause 23, wherein the progression of the malignant tumor includes metastasis of the malignant tumor in the patient.
25. The method according to clause 23 or 24, wherein the biological sample is selected from one of the following: a blood sample, a tissue sample, and a lymph sample.
26. The method according to any one of clauses 23 to 25, wherein the malignant tumor is selected from a group consisting of the following: breast cancer, colorectal cancer, large intestine cancer, skin cancer, pancreatic cancer, prostate cancer, liver cancer, lung cancer, gastric cancer, leukemia, bile duct cancer, cervical cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
27. A method for producing an antibody or an antigen-binding fragment thereof targeting human tumor cells that express ITGA2, wherein the method comprises:
   (i) culturing the host cell according to clause 11 in a condition suitable for expressing the nucleic acid molecule or the expression vector, and
   (ii) isolating and purifying the antibody or the antigen-binding fragment thereof expressed by the nucleic acid molecule or the expression vector.

### Set D of clauses

1. An isolated antibody specifically binding to an ITGA2 protein or an antigen-binding fragment thereof, comprising a light chain variable region comprising LCDR1, LCDR2, and LCDR3 sequences, and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
   1) a light chain variable region that comprises (a) LCDR1 as set forth in SEQ ID NO: 162, (b) LCDR2 as set forth in SEQ ID NO: 163, and (c) LCDR3 as set forth in SEQ ID NO: 164, and a heavy chain variable region that comprises (d) HCDR1 as set forth in SEQ ID NO: 166, (e) HCDR2 as set forth in SEQ ID NO: 167, and (f) HCDR3 as set forth in SEQ ID NO: 168;
   2) a light chain variable region that comprises (a) LCDR1 as set forth in SEQ ID NO: 170, (b) LCDR2 as set forth in SEQ ID NO: 171, and (c) LCDR3 as set forth in SEQ ID NO: 172, and a heavy chain variable region that comprises (d) HCDR1 as set forth in SEQ ID NO: 174, (e) HCDR2 as set forth in SEQ ID NO: 175, and (f) HCDR3 as set forth in SEQ ID NO:176;
   3) a light chain variable region that comprises (a) LCDR1 as set forth in SEQ ID NO: 178, (b) LCDR2 as set forth in SEQ ID NO: 179, and (c) LCDR3 as set forth in SEQ ID NO: 180, and a heavy chain variable region that comprises (d) HCDR1 as set forth in SEQ ID NO: 182, (e) HCDR2 as set forth in SEQ ID NO: 183, and (f) HCDR3 as set forth in SEQ ID NO: 184;
   4) a light chain variable region that comprises (a) LCDR1 as set forth in SEQ ID NO: 186, (b) LCDR2 as set forth in SEQ ID NO: 187, and (c) LCDR3 as set forth in SEQ ID NO: 188, and a heavy chain variable region that comprises (d) HCDR1 as set forth in SEQ ID NO: 190, (e) HCDR2 as set forth in SEQ ID NO: 191, and (f) HCDR3 as set forth in SEQ ID NO: 192; and
   5) a light chain variable region that comprises (a) LCDR1 as set forth in SEQ ID NO: 194, (b) LCDR2 as set forth in SEQ ID NO: 195, and (c) LCDR3 as set forth in SEQ ID NO: 196, and a heavy chain variable region that comprises (d) HCDR1 as set forth in SEQ ID NO: 198, (e) HCDR2 as set forth in SEQ ID NO: 199, and (f) HCDR3 as set forth in SEQ ID NO: 200.
2. The isolated antibody or the antigen binding fragment thereof according to clause 1, wherein the isolated antibody or the antigen binding fragment thereof comprises a light chain variable region and a heavy chain variable region selected from at least one of the following groups:
   1) a light chain variable region that comprises an amino acid sequence as set forth in SEQ ID NO:161, or an amino acid sequence that is at least 85%, at least 90%, at least 95%, at least 96%, at least 97%, at least 98%, or at least 99% identical to the amino acid sequence as set forth in SEQ ID NO: 161, and a heavy chain variable region that comprises an amino acid sequence as set forth in SEQ ID NO: 165, or an amino acid sequence that is at least 85%, at least 90%, at least 95%, at least 96%, at least 97%, at least 98%, or at least 99% identical to the amino acid sequence as set forth in SEQ ID NO:165;
   2) a light chain variable region that comprises an amino acid sequence as set forth in SEQ ID NO: 169, or an amino acid sequence that is at least 85%, at least 90%, at least 95%, at least 96%, at least 97%, at least 98%, or at least 99% identical to the amino acid sequence as set forth in SEQ ID NO: 169, and a heavy chain variable region that comprises an amino acid sequence as set forth in SEQ ID NO: 173, or an amino acid sequence that is at least 85%, at least 90%, at least 95%, at least 96%, at least 97%, at least 98%, or at least 99% identical to the amino acid sequence as set forth in SEQ ID NO: 173;
   3) a light chain variable region that comprises an amino acid sequence as set forth in SEQ ID NO: 177, or an amino acid sequence that is at least 85%, at least 90%, at least 95%, at least 96%, at least 97%, at least 98%, or at least 99% identical to the amino acid sequence as set forth in SEQ ID NO: 177, and a heavy chain variable region that comprises an amino acid sequence as set forth in SEQ ID NO:181, or an amino acid sequence that is at least 85%, at least 90%, at least 95%, at least 96%, at least 97%, at least 98%, or at least 99% identical to the amino acid sequence as set forth in SEQ ID NO:181;
   4) a light chain variable region that comprises an amino acid sequence as set forth in SEQ ID NO:185, or an amino acid sequence that is at least 85%, at least 90%, at least 95%, at least 96%, at least 97%, at least 98%, or at least 99% identical to the amino acid sequence as set forth in SEQ ID NO: 185, and a heavy chain variable region that comprises an amino acid sequence as set forth in SEQ ID NO:189, or an amino acid sequence that is at least 85%, at least 90%, at least 95%, at least 96%, at least 97%, at least 98%, or at least 99% identical to the amino acid sequence as set forth in SEQ ID NO:189; and
   5) a light chain variable region that comprises an amino acid sequence as set forth in SEQ ID NO:193, or an amino acid sequence that is at least 85%, at least 90%, at least 95%, at least 96%, at least 97%, at least 98%, or at least 99% identical to the amino acid sequence as set forth in SEQ ID NO: 193, and a heavy chain variable region that comprises an amino acid sequence as set forth in SEQ ID NO:197, or an amino acid sequence that is at least 85%, at least 90%, at least 95%, at least 96%, at least 97%, at least 98%, or at least 99% identical to the amino acid sequence as set forth in SEQ ID NO:197.
3. The isolated antibody or the antigen binding fragment thereof according to clause 1 or 2, wherein the isolated antibody or the antigen binding fragment thereof further comprises a light chain constant region and a heavy chain constant region,
   the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201,
   the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202.
4. The isolated antibody or the antigen binding fragment thereof according to any one of clauses 1 to 3, wherein the isolated antibody or the antigen binding fragment thereof comprises a light chain and a heavy chain selected from at least one of the following groups:
   1) a light chain that comprises an amino acid sequence as set forth in SEQ ID NO:233, or an amino acid sequence that is at least 85%, at least 90%, at least 95%, at least 96%, at least 97%, at least 98%, or at least 99% identical to the amino acid sequence as set forth in SEQ ID NO:233, and a heavy chain that comprises an amino acid sequence as set forth in SEQ ID NO:234, or an amino acid sequence that is at least 85%, at least 90%, at least 95%, at least 96%, at least 97%, at least 98%, or at least 99% identical to the amino acid sequence as set forth in SEQ ID NO:234;
   2) a light chain that comprises an amino acid sequence as set forth in SEQ ID NO:235, or an amino acid sequence that is at least 85%, at least 90%, at least 95%, at least 96%, at least 97%, at least 98%, or at least 99% identical to the amino acid sequence as set forth in SEQ ID NO:235, and a heavy chain that comprises an amino acid sequence as set forth in SEQ ID NO:236, or an amino acid sequence that is at least 85%, at least 90%, at least 95%, at least 96%, at least 97%, at least 98%, or at least 99% identical to the amino acid sequence as set forth in SEQ ID NO:236;
   3) a light chain that comprises an amino acid sequence as set forth in SEQ ID NO:237, or an amino acid sequence that is at least 85%, at least 90%, at least 95%, at least 96%, at least 97%, at least 98%, or at least 99% identical to the amino acid sequence as set forth in SEQ ID NO:237, and a heavy chain that comprises an amino acid sequence as set forth in SEQ ID NO:238, or an amino acid sequence that is at least 85%, at least 90%, at least 95%, at least 96%, at least 97%, at least 98%, or at least 99% identical to the amino acid sequence as set forth in SEQ ID NO:238;
   4) a light chain that comprises an amino acid sequence as set forth in SEQ ID NO:239, or an amino acid sequence that is at least 85%, at least 90%, at least 95%, at least 96%, at least 97%, at least 98%, or at least 99% identical to the amino acid sequence as set forth in SEQ ID NO:239, and a heavy chain that comprises an amino acid sequence as set forth in SEQ ID NO:240, or an amino acid sequence that is at least 85%, at least 90%, at least 95%, at least 96%, at least 97%, at least 98%, or at least 99% identical to the amino acid sequence as set forth in SEQ ID NO:240; and
   5) a light chain that comprises an amino acid sequence as set forth in SEQ ID NO:241, or an amino acid sequence that is at least 85%, at least 90%, at least 95%, at least 96%, at least 97%, at least 98%, or at least 99% identical to the amino acid sequence as set forth in SEQ ID NO:241, and a heavy chain that comprises an amino acid sequence as set forth in SEQ ID NO:242, or an amino acid sequence that is at least 85%, at least 90%, at least 95%, at least 96%, at least 97%, at least 98%, or at least 99% identical to the amino acid sequence as set forth in SEQ ID NO:242.
5. The isolated antibody or the antigen binding fragment thereof according to any one of clauses 1 to 4, wherein the isolated antibody or the antigen binding fragment thereof consists of a light chain and a heavy chain selected from at least one of the following groups:
   1) a light chain that consists of an amino acid sequence as set forth in SEQ ID NO:233, and a heavy chain that consists of an amino acid sequence as set forth in SEQ ID NO:234;
   2) a light chain that consists of an amino acid sequence as set forth in SEQ ID NO:235, and a heavy chain that consists of an amino acid sequence as set forth in SEQ ID NO:236;
   3) a light chain that consists of an amino acid sequence as set forth in SEQ ID NO:237, and a heavy chain that consists of an amino acid sequence as set forth in SEQ ID NO:238;
   4) a light chain that consists of an amino acid sequence as set forth in SEQ ID NO:239, and a heavy chain that consists of an amino acid sequence as set forth in SEQ ID NO:240; and
   5) a light chain that consists of an amino acid sequence as set forth in SEQ ID NO:241, and a heavy chain that consists of an amino acid sequence as set forth in SEQ ID NO:242.
6. An isolated antibody specifically binding to an ITGA2 protein or an antigen-binding fragment thereof, wherein the isolated antibody or the antigen-binding fragment thereof comprises a light chain and a heavy chain selected from at least one of the following groups:
   1) a light chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31, and
      a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32;
   2) a light chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33, and
      a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34;
   3) a light chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35, and
      a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36;
   4) a light chain that comprises an amino acid sequence encoded by a nucleotide sequence as set forth in SEQ ID NO:37, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:37, or consists of an amino acid sequence encoded by the nucleotide sequence as set forth in SEQ ID NO:37, and
      a heavy chain that comprises an amino acid sequence encoded by a nucleotide sequence as set forth in SEQ ID NO:38, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:38, or consists of an amino acid sequence encoded by the nucleotide sequence as set forth in SEQ ID NO:38; and
   5) a light chain that comprises an amino acid sequence encoded by a nucleotide sequence as set forth in SEQ ID NO:39, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:39, or consists of an amino acid sequence encoded by the nucleotide sequence as set forth in SEQ ID NO:39, and
      a heavy chain that comprises an amino acid sequence encoded by a nucleotide sequence as set forth in SEQ ID NO:40, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:40, or consists of an amino acid sequence encoded by the nucleotide sequence as set forth in SEQ ID NO:40.
7. The isolated antibody or the antigen binding fragment thereof according to any one of clauses 1 to 6,
   wherein the antibody is capable of binding to an ITGA2 protein in vitro at an EC50 of less than 1 nM; and/or
   wherein the antibody is capable of binding to an ITGA2 protein on the surface of HuCCT1 cells with an EC50 of less than 5 nM;
   and/or wherein the antibody is a monoclonal antibody, a polyclonal antibody, a chimeric antibody, or a humanized antibody.
8. An isolated nucleic acid molecule encoding the antibody or the antigen-binding fragment thereof according to any one of clauses 1 to 7; or
   the nucleic acid molecule comprises or consists of a nucleotide sequence combination selected from any one of the following nucleotide sequences: SEQ ID NO:31 and SEQ ID NO:32; SEQ ID NO:33 and SEQ ID NO:34; SEQ ID NO:35 and SEQ ID NO:36; SEQ ID NO:37 and SEQ ID NO:38; and SEQ ID NO:39 and SEQ ID NO:40.
9. The isolated nucleic acid molecule according to clause 8, wherein the nucleic acid molecule is operably linked to an expression regulatory sequence.
10. An expression vector comprising the nucleic acid molecule according to clause 8 or 9.
11. A host cell comprising the nucleic acid molecule according to clause 8 or 9 or the expression vector according to clause 10.
12. A hybridoma cell expressing the antibody or the antigen-binding fragment thereof according to any one of clauses 1 to 7.
13. An antibody-drug conjugate of the formula:
   Ab-L-D, wherein:
      "Ab" represents at least one antibody or antigen-binding fragment thereof according to the present invention described above;
      "L" represents the linker that connects the antibody moiety to the partner molecule; and "D" represents the partner molecule;
      or
   Ab-(L-D)p, wherein:
      "Ab" represents at least one antibody or antigen-binding fragment thereof according to the present invention described above;
      "L" represents the linker that connects the antibody moiety to the partner molecule;
      "D" represents the partner molecule; and
      "p" represents the number of linker/partner molecules (preferably drug payloads) conjugated to the antibody or the antigen-binding fragment thereof according to the present invention described above; optionally, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.
14. The antibody-drug conjugate according to clause 13, wherein the linker is selected from one of the following: vc, mc-GGFG, AcLysvc, mc, MalPeg6, m(H20)c and m(H20)cvc; and/or the linker is cleavable.
15. The antibody-drug conjugate according to clause 13 or 14, wherein
   the partner molecule is a drug payload, wherein the drug payload is a therapeutic molecule; optionally, the drug payload is selected from: a cytotoxic agent, a cytostatic agent, an immunomodulator and a chemotherapeutic agent; optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD; or the partner molecule is a marker molecule; optionally, the marker molecule is selected from one of: a radioactive label (for example, an isotope), a fluorescent label (for example, a fluorescent dye), a chemical substance, and an enzyme or label that can detect a modification.
16. A pharmaceutical composition comprising the antibody or the antigen-binding fragment thereof according to any one of clauses 1-7, or the antibody-drug conjugate according to any one of clauses 13 to 15, and optionally a pharmaceutically acceptable carrier.
17. Use of the antibody or the antigen-binding fragment thereof according to any one of the clauses 1-7 or the antibody-drug conjugate according to any one of the clauses 13 to 15 in preparing a pharmaceutical composition; optionally, the pharmaceutical composition is used for treating a malignant tumor, preventing and/or treating metastasis or relapse of a malignant tumor in a patient; optionally, the pharmaceutical composition is used for detecting presence of a malignant tumor in a patient; and optionally, the pharmaceutical composition is used for prognosticating relapse or progression of a malignant tumor in a patient.
18. A method for treating a malignant tumor, preventing and/or treating metastasis or relapse of a malignant tumor in a patient, comprising administering to the patient an effective amount of the antibody or the antigen-binding fragment thereof according to any one of clauses 1 to 7, the antibody-drug conjugate according to any one of clauses 13 to 15, or the pharmaceutical composition according to clause 16.
19. The method according to clause 18, wherein the malignant tumor is selected from a group consisting of the following: breast cancer, large intestine cancer, skin cancer, pancreatic cancer, prostate cancer, liver cancer, lung cancer, gastric cancer, leukemia, bile duct cancer, cervical cancer, colorectal cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
20. The method according to clause 18 or 19, further comprising administering to the patient other anti-tumor treatments, such as administering a chemotherapeutic agent, an antibody targeting other tumor-specific antigens, or a radiation therapy.
21. A method for detecting presence of tumor cells in a biological sample, comprising:
   a) contacting the biological sample with the antibody or antigen-binding fragment thereof according to any one of clauses 1 to 7;
   b) detecting binding of the antibody or antigen-binding fragment thereof to a target antigen in the biological sample,
   wherein the detected binding represents presence of the tumor cells in the biological sample.
22. A method for detecting presence of a malignant tumor in a patient, comprising:
   a) contacting the biological sample obtained from the patient with the antibody or antigen-binding fragment thereof according to any one of clauses 1 to 7;
   b) detecting binding of the antibody or the antigen-binding fragment thereof to a target antigen in the biological sample, wherein the detected binding represents presence of the malignant tumor in the patient.
23. A method for prognosticating relapse or progression of a malignant tumor in a patient, wherein the method comprises:
   (a) isolating a biological sample comprising tumor cells from the patient;
   (b) contacting the biological sample comprising tumor cells with the antibody or antigen-binding fragment thereof according to any one of clauses 1 to 7; and
   (c) identifying presence of the tumor cells that bind to the antibody or the antigen-binding fragment thereof,
   thereby prognosticating the relapse or progression of the malignant tumor in the patient.
24. The method according to clause 23, wherein the progression of the malignant tumor includes metastasis of the malignant tumor in the patient.
25. The method according to clause 23 or 24, wherein the biological sample is selected from one of the following: a blood sample, a tissue sample, and a lymph sample.
26. The method according to any one of clauses 23 to 25, wherein the malignant tumor is selected from a group consisting of the following: breast cancer, colorectal cancer, large intestine cancer, skin cancer, pancreatic cancer, prostate cancer, liver cancer, lung cancer, gastric cancer, leukemia, bile duct cancer, cervical cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
27. A method for producing an antibody or an antigen-binding fragment thereof targeting human tumor cells that express ITGA2, wherein the method comprises:
   (i) culturing the host cell according to clause 11 in a condition suitable for expressing the nucleic acid molecule or the expression vector, and
   (ii) isolating and purifying the antibody or the antigen-binding fragment thereof expressed by the nucleic acid molecule or the expression vector.

## Claims

1. An isolated antibody specifically binding to an ITGA2 protein or an antigen-binding fragment thereof, comprising a light chain variable region comprising LCDR1, LCDR2, and LCDR3 sequences and a heavy chain variable region comprising HCDR1, HCDR2, and HCDR3 sequences, wherein the antibody or the antigen-binding fragment thereof comprises a light chain variable region and a heavy chain variable region selected from at least one of the following groups:
1) a light chain variable region that comprises (a) LCDR1 as set forth in SEQ ID NO: 162, (b) LCDR2 as set forth in SEQ ID NO: 163, and (c) LCDR3 as set forth in SEQ ID NO: 164, and a heavy chain variable region that comprises (d) HCDR1 as set forth in SEQ ID NO: 166, (e) HCDR2 as set forth in SEQ ID NO: 167, and (f) HCDR3 as set forth in SEQ ID NO: 168;
2) a light chain variable region that comprises (a) LCDR1 as set forth in SEQ ID NO: 170, (b) LCDR2 as set forth in SEQ ID NO: 171, and (c) LCDR3 as set forth in SEQ ID NO: 172, and a heavy chain variable region that comprises (d) HCDR1 as set forth in SEQ ID NO: 174, (e) HCDR2 as set forth in SEQ ID NO: 175, and (f) HCDR3 as set forth in SEQ ID NO: 176;
3) a light chain variable region that comprises (a) LCDR1 as set forth in SEQ ID NO: 178, (b) LCDR2 as set forth in SEQ ID NO: 179, and (c) LCDR3 as set forth in SEQ ID NO: 180, and a heavy chain variable region that comprises (d) HCDR1 as set forth in SEQ ID NO: 182, (e) HCDR2 as set forth in SEQ ID NO: 183, and (f) HCDR3 as set forth in SEQ ID NO: 184;
4) a light chain variable region that comprises (a) LCDR1 as set forth in SEQ ID NO: 186, (b) LCDR2 as set forth in SEQ ID NO: 187, and (c) LCDR3 as set forth in SEQ ID NO: 188, and a heavy chain variable region that comprises (d) HCDR1 as set forth in SEQ ID NO: 190, (e) HCDR2 as set forth in SEQ ID NO: 191, and (f) HCDR3 as set forth in SEQ ID NO: 192; and
5) a light chain variable region that comprises (a) LCDR1 as set forth in SEQ ID NO: 194, (b) LCDR2 as set forth in SEQ ID NO: 195, and (c) LCDR3 as set forth in SEQ ID NO: 196, and a heavy chain variable region that comprises (d) HCDR1 as set forth in SEQ ID NO: 198, (e) HCDR2 as set forth in SEQ ID NO: 199, and (f) HCDR3 as set forth in SEQ ID NO: 200.

2. The isolated antibody or the antigen binding fragment thereof according to claim 1, wherein the isolated antibody or the antigen binding fragment thereof comprises a light chain variable region and a heavy chain variable region selected from at least one of the following groups:
1) a light chain variable region that comprises an amino acid sequence as set forth in SEQ ID NO:161, or an amino acid sequence that is at least 85%, at least 90%, at least 95%, at least 96%, at least 97%, at least 98%, or at least 99% identical to the amino acid sequence as set forth in SEQ ID NO:161, and a heavy chain variable region that comprises an amino acid sequence as set forth in SEQ ID NO:165, or an amino acid sequence that is at least 85%, at least 90%, at least 95%, at least 96%, at least 97%, at least 98%, or at least 99% identical to the amino acid sequence as set forth in SEQ ID NO:165;
2) a light chain variable region that comprises an amino acid sequence as set forth in SEQ ID NO:169, or an amino acid sequence that is at least 85%, at least 90%, at least 95%, at least 96%, at least 97%, at least 98%, or at least 99% identical to the amino acid sequence as set forth in SEQ ID NO:169, and a heavy chain variable region that comprises an amino acid sequence as set forth in SEQ ID NO:173, or an amino acid sequence that is at least 85%, at least 90%, at least 95%, at least 96%, at least 97%, at least 98%, or at least 99% identical to the amino acid sequence as set forth in SEQ ID NO:173;
3) a light chain variable region that comprises an amino acid sequence as set forth in SEQ ID NO:177, or an amino acid sequence that is at least 85%, at least 90%, at least 95%, at least 96%, at least 97%, at least 98%, or at least 99% identical to the amino acid sequence as set forth in SEQ ID NO:177, and a heavy chain variable region that comprises an amino acid sequence as set forth in SEQ ID NO:181, or an amino acid sequence that is at least 85%, at least 90%, at least 95%, at least 96%, at least 97%, at least 98%, or at least 99% identical to the amino acid sequence as set forth in SEQ ID NO:181;
4) a light chain variable region that comprises an amino acid sequence as set forth in SEQ ID NO:185, or an amino acid sequence that is at least 85%, at least 90%, at least 95%, at least 96%, at least 97%, at least 98%, or at least 99% identical to the amino acid sequence as set forth in SEQ ID NO:185, and a heavy chain variable region that comprises an amino acid sequence as set forth in SEQ ID NO:189, or an amino acid sequence that is at least 85%, at least 90%, at least 95%, at least 96%, at least 97%, at least 98%, or at least 99% identical to the amino acid sequence as set forth in SEQ ID NO:189; and
5) a light chain variable region that comprises an amino acid sequence as set forth in SEQ ID NO:193, or an amino acid sequence that is at least 85%, at least 90%, at least 95%, at least 96%, at least 97%, at least 98%, or at least 99% identical to the amino acid sequence as set forth in SEQ ID NO:193, and a heavy chain variable region that comprises an amino acid sequence as set forth in SEQ ID NO:197, or an amino acid sequence that is at least 85%, at least 90%, at least 95%, at least 96%, at least 97%, at least 98%, or at least 99% identical to the amino acid sequence as set forth in SEQ ID NO:197.

3. The isolated antibody or the antigen binding fragment thereof according to claim 1 or 2, wherein the isolated antibody or the antigen binding fragment thereof further comprises a light chain constant region and/or a heavy chain constant region,
the light chain constant region comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201, and
the heavy chain constant region comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:234.

4. The isolated antibody or the antigen binding fragment thereof according to any one of claims 1 to 3, wherein the isolated antibody or the antigen binding fragment thereof comprises a light chain and a heavy chain selected from at least one of the following groups:
1) a light chain that comprises an amino acid sequence as set forth in SEQ ID NO:233, or an amino acid sequence that is at least 85%, at least 90%, at least 95%, at least 96%, at least 97%, at least 98%, or at least 99% identical to the amino acid sequence as set forth in SEQ ID NO:233, and a heavy chain that comprises an amino acid sequence as set forth in SEQ ID NO:234, or an amino acid sequence that is at least 85%, at least 90%, at least 95%, at least 96%, at least 97%, at least 98%, or at least 99% identical to the amino acid sequence as set forth in SEQ ID NO:234;
2) a light chain that comprises an amino acid sequence as set forth in SEQ ID NO:235, or an amino acid sequence that is at least 85%, at least 90%, at least 95%, at least 96%, at least 97%, at least 98%, or at least 99% identical to the amino acid sequence as set forth in SEQ ID NO:235, and a heavy chain that comprises an amino acid sequence as set forth in SEQ ID NO:236, or an amino acid sequence that is at least 85%, at least 90%, at least 95%, at least 96%, at least 97%, at least 98%, or at least 99% identical to the amino acid sequence as set forth in SEQ ID NO:236;
3) a light chain that comprises an amino acid sequence as set forth in SEQ ID NO:237, or an amino acid sequence that is at least 85%, at least 90%, at least 95%, at least 96%, at least 97%, at least 98%, or at least 99% identical to the amino acid sequence as set forth in SEQ ID NO:237, and a heavy chain that comprises an amino acid sequence as set forth in SEQ ID NO:238, or an amino acid sequence that is at least 85%, at least 90%, at least 95%, at least 96%, at least 97%, at least 98%, or at least 99% identical to the amino acid sequence as set forth in SEQ ID NO:238;
4) a light chain that comprises an amino acid sequence as set forth in SEQ ID NO:239, or an amino acid sequence that is at least 85%, at least 90%, at least 95%, at least 96%, at least 97%, at least 98%, or at least 99% identical to the amino acid sequence as set forth in SEQ ID NO:239, and a heavy chain that comprises an amino acid sequence as set forth in SEQ ID NO:240, or an amino acid sequence that is at least 85%, at least 90%, at least 95%, at least 96%, at least 97%, at least 98%, or at least 99% identical to the amino acid sequence as set forth in SEQ ID NO:240; and
5) a light chain that comprises an amino acid sequence as set forth in SEQ ID NO:241, or an amino acid sequence that is at least 85%, at least 90%, at least 95%, at least 96%, at least 97%, at least 98%, or at least 99% identical to the amino acid sequence as set forth in SEQ ID NO:241, and a heavy chain that comprises an amino acid sequence as set forth in SEQ ID NO:242, or an amino acid sequence that is at least 85%, at least 90%, at least 95%, at least 96%, at least 97%, at least 98%, or at least 99% identical to the amino acid sequence as set forth in SEQ ID NO:242.

5. The isolated antibody or the antigen binding fragment thereof according to any one of claims 1 to 4, wherein the isolated antibody or the antigen binding fragment thereof consists of a light chain and a heavy chain selected from at least one of the following groups:
1) a light chain that consists of an amino acid sequence as set forth in SEQ ID NO:233, and a heavy chain that consists of an amino acid sequence as set forth in SEQ ID NO:234;
2) a light chain that consists of an amino acid sequence as set forth in SEQ ID NO:235, and a heavy chain that consists of an amino acid sequence as set forth in SEQ ID NO:236;
3) a light chain that consists of an amino acid sequence as set forth in SEQ ID NO:237, and a heavy chain that consists of an amino acid sequence as set forth in SEQ ID NO:238;
4) a light chain that consists of an amino acid sequence as set forth in SEQ ID NO:239, and a heavy chain that consists of an amino acid sequence as set forth in SEQ ID NO:240; and
5) a light chain that consists of an amino acid sequence as set forth in SEQ ID NO:241, and a heavy chain that consists of an amino acid sequence as set forth in SEQ ID NO:242.

6. An isolated antibody specifically binding to an ITGA2 protein or an antigen-binding fragment thereof, wherein the isolated antibody or the antigen-binding fragment thereof comprises a light chain and a heavy chain selected from at least one of the following groups:
1) a light chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31, and
a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32;
2) a light chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33, and
a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34;
3) a light chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35, and
a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36;
4) a light chain that comprises an amino acid sequence encoded by a nucleotide sequence as set forth in SEQ ID NO:37, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:37, or consists of an amino acid sequence encoded by the nucleotide sequence as set forth in SEQ ID NO:37, and
a heavy chain that comprises an amino acid sequence encoded by a nucleotide sequence as set forth in SEQ ID NO:38, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:38, or consists of an amino acid sequence encoded by the nucleotide sequence as set forth in SEQ ID NO:38; and
5) a light chain that comprises an amino acid sequence encoded by a nucleotide sequence as set forth in SEQ ID NO:39, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:39, or consists of an amino acid sequence encoded by the nucleotide sequence as set forth in SEQ ID NO:39, and
a heavy chain that comprises an amino acid sequence encoded by a nucleotide sequence as set forth in SEQ ID NO:40, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:40, or consists of an amino acid sequence encoded by the nucleotide sequence as set forth in SEQ ID NO:40.

7. The isolated antibody or the antigen binding fragment thereof according to any one of claims 1 to 6,
wherein the antibody is capable of binding to an ITGA2 protein in vitro at an EC50 of less than 1 nM; and/or
wherein the antibody is capable of binding to an ITGA2 protein on the surface of HuCCT1 cells with an EC50 of less than 5 nM; and/or
wherein the antibody is a monoclonal antibody, a polyclonal antibody, a chimeric antibody, or a humanized antibody.

8. An isolated nucleic acid molecule encoding the antibody or the antigen-binding fragment thereof according to any one of claims 1 to 7; or
the nucleic acid molecule comprises or consists of a nucleotide sequence combination selected from any one of the following nucleotide sequences: SEQ ID NO:31 and SEQ ID NO:32; SEQ ID NO:33 and SEQ ID NO:34; SEQ ID NO:35 and SEQ ID NO:36; SEQ ID NO:37 and SEQ ID NO:38; and SEQ ID NO:39 and SEQ ID NO:40.

9. An antibody-drug conjugate of the formula:
Ab-L-D, wherein:
"Ab" represents at least one isolated antibody or antigen-binding fragment thereof according to any one of claims 1 to 7;
"L" represents a linker that connects an antibody moiety to a partner molecule; and
"D" represents the partner molecule;
or
Ab-(L-D)p, wherein:
"Ab" represents at least one isolated antibody or antigen-binding fragment thereof according to any one of claims 1 to 7;
"L" represents a linker that connects an antibody moiety to a partner molecule;
"D" represents the partner molecule; and
"p" represents the number of linker/partner molecules (preferably drug payloads) conjugated to the antibody or the antigen-binding fragment thereof according to any one of claims 1 to 7; optionally, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, *4.5, 4.6, 4.7, 4.8, 4.9, 5.0, 5.1, 5.2, 5.3,* 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.

10. The antibody-drug conjugate according to claim 9, wherein the linker is selected from one of the following: vc, mc-GGFG, AcLysvc, mc, MalPeg6, m(H20)c and m(H20)cvc; and/or the linker is cleavable.

11. The antibody-drug conjugate according to claim 9 or 10, wherein
the partner molecule is a drug payload, wherein the drug payload is a therapeutic molecule; optionally, the drug payload is selected from: a cytotoxic agent, a cytostatic agent, an immunomodulator and a chemotherapeutic agent; optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD; or
the partner molecule is a marker molecule; optionally, the marker molecule is selected from one of: a radioactive label (for example, an isotope), a fluorescent label (for example, a fluorescent dye), a chemical substance, and an enzyme or label that is capable of detecting a modification.

12. A pharmaceutical composition comprising the antibody or the antigen-binding fragment thereof according to any one of claims 1 to 7, or the antibody-drug conjugate according to claim 9 or 10, and optionally a pharmaceutically acceptable carrier.

13. Use of the antibody or the antigen-binding fragment thereof according to any one of claims 1 to 7 or the antibody-drug conjugate according to any one of claims 9 to 11 in preparing a pharmaceutical composition; optionally, the pharmaceutical composition is used for treating a malignant tumor, preventing and/or treating metastasis or relapse of a malignant tumor in a patient; optionally, the pharmaceutical composition is used for detecting presence of a malignant tumor in a patient; and optionally, the pharmaceutical composition is used for prognosticating relapse or progression of a malignant tumor in a patient.

14. A method for treating a malignant tumor, preventing and/or treating metastasis or relapse of a malignant tumor in a patient, wherein the method comprises administering to the patient an effective amount of the antibody or the antigen-binding fragment thereof according to any one of claims 1 to 7, the antibody-drug conjugate according to any one of claims 9 to 11, or the pharmaceutical composition according to claim 12.

15. The method according to claim 14, wherein the malignant tumor is selected from a group consisting of the following: breast cancer, large intestine cancer, skin cancer, pancreatic cancer, prostate cancer, liver cancer, lung cancer, gastric cancer, leukemia, bile duct cancer, cervical cancer, colorectal cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.

16. The method according to claim 14 or 15, further comprising administering to the patient other anti-tumor treatments, such as administering a chemotherapeutic agent, an antibody targeting other tumor-specific antigens, or a radiation therapy.
